# EUROPÄISCHE PATENTSCHRIFT

(11) **EP 1 478 628 B1**
(45) Veröffentlichungstag und Bekanntmachung des Hinweises auf die Patenterteilung: **08.10.2008**
(21) Anmeldenummer: 03708083.5
(22) Anmeldetag: 06.02.2003
(51) Int. Cl.: C07D 251/48, C07D 409/12, C07D 405/12, C07D 401/12, C07D 403/12, C07C 279/26, A01N 43/68, A01N 43/90, C07D 495/04, C07D 417/12, C07D 413/12, C07C 211/42, C07D 471/04

(54) **2-AMINO-4-BICYCLYLAMINO-6H-1,3,5-TRIAZINE, VERFAHREN ZU DEREN HERSTELLUNG UND DEREN VERWENDUNG ALS HERBIZIDE UND PFLANZENWACHSTUMSREGULATOREN**
2-AMINO-4-BICYCLYL AMINO-6H-1.3.5-TRIAZINES, METHOD FOR THE PRODUCTION AND USE THEREOF AS HERBICIDES AND PLANT GROWTH REGULATORS
2-AMINO-4-BICYCLOAMINO-6H-1,3,5-TRIAZINES, PROCEDE PERMETTANT DE LES PRODUIRE ET LEUR UTILISATION COMME HERBICIDES ET REGULATEURS DE CROISSANCE DE PLANTES

(30) Priorität: 20.02.2002 DE 10207037
(43) Veröffentlichungstag der Anmeldung: 24.11.2004
(73) Patentinhaber: Bayer CropScience AG, 40789 Monheim (DE)
(72) Erfinder: MINN, Klemens, 65795 Hattersheim (DE); AHRENS, Hartmut, 60598 Frankfurt am Main (DE); DIETRICH, Hansjörg, 65719 Hofheim (DE); WILLMS, Lothar, 65719 Hofheim (DE); AULER, Thomas, 65812 Bad Soden (DE); BIERINGER, Hermann, 65817 Eppstein (DE); MENNE, Hubert, 65719 Hofheim (DE)
(86) Internationale Anmeldenummer: PCT/EP2003/001157
(87) Internationale Veröffentlichungsnummer: WO 2003/070710

(56) Entgegenhaltungen:
- EP-A- 0 864 567
- WO-A-97/31904
- DE-A- 2 505 301
- DE-A- 19 921 883
- US-A- 4 156 670
- PATENT ABSTRACTS OF JAPAN vol. 1996, no. 11, 29. November 1996 (1996-11-29) -& JP 08 183781 A (IDEMITSU KOSAN CO LTD), 16. Juli 1996 (1996-07-16)

## Beschreibung

2-Amino-4-bicyclylamino-6H-1,3,5-triazine, Verfahren zu deren Herstellung und deren Verwendung als Herbizide und Pflanzenwachstumsregulatoren.

Die Erfindung betrifft das technische Gebiet der Herbizide und Pflanzenwachstumsregulatoren, insbesondere der Herbizide zur Bekämpfung von Unkräutern und Ungräsern in Nutzpflanzenkulturen und unerwünschtem Wachstum von Pflanzen generell.

Es ist bekannt, dass 2-Amino-4-cyclohexylamino-6-perhaloalkyl-1,3,5-triazine (siehe z. B. US-A-3816419 und US-A-3932167) oder 2-Amino-4-alkylamino-6-halogenalkyl-1,3,5-triazine (WO-A-90/09378 (EP-A-411153), WO-A-88/02368 (EP-A-283522), WO-A-94/24086, EP-A-509544, EP-A-492,615) und auch 2-Amino-4-bicyclylamino-1,3,5-triazine (WO 97/31904 (DE-A-19607-450), WO-A-97/19936) herbizide und pflanzenwachstumsregulierende Eigenschaften besitzen.

Die Anwendung der Derivate dieses Typs als selektive Herbizide zur Schadpflanzenbekämpfung oder als Pflanzenwachstumsregulatoren in verschiedenen Nutzpflanzenkulturen erfordert jedoch häufig eine unzeitgemäße Aufwandmenge oder führt zu unerwünschten Schädigungen der Nutzpflanzen. Zudem ist die Anwendung der Wirkstoffe in vielen Fällen wegen verhältnismäßig hoher Herstellkosten unwirtschaftlich. Überraschenderweise wurden nun neue bicyclisch substituierte 2,4-Diamino-1,3,5-triazine gefunden, welche vorteilhaft als Herbizide und Pflanzenwachstumsregulatoren eingesetzt werden können. Die Verbindungen dieser Reihe zeichnen sich neben gutem Wirksamkeitsprofil und Kulturpflanzenverträglichkeit durch kostengünstige Herstellprozesse und Zugangsmöglichkeiten für den Triazinteil aus, da in den hier beschriebenen Substanzen das Triazin aus preiswerten und gut zugänglichen Vorstufen hergestellt und daher auf die Verwendung teurer und schwer zugänglicher Zwischenprodukte verzichtet werden kann.

Gegenstand der vorliegenden Erfindung sind Verbindungen der Formel (I) und deren Salze, 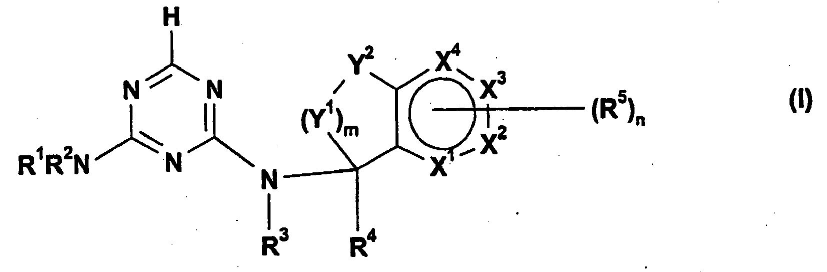 worin
R¹ und R² jeweils unabhängig voneinander Wasserstoff, eine Gruppe der Formel NR'R", worin R' und R" jeweils unabhängig voneinander H, (C₁-C₆)Alkyl, (C₂-C₆)Alkenyl, (C₂-C₆)Alkinyl, (C₃-C₆)Cycloalkyl oder (C₅-C₆)Cycloalkenyl bedeuten, oder einen (acyclischen oder cyclischen) Kohlenwasserstoffrest oder Kohlenwasserstoffoxyrest mit jeweils 1 bis 10 C-Atomen, vorzugsweise 1 bis 6 C-Atomen, oder einen Heterocyclylrest, Heterocyclyloxyrest, Heterocyclylthiorest oder Heterocyclylaminorest mit jeweils 3 bis 9 Ringatomen und 1 bis 3 Heteroringatomen aus der Gruppe N, O und S, wobei jeder der sechs letztgenannten Reste unsubstituiert oder substituiert ist, oder einen Acylrest einer organischen Säure mit 1 bis 20 C-Atomen, vorzugsweise mit 1 bis 12 C-Atomen, insbesondere mit 1 bis 8 C-Atomen, oder R¹ und R² gemeinsam mit dem Stickstoffatom der Gruppe NR¹R² einen gesättigten oder ungesättigten, nicht aromatischen heterocyclischen Rest mit 3 bis 9 Ringatomen und 1 bis 4 Heteroringatomen, wobei neben dem N-Atom die gegebenenfalls weiteren Heteroringatome aus der Gruppe N, O und S ausgewählt sind und der Rest unsubstituiert oder substituiert ist, wobei jeder der kohlenstoffhaltigen Reste R¹ und R² inklusive Substituenten 1 bis 20 C-Atome, vorzugsweise 1 bis 12 C-Atome, insbesondere 1 bis 10 C-Atome aufweist,
R³ Wasserstoff, Amino, (C₁-C₄)Alkylamino, Di(C₁-C₄)alkylamino, (C₁-C₆)Alkyl, (C₁-C₆)Alkoxy, (C₂-C₆)Alkenyl, (C₂-C₆)Alkinyl, (C₃-C₆)Cycloalkyl, (C₅-C₆)Cycloalkenyl, Phenyl oder Heterocyclyl,
   wobei jeder der letztgenannten 10 Reste unabhängig voneinander unsubstituiert oder substituiert ist und der jeweilige Rest inklusive Substituenten 1 bis 12 C-Atome, insbesondere 1 bis 10 C-Atome aufweist, oder einen Acylrest der Formel -B*-A*, worin
   A* Wasserstoff oder einen acyclischen oder cyclischen Kohlenwasserstoffrest mit 1 bis 10 C-Atomen, vorzugsweise mit 1 bis 6 C-Atomen, der unsubstituiert oder substituiert ist, und
   B* eine divalente Gruppe der Formeln -CO-, -CO-O-, -CO-NR'-, -S(O)ₚ- oder -S(O)ₚ-O-, wobei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 6 C-Atomen ist,
      bedeuten,
   wobei -B*-A* inklusive Substituenten 1 bis 12, vorzugsweise 1 bis 10 C-Atome aufweist,
R⁴ einen Rest der Formel -Z¹-R⁶, wobei Z¹ und R⁶ wie unten angegeben definiert sind,
R⁵ jeweils unabhängig voneinander Halogen, Cyano, Isocyanato, Nitro, einen Rest der Formel -Z²-R⁷, wobei Z² und R⁷ wie unten angegeben definiert sind, oder zwei benachbarte Reste R⁵ gemeinsam einen ankondensierten Cyclus mit 4 bis 6 Ringatomen, der carbocyclisch ist oder 1 bis 3 Heteroringatome aus der Gruppe O, S und N enthält und der unsubstituiert oder durch einen oder mehrere Reste aus der Gruppe Halogen, (C₁-C₄)Alkyl und Oxo substituiert ist,
R⁶ , R⁷, R⁸, R⁹ jeweils unabhängig voneinander Wasserstoff, ausgenommen R⁷ = Wasserstoff, wenn Z² = direkte Bindung, oder einen acyclischen Kohlenwasserstoffrest mit 1 bis 20 C-Atomen, vorzugsweise mit 1 bis 10 C-Atomen, oder einen cyclischen, vorzugsweise monocyclischen Kohlenwasserstoffrest mit vorzugsweise 3 bis 8 C-Atomen, insbesondere mit 3 bis 6 C-Atomen, oder einen heterocyclischen Rest, vorzugsweise mit 3 bis 9 Ringatomen und 1 bis 3 Heteroringatomen aus der Gruppe N, O und S, wobei jeder der drei letztgenannten Reste unsubstituiert oder substituiert ist und inklusive Substituenten vorzugsweise bis zu 30 C-Atome, insbesondere bis zu 20 C-Atome aufweist,
   oder R⁸ und R⁹ zusammen mit dem C-Atom einer Gruppe CR⁸R⁹ (für Y¹ oder Y²) oder zwei Reste R⁸ oder R⁹ aus zwei Gruppen Y¹ und/oder Y² gemeinsam mit den verknüpften Atomen der Gruppen Y¹ und/oder Y² jeweils einen carbocyclischen Rest mit 3 bis 8 C-Atomen, insbesondere mit 3 bis 6 C-Atomen, oder einen heterocyclischen Rest, vorzugsweise mit 3 bis 9 Ringatomen und 1 bis 3 Heteroringatomen aus der Gruppe N, O und S, wobei jeder der zwei letztgenannten Reste unsubstituiert oder substituiert ist und inklusive Substituenten vorzugsweise bis zu 16 C-Atome, insbesondere bis zu 12 C-Atome aufweist.
X¹, X², X³, X⁴ jeweils unabhängig voneinander ein Kohlenstoffatom, das mit einem Wasserstoffatom oder einer der definierten Substituenten R⁵ substituiert ist, oder ein Stickstoffatom oder zwei benachbarte Symbole X¹, X², X³ und X⁴ jeweils gemeinsam eine divalente Gruppe der Formel -O-, -S-, -NH- oder -NR-, worin R wie für R³ definiert ist und vorzugsweise Wasserstoff oder (C₁-C₆)Alkyl bedeutet, vorausgesetzt die Gruppen X¹, X², X³, X⁴ bilden zusammen mit der verknüpften C₂-Einheit des ankondensierten Rings einen carbocyclischen oder heterocyclischen aromatischen fünfgliedrigen oder sechsgliedrigen Ring,
(Y¹)ₘ m divalente Gruppen Y¹, wobei jede Gruppe Y¹ unabhängig von anderen Gruppe Y¹ eine Gruppe der Formel -O-, -CO-, -C(=NR*)-, -S(O)q-, -NR*- oder -N(O)-, wobei q = 0, 1 oder 2 ist und R* wie für R³ definiert ist und vorzugsweise Wasserstoff, (C₁-C₆)Alkyl, Benzyl oder Phenyl bedeutet, insbesondere Wasserstoff, (C₁-C₄)Alkyl oder Phenyl ist, oder eine Gruppe der Formel CR⁸R⁹, wobei R⁸ und R⁹ wie oben definiert ist, bedeutet, und
Y² eine Gruppe wie für Y¹ definiert oder eine direkte Bindung, wobei zwei benachbarte Gruppen der Symbolpaare Y¹ und Y¹ oder der Symbolpaare Y¹ und Y² nicht heteroatomige Gruppen derselben Bedeutung darstellen und wobei die Gruppen (Y¹)ₘ und Y² zusammen mit der verknüpften C₂-Einheit des aromatischen Rings und dem mit R⁴ verknüpften C-Atom einen ankondensierten carbocyclischen oder heterocyclischen nicht aromatischen viergliedrigen bis achtgliedrigen Ring bilden,
Z¹ und die Gruppen Z² jeweils unabhängig voneinander eine direkte Bindung oder eine divalente Gruppe der Formel -O-, -S(O)ₚ-, -S(O)ₚ-O-, -O-S(O)ₚ-, -CO-, -O-CO-, -CO-O-, -NR'-, -O-NR'-, -NR'-O-, -NR'-CO-, -CO-NR'- bedeutet und dabei p = 0, 1 oder 2 ist und R' Wasserstoff, Alkyl mit 1 bis 6 C-Atomen, Phenyl, Benzyl, (C₃-C₆)Cycloalkyl oder Alkanoyl mit 1 bis 6 C-Atomen ist,
m 0, 1, 2, 3 oder 4, insbesondere 1 oder 2, und
n 0, 1, 2, 3 oder 4, insbesondere 0, 1 oder 2, ganz besonders 1 oder 2, bedeuten.
   Die Verbindungen der Formel (I) können durch Anlagerung einer geeigneten anorganischen oder organischen Säure, wie beispielsweise HCl, HBr, H₂SO₄ oder HNO₂ aber auch Oxalsäure oder Sulfonsäuren an eine basische Gruppe, wie z.B. Amino oder Alkylamino, Salze bilden. Geeignete Substituenten, die in deprotonierter Form vorliegen, wie z.B. Sulfonsäuren oder Carbonsäuren, können innere Salze mit ihrerseits protonierbaren Gruppen, wie Aminogruppen bilden. Salze können ebenfalls dadurch gebildet werden, dass bei geeigneten Substituenten, wie z.B. Sulfonsäuren oder Carbonsäuren, der Wasserstoff durch ein für die Landwirtschaft geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quatemäre) Ammoniumsalze.

In Formel (I) und allen nachfolgenden Formeln gelten nachstehende Definitionen:

In den divalenten Gruppen wie B*, Z¹ und Z², welche auch mehratomige Brücken wie -CO-NR'- symbolisieren können, bedeutet ein zusammengesetzter Rest in der Schreibweise -B*-A* oder -Z¹-R⁶ die Gruppe -CO-NR'-A* bzw. -CO-NR'-R⁶ und nicht die Gruppe -NR'-CO-A* bzw. -NR'-CO-R⁶.

In den divalenten Gruppen -(Y¹)ₘ- ist die Verknüpfung so definiert, dass die links geschriebene freie Bindungsstelle an dem C-Atom gebunden ist, das mit R⁴ substituiert ist; entsprechend ist die links geschriebene freie Bindungsstelle der nächsten Gruppe Y¹ mit der rechts geschriebenen freien Bindung der ersten Gruppe Y¹ verknüpft.

Eine Gruppe der Formel -S(O)ₚ- bedeutet ein Schwefelatom mit zwei freien Bindungen, an dem p Sauerstoffatome doppelt gebunden sind, d. h., wenn p = 0, 1 oder 2, eine Gruppe der Formel -S-, -S(=O)- (oder kurz -SO-, Sulfinyl) oder -S(=O)₂-(oder kurz -SO₂-, Sulfonyl) und nicht etwa auch -S-O- (Sulfenyloxy) oder -SO-O-(Sulfinyloxy).

Heteroatomige Gruppen bedeuten einatomige oder mehratomige Gruppen mit stark funktionalem Charakter, welche aus einem oder mehreren von Kohlenstoffatomen verschiedenen Atomen (Heteroatomen) bestehen, jedoch auch Kohlenstoffatome enthalten können. Im Falle von Y¹ sind dies Gruppen der Formel -O-, -CO-, - C(=NR*)-, -S(O)q-, -NR*- oder -N(O)-, im Gegensatz zu Gruppen der Formel CR⁸R⁹.

Die Begriffe Alkyl, Alkoxy, Haloalkyl, Haloalkoxy, Alkylamino und Alkylthio sowie die Begriffe für entsprechende ungesättigte und/oder substituierte Reste umfassen jeweils sowohl betreffende Reste mit geradkettigem als auch mit verzweigtem (nicht cyclischem) Kohlenstoffgerüst.

Der Ausdruck "(C₁-C₄)Alkyl" bedeutet eine Kurzschreibweise für Alkyl mit einem bis 4 Kohlenstoffatomen, d. h. umfasst die Reste Methyl, Ethyl, 1-Propyl, 2-Propyl, 1-Butyl, 2-Butyl, 2-Methylpropyl or tert-Butyl. Allgemeine Alkylreste mit einem größeren angegebenen Bereich von C-Atomen, z. B. "(C₁-C₆)Alkyl" umfassen entsprechend auch gradkettige oder verzweigte Alkylreste mit einer größeren Zahl von C-Atomen, d. h. gemäß Beispiel auch die Alkylreste mit 5 und 6 C-Atomen.
Wenn nicht speziell angegeben, sind bei den Kohlenwasserstoffresten wie Alkyl-, Alkenyl- und Alkinylresten, auch in zusammengesetzten Resten, die niederen Kohlenstoffgerüste, z.B. mit 1 bis 6 C-Atomen, insbesondere mit 1 bis 4 C-Atomen, bzw. bei ungesättigten Gruppen mit 2 bis 6 C-Atomen, insbesondere mit 2 bis 4 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Vinyl, Allyl, 1-Methyl-2-propenyl, 2-Methyl-2-propenyl, 2-Butenyl, Pentenyl, 2-Methylpentenyl oder Hexenyl, vorzugsweise Allyl, 1-Methylprop-2-en-1-yl, 2-Methyl-prop-2-en-1-yl, But-2-en-1-yl, But-3-en-1-yl, 1-Methyl-but-3-en-1-yl oder 1-Methyl-but-2-en-1-yl.
Alkenyl schließt insbesondere auch geradkettige oder verzweigte Kohlenwasserstoffreste mit mehr als einer Doppelbindung ein, wie 1,3-Butadienyl und 1,4-Pentadienyl, aber auch Allenyl- oder Kumulenyl-reste mit einer bzw. mehreren kumulierten Doppelbindungen, wie beispielsweise Allenyl (1,2-Propadienyl), 1,2-Butadienyl und 1,2,3-Pentatrienyl.

Alkinyl bedeutet z.B. Propargyl, But-2-in-1-yl, But-3-in-1-yl, 1-Methyl-but-3-in-1-yl. Alkinyl schließt insbesondere auch geradkettige oder verweigte Kohlenwasserstoffreste mit mehr als einer Dreifachbindung oder auch mit einer oder mehreren Dreifachbindungen und einer oder mehreren Doppelbindungen ein, wie beispielsweise 1,3-Butatrienyl bzw. 3-Penten-1-in-1-yl.

Alkyliden, z. B. auch in der Form (C₁-C₁₀)Alkyliden, bedeutet den Rest eines geradkettigen oder verzweigten Alkans, der über eine Zweifachbindung gebunden ist, wobei die Position der Bindungsstelle noch nicht festgelegt ist. Im Falle eines verzweigten Alkans kommen naturgemäß nur Positionen in Frage, an denen zwei H-Atome durch die Doppelbindung ersetzt werden können; Reste sind z. B. =CH₂, =CH-CH₃, =C(CH₃)-CH₃, =C(CH₃)-C₂H₅ oder =C(C₂H₅)-C₂H₅.

Cycloalkyl bedeutet ein carbocyclisches, gesättigtes Ringsystem mit vorzugsweise 3-8 C-Atomen, z.B. Cyclopropyl, Cyclobutyl, Cyclopentyl oder Cyclohexyl. Im Falle von substituiertem Cycloalkyl werden cyclische Systeme mit Substituenten umfaßt, wobei die Substituenten mit einer Doppelbindung am Cycloalkylrest, z. B. eine Alkylidengruppe wie Methyliden, gebunden sind. Im Falle von substituiertem Cycloalkyl werden auch mehrcyclische aliphatische Systeme umfaßt, wie beispielsweise Bicyclo[1.1.0]butan-1-yl, Bicyclo[1.1.0]butan-2-yl, Bicyclo[2.1.0]pentan-1-yl, Bicyclo[2.1.0]pentan-2-yl, Bicyclo[2.1.0]pentan-5-yl, Adamantan-1-yl und Adamantan-2-yl.

Cycloalkenyl bedeutet ein carbocyclisches, nicht aromatisches, partiell ungesättigtes Ringsystem mit vorzugsweise 4-8 C-Atomen, z.B. 1-Cyclobutenyl; 2-Cyclobutenyl, 1-Cyclopentenyl, 2-Cyclopentenyl, 3-Cyclopentenyl, oder 1-Cyclohexenyl, 2-Cyclohexenyl, 3-Cyclohexenyl, 1,3-Cyclohexadienyl oder 1,4-Cyclohexadienyl. Im Falle von substituiertem Cycloalkenyl gelten die Erläuterungen für substituiertes Cycloalkyl entsprechend

Halogen bedeutet beispielsweise Fluor, Chlor, Brom oder Iod. Haloalkyl, -alkenyl und alkinyl bedeuten durch gleiche oder verschiedene Halogenatome, vorzugsweise aus der Gruppe Fluor, Chlor und Brom, insbesondere aus der Gruppe Fluor und Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; Entsprechendes gilt für Haloalkenyl und andere durch Halogen substituierte Reste.

Aryl bedeutet ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl.

Ein heterocyclischer Rest oder Ring (Heterocyclyl) kann gesättigt, ungesättigt oder heteroaromatisch sein; wenn nicht anders definiert, enthält er vorzugsweise ein oder mehrere, insbesondere 1, 2 oder 3 Heteroatome im heterocyclischen Ring, vorzugsweise aus der Gruppe N, O, und S; vorzugsweise ist er ein aliphatischer Heterocyclylrest mit 3 bis 7 Ringatomen oder ein heteroaromatischer Rest mit 5 oder 6 Ringatomen. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält. Vorzugsweise ist er ein heteroaromatischer Ring mit einem Heteroatom aus der Gruppe N, O und S, beispielsweise Pyridyl, Pyrrolyl, Thienyl oder Furyl; weiterhin bevorzugt ist er ein entsprechender heteroaromatischer Ring mit 2 oder 3 Heteroatomen, z. B. Pyrimidinyl, Pyridazinyl, Pyrazinyl, Triazinyl, Thiazolyl, Thiadiazolyl, Oxazolyl, Isoxazolyl, Pyrazolyl, Imidazolyl, und Triazolyl. Weiterhin bevorzugt ist er ein partiell oder vollständig hydrierter heterocyclischer Rest mit einem Heteroatom aus der Gruppe N, O und S, beispielsweise Oxiranyl, Oxetanyl, Oxolanyl (= Tetrahydrofuryl), Oxanyl, Pyrrolinyl, Pyrrolidyl oder Piperidyl.

Weiterhin bevorzugt ist er ein partiell oder vollständig hydrierter heterocyclischer Rest mit 2 Heteroatomen aus der Gruppe N, O und S, beispielsweise Piperazinyl, Dioxolanyl, Oxazolinyl, Isoxazolinyl, Oxazolidinyl, Isoxazolidinyl und Morpholinyl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Bevorzugte Beispiele für Heterocyclyl sind ein heterocyclischer Rest mit 3 bis 6 Ringatomen aus der Gruppe Pyridyl, Thienyl, Furyl, Pyrrolyl, Oxiranyl, 2-Oxetanyl, 3-Oxetanyl, Oxolanyl (= Tetrahydrofuryl), Pyrrolidyl, Piperidyl, insbesondere Oxiranyl, 2-Oxetanyl, 3-Oxetanyl oder Oxolanyl, oder ist ein heterocyclischer Rest mit zwei oder drei Heteroatomen, beispielsweise Pyrimidinyl, Pyridazinyl, Pyrazinyl, Triazinyl, Thienyl, Thiazolyl, Thiadiazolyl, Oxazolyl, Isoxazolyl, Pyrazolyl, Triazolyl, Piperazinyl, Dioxolanyl, Oxazolinyl, Isoxazolinyl, Oxazolidinyl, Isoxazolidinyl oder Morpholinyl.

Wenn ein Grundkörper "durch einen oder mehrere Reste" aus einer Aufzählung von Resten (= Gruppe) oder einer generisch definierten Gruppe von Resten substituiert ist, so schließt dies jeweils die gleichzeitige Substitution durch mehrere gleiche und/oder strukturell unterschiedliche Reste ein.

Substituierte Reste, wie ein substituierter Alkyl-, Alkenyl-, Alkinyl-, Aryl-, Phenyl-, Benzyl-, Heterocyclyl- und Heteroarylrest, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Alkylthio, Hydroxy, Amino, Nitro, Carboxy, Cyano, Azido, Alkoxycarbonyl, Alkylcarbonyl, Formyl, Carbamoyl, Mono- und Dialkylaminocarbonyl, substituiertes Amino, wie Acylamino, Mono- und Dialkylamino, und Alkylsulfinyl, Alkylsulfonyl und, im Falle cyclischer Reste; auch Alkyl; Haloalkyl, Alkylthio-alkyl, Alkoxy-alkyl, gegebenfalls substituiertes Mono- und Dialkyl-aminoalkyl und Hydroxy-alkyl bedeuten; im Begriff "substituierte Reste" wie substituiertes Alkyl etc. sind als Substituenten zusätzlich zu den genannten gesättigten kohlenwasserstoffhaltigen Resten entsprechende ungesättigte aliphatische und aromatische Reste, wie gegebenenfalls substituiertes Alkenyl, Alkinyl, Alkenyloxy, Alkinyloxy, Phenyl, Phenoxy etc. eingeschlossen. Im Falle von substituierten cyclischen Resten mit aliphatischen Anteilen im Ring werden auch cyclische Systeme mit solchen Substituenten umfaßt, die mit einer Doppelbindung am Ring gebunden sind z.B. mit einer Alkylidengruppe wie Methyliden oder Ethyliden substituiert sind.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

### Bevorzugt Substituenten für die Subtituentenebenen sind beispielsweise

Amino, Hydroxy, Halogen, Nitro, Cyano, Mercapto, Carboxy, Carbonamid, SF₅, Aminosulfonyl, Alkyl, Cycloalkyl, Alkenyl, Cycloalkenyl, Alkinyl, Monoalkyl-amino, Dialkyl-amino, N-Alkanoyl-amino, Alkoxy, Alkenyloxy, Alkinyloxy, Cycloalkoxy, Cycloalkenyloxy, Alkoxy-carbonyl, Alkenyloxy-carbonyl, Alkinyloxy-carbonyl, Aryloxycarbonyl, Alkanoyl, Alkenyl-carbonyl, Alkinyl-carbonyl, Aryl-carbonyl, Alkylthio, Cycloalkylthio, Alkenylthio, Cycloalkenylthio, Alkinylthio, Alkylsulfinyl, Alkylsulfonyl, Monoalkyl-aminosulfonyl, Dialkyl-aminosulfonyl, N-Alkyl-aminocarbonyl, N,N-Dialkylaminocarbonyl, N-Alkanoyl-amino-carbonyl, N-Alkanoyl-N-alkyl-aminocarbonyl, Aryl, Aryloxy, Benzyl, Benzyloxy, Benzylthio, Arylthio, Arylamino und Benzylamino.

Bei Resten mit C-Atomen sind solche mit 1 bis 6 Atomen, vorzugsweise 1 bis 4 C-Atomen, insbesondere 1 oder 2 C-Atomen bevorzugt. Bevorzugt sind in der Regel Substituenten aus der Gruppe Halogen, z.B. Fluor und Chlor, (C₁-C₄)Alkyl, vorzugsweise Methyl oder Ethyl, (C₁-C₄)Haloalkyl, vorzugsweise Trifluormethyl, (C₁-C₄)Alkoxy, vorzugsweise Methoxy oder Ethoxy, (C₁-C₄)Haloalkoxy, Nitro und Cyano. Besonders bevorzugt sind dabei die Substituenten Methyl, Methoxy, Fluor und Chlor.

Substituiertes Amino wie mono- oder disubstituiertes Amino bedeutet einen Rest aus der Gruppe der substituierten Aminoreste, welche beispielsweise durch einen bzw. zwei gleiche oder verschiedene Reste aus der Gruppe Alkyl, Alkoxy, Acyl und Aryl N-substituiert sind; vorzugsweise Mono- und Dialkyl-amino, Mono- und Diarylamino, Acylamino, N-Alkyl-N-arylamino, N-Alkyl-N-acylamino sowie N-Heterocyclen; dabei sind Alkylreste mit 1 bis 4 C-Atomen bevorzugt; Aryl ist dabei vorzugsweise Phenyl oder substituiertes Phenyl; für Acyl gilt dabei die weiter unten genannte Definition, vorzugsweise (C₁-C₄)Alkanoyl. Entsprechenes gilt für substituiertes Hydroxylamino oder Hydrazino.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)Alkyl, (C₁-C₄)Alkoxy, (C₁-C₄)Halogenalkyl, (C₁-C₄)Halogenalkoxy und Nitro substituiert ist, z.B. o-, m- und p-Tolyl, Dimethylphenyle, 2-, 3- und 4-Chlorphenyl, 2-, 3- und 4-Trifluormethyl- und -Trichlormethylphenyl, 2,4-, 3,5-, 2,5- und 2,3-Dichlorphenyl, o-, m- und p-Methoxyphenyl.

Acyl bedeutet einen Rest einer organischen Säure, der formal durch Abtrennen einer Hydroxygruppe an der Säurefunktion entsteht, wobei der organische Rest in der Säure auch über ein Heteroatom mit der Säurefunktion verbunden sein kann. Beispiele für Acyl sind der Rest -CO-R einer Carbonsäure HO-CO-R und Reste davon abgeleiteter Säuren wie der Thiocarbonsäure, gegebenenfalls N-substituierten Iminocarbonsäuren oder der Rest von Kohlensäuremonoestem, N-substituierter Carbaminsäure, Sulfonsäuren, Sulfinsäuren, N-substituierter Sulfonamidsäuren, Phosphonsäuren oder Phosphinsäuren.

Acyl bedeutet beispielsweise Formyl, Alkylcarbonyl wie [(C₁-C₄)Alkyl]-carbonyl, Phenylcarbonyl, Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren. Dabei können die Reste jeweils im Alkyl- oder Phenylteil noch weiter substituiert sein, beispielsweise im Alkylteil durch einen oder mehrere Reste aus der Gruppe Halogen, Alkoxy, Phenyl und Phenoxy; Beispiele für Substituenten im Phenylteil sind die bereits weiter oben allgemein für substituiertes Phenyl erwähnten Substituenten.

Acyl bedeutet vorzugsweise einen Acylrest im engeren Sinne, d. h. einen Rest einer organischen Säure, bei der die Säuregruppe direkt mit dem C-Atom eines organischen Restes verbunden ist, beispielsweise Formyl, Alkylcarbonyl wie Acetyl oder [(C₁-C₄)Alkyl]-carbonyl, Phenylcarbonyl, Alkylsulfonyl, Alkylsulfinyl und andere Reste von organischen Säuren.

Die Verbindungen der Formel (I) umfaßt auch alle Tautomeren, die durch Wasserstoffverschiebung mit den bezeichneten Verbindungen der Formel (I) im Gleichgewicht stehen.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in der allgemeinen Formel (I) nicht gesondert angegeben sind. Die durch ihre spezifische Raumform definierten möglichen Stereoisomeren, wie Enantiomere, Diastereomere, Z- und E-Isomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden. Entsprechendes gilt auch für die weiter unten genannten anderen allgemeinen Formeln.

Beispielsweise stellt das C-Atom in Formel (I), mit dem der Bicyclus an der einen Aminogruppe des Diaminotriazins gebunden ist, ein Chiralitätszentrum dar, das in der R- oder S-Konfiguration (Cahn-Ingold- Prelog-Nomenklatur) vorlieben kann.

Gegenstand der Erfindung sind deshalb insbesondere auch die bezüglich des Enantiomerenüberschusses (% ee = "enantiomeric excess") bezogen auf das genannte Chiralitätszentrum gereinigten oder angereicherten herbizid wirksamen Stereoisomere (Enantiomere bei einem Chiralitätszentrum; oder auch Enantiomere/Diastereomere, wenn mehrere Chiralitätszentren vorhanden sind).

Vor allem aus den Gründen der höheren herbiziden Wirkung, besseren Selektivität und/oder besseren Herstellbarkeit sind erfindungsgemäße Verbindungen der genannten Formel (I) oder deren Salze von besonderem Interesse, worin einzelne Reste eine der bereits genannten oder im Folgenden genannten bevorzugten Bedeutungen haben, oder insbesondere solche, worin eine oder mehrere der bereits genannten oder im Folgenden genannten bevorzugten Bedeutungen kombiniert auftreten.

Bevorzugt sind auch erfindungsgemäße Verbindungen, der allgemeinen Formel (I) und deren Salze, worin eine oder mehrere Gruppen, Symbole bzw. Reste R¹, R², R³, R⁴, R⁵, Y¹, Y², X¹, X², X³, X⁴,Y¹, Y², n und m wie in einem der genannten spezifischen Beispiele (Ausführungsbeispiele, Tabellenbeispiele) weiter unten definiert sind oder entsprechend der generischen Definition definiert sind, die zu der beispielhaft genannten Definition gehört (z.B. Ethoxy gehört zu Alkoxy oder vorzugsweise (C₁-C₆)Alkoxy, etc.).

Im Folgenden werden erfindungsgemäße Verbindungen der Formel (I) und deren Salze auch kurz als "erfindungsgemäße Verbindungen (I)" oder noch kürzer als "Verbindungen (I)" bezeichnet.

Unabhängig von den jeweils anderen Bedeutungen der Resten und Symbole aus der Gruppe R¹, R², R³, R⁴, R⁵, R⁶, R⁷, R⁸, R⁹, X¹, X², X³, X⁴, Y¹, Y², n und m, einschließlich der allgemeinen Reste der dazu entsprechenden Unterbedeutungen, und vorzugsweise in Kombination mit bevorzugten Bedeutungen von einem oder mehreren dieser Reste sind erfindungsgemäße Verbindungen (I) mit nachfolgend aufgeführten bevorzugten Bedeutungen der betreffenden Reste von besonderem Interesse.
R¹ und R² bedeuten vorzugsweise jeweils unabhängig voneinander Wasserstoff, Amino, Alkylamino oder Dialkylamino mit jeweils 1 bis 4 C-Atomen im Alkylrest oder (C₃-C₆)Cycloalkyl-amino, (C₅-C₆)Cycloalkenyl-amino, einen (acyclischen oder cyclischen) Kohlenwasserstoffrest oder Kohlenwasserstoffoxyrest mit jeweils 1 bis 10 C-Atomen, vorzugsweise mit 1 bis 6 C-Atomen, oder einen Heterocyclylrest, Heterocyclyloxyrest, Heterocyclylthiorest oder Heterocyclylaminorest mit jeweils 3 bis 9 Ringatomen, vorzugsweise 3 bis 6 Ringatomen und 1 bis 3 Heteroringatomen aus der Gruppe N, O und S, wobei jeder der sechs letztgenannten Reste unsubstituiert oder durch einen oder mehrere Reste aus der Gruppe Halogen, Hydroxy, Amino, Nitro, Formyl, Aminocarbonyl, Carboxy, Sulfonyl, Cyano, Thiocyanato, (C₁-C₆)Alkoxy, (C₂-C₆)Alkenyloxy, (C₂-C₆)Alkinyloxy, (C₁-C₆)Alkoxy-poly(alkylenoxy), Hydroxy-poly(alkylenoxy), (C₁-C₆)Alkylthio, Mono- und Di[(C₁-C₆)alkyl]amino, [(C₁-C₆)Alkyl]carbonyl, [(C₂-C₆)Alkenyl]carbonyl, [(C₂-C₆)Alkinyl]carbonyl, [(C₁-C₆)Alkoxy]carbonyl, [(C₂-C₆)Alkenyloxy]carbonyl, [(C₂-C₆)Alkinyloxy]carbonyl, Mono- und Di-[(C₁-C₆)alkyl]amino-carbonyl, Phenyl, Phenoxy, (C₃-C₆)Cycloalkyl, Phenylcarbonyl, Phenoxycarbonyl, Heterocyclyl, (C₁-C₄)Alkylsulfonyl und im Falle cyclischer Reste auch (C₁-C₄)Alkyl substituiert ist,
   wobei jeder der letztgenannten 24 Substituenten unsubstituiert oder durch einen oder mehrere Reste aus der Gruppe Halogen, CN, SCN, NO₂, (C₁-C₄)Alkoxy, (C₁-C₄)Alkylthio, (C₁-C₄)Haloalkoxy, Formyl, (C₁-C₄)Alkylcarbonyl, [(C₁-C₄)Haloalkyl]carbonyl, [(C₁-C₄)Alkoxy]carbonyl, [(C₁-C₄)Haloalkoxy]carbonyl und im Falle cyclischer Reste auch (C₁-C₄)Alkyl und (C₁-C₄)Haloalkyl substituiert ist,
   oder einen Acylrest einer organischen Säure aus der Gruppe Formyl, Aminocarbonyl, Mono- oder Di-[(C₁-C₄)alkyl]amino-carbonyl, [(C₁-C₆)Alkyl]carbonyl, [(C₂-C₆)Alkenyl]carbonyl, [(C₂-C₆)Alkinyl]carbonyl, [(C₁-C₆)Alkoxy]carbonyl, [(C₂-C₆)Alkenyloxy]carbonyl, Phenylcarbonyl, (C₁-C₆)Alkylsulfonyl, wobei jeder der letztgenannten 9 Reste im aliphatischen Teil oder im Phenylring unsubstituiert oder durch einen oder mehrere Reste aus der Gruppe Halogen, CN, SCN, NO₂, (C₁-C₄)Alkoxy, (C₁-C₄)Haloalkoxy, gegebenenfalls substituiertes und vorzugsweise unsubstituiertes Phenyl und im Falle cyclischer Reste auch (C₁-C₆)Alkyl und (C₁-C₆)Haloalkyl substituiert ist,
   wobei Heterocyclyl, wenn nicht näher definiert, auch in zusammengesetzten Resten jeweils 3 bis 9 Ringatome, vorzugsweise 3 bis 6 Ringatome, und 1 bis 3 Heteroringatome aus der Gruppe N, O und S aufweist,
   oder
   R¹ und R² bedeuten gemeinsam mit dem Stickstoffatom der Gruppe NR¹R² einen gesättigten oder ungesättigten, nicht aromatischen heterocyclischen Rest mit 3 bis 6 Ringatomen und 1 bis 4 Heteroringatomen, wobei neben dem N-Atom die gegebenenfalls weiteren Heteroringatome aus der Gruppe N, O und S ausgewählt sind und der Rest unsubstituiert oder durch einen oder mehrere Reste aus der Gruppe Halogen, CN, (C₁-C₄)Alkyl, (C₁-C₄)Haloalkyl, (C₁-C₄)Alkoxy, (C₁-C₄)Haloalkoxy, (C₁-C₄)Alkoxy-carbonyl und Oxo substituiert ist.
R¹ und R² bedeuten weiter bevorzugt jeweils unabhängig voneinander Wasserstoff, Amino, Mono- oder Di[(C₁-C₄)alkyl]amino oder (C₃-C₆)Cycloalkylamino, (C₅-C₆)Cycloalkenyl-amino, einen (acyclischen oder cyclischen) Kohlenwasserstoffrest oder Kohlenwasserstoffoxyrest mit jeweils 1 bis 10 C-Atomen, vorzugsweise mit 1 bis 6 C-Atomen, oder einen Heterocyclylrest, Heterocyclyloxyrest, Heterocyclylthiorest oder Heterocyclylaminorest mit jeweils 3 bis 9 Ringatomen, vorzugsweise 3 bis 6 Ringatomen, und 1 bis 3 Heteroringatomen aus der Gruppe N, O und S, wobei jeder der sechs letztgenannten Reste unsubstituiert oder durch einen oder mehrere Reste aus der Gruppe Halogen, Hydroxy, Amino, Nitro, Formyl, Aminocarbonyl, Carboxy, Sulfonyl, Cyano, Thiocyanato, (C₁-C₄)Alkoxy, (C₂-C₄)Alkenyloxy, (C₂-C₄)Alkinyloxy, (C₁-C₄)Alkoxy-poly(alkylenoxy), Hydroxy-poly(alkylenoxy), (C₁-C₄)Alkylthio, Mono- und Di[(C₁-C₄)alkyl]amino, [(C₁-C₄)Alkyl]carbonyl, [(C₂-C₄)Alkenyl]carbonyl, [(C₂-C₄)Alkinyl]carbonyl, [(C₁-C₄)Alkoxy]carbonyl, [(C₂-C₄)Alkenyloxy]carbonyl, [(C₂-C₄)Alkinyloxy]carbonyl, Mono- und Di-[(C₁-C₄)alkyl]amino-carbonyl, Phenyl, Phenoxy, (C₃-C₆)Cycloalkyl, Phenylcarbonyl, Phenoxycarbonyl, Heterocyclyl, (C₁-C₄)Alkylsulfonyl und im Falle cyclischer Reste auch (C₁-C₄)Alkyl substituiert ist,
   wobei jeder der letztgenannten 24 Substituenten unsubstituiert oder durch einen oder mehrere Reste aus der Gruppe Halogen, CN, SCN, NO₂, (C₁-C₄)Alkoxy, (C₁-C₄)Haloalkoxy, Formyl, (C₁-C₄)Alkylcarbonyl, [(C₁-C₄)Haloalkyl]carbonyl, [(C₁-C₄)Alkoxy]carbonyl, [(C₁-C₄)Haloalkoxy]carbonyl und im Falle cyclischer Reste auch (C₁-C₄)Alkyl und (C₁-C₄)Haloalkyl substituiert ist,
   oder einen Acrylrest einer organischen Säure aus der Gruppe Formyl, Aminocarbonyl, Mono- oder Di-[(C₁-C₄)alkyl]amino-carbonyl, [(C₁-C₄)Alkyl]carbonyl, [(C₂-C₄)Alkenyl]carbonyl, [(C₂-C₄)Alkinyl]carbonyl, [(C₁-C₄)Alkoxy]carbonyl, [(C₂-C₄)Alkenyloxy]carbonyl, Phenylcarbonyl, (C₁-C₄)Alkylsulfonyl, wobei jeder der letztgenannten 9 Reste im aliphatischen Teil oder im Phenylring unsubstituiert oder durch einen oder mehrere Reste aus der Gruppe Halogen, CN, SCN, NO₂, (C₁-C₄)Alkoxy, (C₁-C₄)Haloalkoxy, gegebenenfalls substituiertes und vorzugsweise unsubstituiertes Phenyl und im Falle cyclischer Reste auch (C₁-C₄)Alkyl und (C₁-C₄)Haloalkyl substituiert ist,
   wobei Heterocyclyl, wenn nicht näher definiert, auch in zusammengesetzten Resten jeweils 3 bis 9 Ringatome, vorzugsweise 3 bis 6 Ringatome, und 1 bis 3 Heteroringatome aus der Gruppe N, O und S aufweist,
   oder
   R¹ und R² bedeuten gemeinsam mit dem Stickstoffatom der Gruppe NR¹R² einen gesättigten oder ungesättigten, nicht aromatischen heterocyclischen Rest mit 3 bis 6 Ringatomen und 1 bis 4 Heteroringatomen, insbesondere 1 oder 2 Heteroringatomen, wobei neben dem N-Atom die gegebenenfalls weiteren Heteroringatome aus der Gruppe N, O und S ausgewählt sind und der Rest unsubstituiert oder durch einen oder mehrere Reste aus der Gruppe Halogen, CN, (C₁-C₄)Alkyl, (C₁-C₄)Haloalkyl, (C₁-C₄)Alkoxy, (C₁-C₄)Alkoxycarbonyl und Oxo substituiert ist.
R¹ und R² bedeuten weiter vorzugsweise jeweils unabhängig voneinander Wasserstoff, Amino, Alkylamino oder Dialkylamino mit jeweils 1 bis 4 C-Atomen im Alkylrest oder (C₃-C₆)Cycloalkyl-amino, (C₅-C₆)Cycloalkenyl-amino, einen (acyclischen oder cyclischen) Kohlenwasserstoffrest oder Kohlenwasserstoffoxyrest mit jeweils 1 bis 10 C-Atomen, vorzugsweise mit 1 bis 6 C-Atomen, oder einen Heterocyclylrest, Heterocyclyloxyrest, Heterocyclylthiorest oder Heterocyclylaminorest mit jeweils 3 bis 9 Ringatomen, vorzugsweise 3 bis 6 Ringatomen, und 1 bis 3 Heteroringatomen aus der Gruppe N, O und S, wobei jeder der sechs letztgenannten Reste unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkoxy, (C₁-C₄)Alkoxy-(C₁-C₄)alkoxy, Hydroxy-(C₁-C₄)alkoxy, (C₁-C₄)Alkoxy-poly(alkylenoxy), Hydroxy-poly(alkylenoxy), (C₁-C₄)Haloalkoxy, (C₁-C₄)Alkylthio, (C₁-C₄)Haloalkylthio, Mono(C₁-C₄)alkylamino, Di(C₁-C₄)alkylamino, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylaminocarbonyl, Phenyl, Phenoxy, (C₃-C₆)Cycloalkyl, Phenylcarbonyl, Phenoxycarbonyl, Heterocyclyl, wobei jeder der letztgenannten 6 Reste unsubstituiert oder durch einen oder mehrere Reste aus der Gruppe Halogen, CN, SCN, NO₂, (C₁-C₄)Alkyl, (C₁-C₄)Haloalkyl, (C₁-C₄)Alkoxy und (C₁-C₄)Haloalkoxy substituiert ist, und (C₁-C₄)Alkylsulfonyl und (C₁-C₄)Haloalkylsulfonyl und im Falle cyclischer Reste auch (C₁-C₄)Alkyl und (C₁-C₄)Haloalkyl substituiert ist,
   oder einen Acylrest einer organischen Säure aus der Gruppe Formyl, [(C₁-C₄)Alkyl]carbonyl, [(C₁-C₄)Haloalkyl]carbonyl, [(C₁-C₄)Alkoxy-(C₁-C₄)alkyl]carbonyl, [(C₂-C₄)Alkenyl]carbonyl, [(C₂-C₄)Haloalkenyl]carbonyl, [(C₁-C₄)Alkoxy]carbonyl, [(C₁-C₄)Haloalkoxy]carbonyl, [(C₁-C₄)Alkoxy-(C₁-C₄)alkoxy]carbonyl, [(C₂-C₄)Alkenyloxy]carbonyl, [(C₂-C₄)Haloalkenyloxy]carbonyl, [(C₁-C₄)Alkinyloxy]carbonyl, Phenylcarbonyl und Benzylcarbonyl, wobei jeder der beiden letztgenannten Reste im Phenylring unsubstituiert oder durch einen oder mehrere Reste aus der Gruppe Halogen, CN, SCN, NO₂, (C₁-C₄)Alkyl, (C₁-C₄)Haloalkyl, (C₁-C₄)Alkoxy, (C₁-C₄)Alkylthio und (C₁-C₄)Haloalkoxy substituiert ist, und (C₁-C₄)Alkylsulfonyl, (C₁-C₄)Haloalkylsulfonyl und [(C₁-C₄)Alkoxy-(C₁-C₄)alkoxy]sulfonyl, wobei Heterocyclyl wie sonst definiert ist.
   Außerdem bevorzugt bedeuten
R¹, R² unabhängig voneinander Wasserstoff, Amino, Formyl, (C₁-C₄)Alkyl, Cyano-(C₁-C₄)alkyl, (C₁-C₄)Alkylamino, Di-[(C₁-C₄)alkyl]-amino, (C₁-C₄)Haloalkyl, Mono-, Di- oder Poly-hydroxy-(C₁-C₄)alkyl, Hydroxy-poly-[(C₂-C₄)alkylenoxy]-(C₁-C₄)alkyl, Mono-, Di- oder Poly-(C₁-C₄)alkoxy-(C₁-C₄)alkyl, (C₁-C₄)Alkoxypoly-[(C₂-C₄)alkylenoxy]-(C₁-C₄)alkyl, (C₁-C₄) Haloalkoxy-(C₁-C₄)alkyl, (C₂-C₆)Alkenyl, (C₂-C₆)Haloalkenyl, (C₂-C₆)Alkinyl, (C₂-C₆)Haloalkinyl, oder Aminocarbonyl oder (C₁-C₄)Alkylamino-(C₁-C₄)alkyl, Di-[(C₁-C₄)alkyl]-amino-(C₁-C₄)alkyl, (C₁-C₄)Alkylamino-carbonyl, Di(C₁-C₄)alkylamino-carbonyl, (C₁-C₅)Alkanoyl, [(C₂-C₄)Alkenyl]carbonyl, [(C₁-C₄)Alkoxy]carbonyl, (C₂-C₄)Alkenyloxy-carbonyl, Aminocarbonyl-(C₁-C₄)alkyl, (C₁-C₄)Alkylamino-carbonyl-(C₁-C₄)alkyl, Di[(C₁-C₄)alkyl]amino-carbonyl-(C₁-C₄)alkyl, (C₁-C₄)Alkoxy-carbonyl-(C₁-C₄)alkyl, (C₁-C₅)Alkanoyl-(C₁-C₄)alkyl, [(C₂-C₄)Alkenyl]carbonyl-(C₁-C₄)alkyl, [(C₁-C₄)Alkoxy]carbonyl-(C₁-C₄)alkyl oder (C₂-C₄)Alkenyloxy-carbonyl-(C₁-C₄)alkyl,
   wobei jeder der letztgenannten 16 Reste unsubstituiert oder durch einen oder mehrere Reste aus der Gruppe Halogen, CN, (C₁-C₄)Alkoxy und (C₁-C₄)Haloalkoxy substituiert ist, oder
   (C₃-C₆)Cycloalkyl, (C₃-C₆)Cycloalkylamino-(C₁-C₄)alkyl, Phenyl, Phenoxy, Phenylcarbonyl, Phenoxycarbonyl, Phenylcarbonyl-(C₁-C₄)alkyl, Phenoxy-(C₁-C₄)alkyl, Phenyl-(C₁-C₄)alkyl, Heterocyclyl, Heterocyclyl-(C₁-C₄)alkyl, Heterocyclylamino, Heterocyclyloxy oder Heterocyclylthio
   oder einen der letztgenannten 14 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkylcarbonyl, (C₁-C₄)Alkoxy-carbonyl und (C₁-C₄)Alkoxy substituiert ist, wobei Heterocyclyl in den Resten jeweils 3 bis 9 Ringatome und 1 bis 3 Heteroringatome aus der Gruppe N, O und S enthält,
   oder
   R¹ und R² gemeinsam mit dem Stickstoffatom der Gruppe NR¹R² einen heterocyclischen Rest mit 3 bis 6 Ringatomen und 1 oder 2 Heteroringatomen, wobei neben dem N-Atom das gegebenenfalls weitere Heteroringatom aus der Gruppe N, O und S ausgewählt ist und der Rest unsubstituiert oder durch einen oder mehrere Reste aus der Gruppe Halogen, (C₁-C₄)Alkyl und Oxo substituiert ist.
   Weiter bevorzugt bedeuten
R¹, R² unabhängig voneinander Wasserstoff, Amino, Formyl, (C₁-C₄)Alkyl, (C₁-C₄)Alkylamino, Di-[(C₁-C₄)alkyl]-amino, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, (C₂-C₆)Alkinyl, Halo-(C₂-C₆)alkinyl, (C₁-C₄)Alkylamino-(C₁-C₄)alkyl oder Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, Aminocarbonyl, (C₁-C₄)Alkylamino-carbonyl oder einen der letztgenannten 10 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, Formyl, (C₁-C₄)Alkyl-carbonyl, (C₁-C₄)Alkoxy-carbonyl und (C₁-C₄)Alkoxy substituiert ist, wobei Heterocyclyl in den Resten vorzugsweise jeweils 3 bis 7 Ringatome und 1 bis 3 Heteroringatome aus der Gruppe N, O und S enthält.

Besonders bevorzugt bedeutet die Gruppe NR¹R² Amino, Hydrazino, Mono- oder Di-alkylamino mit 1 bis 4 C-Atomen in den Alkylresten, (C₁-C₅)Alkanoylamino, N-(C₁-C₅)A)kanoyl-N-(C₁-C₄)Alkyl-amino, [(C₁-C₄)Haloalkyl]-carbonylamino, N-[(C₁-C₄)Haloalkyl]-carbonyl-N-(C₁-C₄)alkyl-amino, [(C₁-C₄)Alkoxy-(C₁-C₄)alkyl-carbonyl]-amino, N-[(C₁-C₄)Alkoxy-(C₁-C₄)alkyl-carbonyl]-N-[(C₁-C₄)alkyl]-amino, Phenylamino, Benzylamino, Benzoylamino, Morpholino, Piperidino, Piperidin-1-yl-amino;
insbesondere Amino, Hydrazino, Methylamino, Ethylamino, Dimethylamino, Acetylamino, Propionylamino, Chloracetylamino, (2-Chlorethyl)-carbonylamino, Trifluormethylcarbonylamino oder Trichlormethylcarbonylamino; ganz besonders Amino.

Von besonderem Interesse sind auch Verbindungen (I), worin
R³ Wasserstoff, Amino, (C₁-C₆)Alkyl, (C₁-C₆)Alkoxy, (C₂-C₆)Alkenyl, (C₂-C₆)Alkinyl, (C₃-C₆)Cycloalkyl, (C₅-C₆)Cycloalkenyl, Phenyl oder Heterocyclyl, wobei jeder der acht letztgenannten Reste unsubstituiert oder durch einen oder mehrere Reste aus der Gruppe Halogen, Hydroxy, Amino, Nitro, Formyl, Carboxy, Cyano, Thiocyanato, (C₁-C₄)Alkoxy, (C₁-C₄)Alkoxy-(C₁-C₄)alkoxy, Hydroxy-(C₁-C₄)alkoxy, (C₁-C₄)Alkoxy-poly(alkylenoxy), Hydroxypoly(alkylenoxy), (C₁-C₄)Haloalkoxy, (C₁-C₄)Alkylthio, (C₁-C₄)Haloalkylthio, Mono(C₁-C₄)alkylamino, Di(C₁-C₄)alkylamino, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, Phenyl, Phenoxy, (C₃-C₆)Cycloalkyl, Phenylcarbonyl, Phenoxycarbonyl, Heterocyclyl, wobei jeder der letztgenannten 6 Reste unsubstituiert oder durch einen oder mehrere Reste aus der Gruppe Halogen, CN, SCN, NO₂ (C₁-C₄)Alkyl, (C₁-C₄)Haloalkyl, (C₁-C₄)Alkoxy und (C₁-C₄)Haloalkoxy substituiert ist, und (C₁-C₄)Alkylsulfonyl und (C₁-C₄)Haloalkylsulfonyl und im Falle cyclischer Reste auch (C₁-C₄)Alkyl und (C₁-C₄)Haloalkyl substituiert ist,
   oder einen Acylrest der Formel -B*-A*, worin
   A* Wasserstoff oder (C₁-C₆)Alkyl, (C₂-C₆)Alkenyl, (C₂-C₆)Alkinyl, (C₃-C₆)Cycloalkyl, (C₅-C₆)Cycloalkenyl oder Phenyl, wobei jeder der sechs letztgenannten Reste unsubstituiert oder durch einen oder mehrere Reste aus der Gruppe Halogen, Hydroxy, Amino, Nitro, Formyl, Carboxy, Cyano, Thiocyanato, (C₁-C₄)Alkoxy, (C₁-C₄)Haloalkoxy, (C₁-C₄)Alkylthio, Mono(C₁-C₄)alkylamino, Di(C₁-C₄)alkylamino, Phenyl und (C₃-C₆)Cycloalkyl, wobei jeder der letztgenannten 2 Reste unsubstituiert oder durch einen oder mehrere Reste aus der Gruppe Halogen, CN, SCN, NO₂, (C₁-C₄)Alkyl, (C₁-C₄)Haloalkyl, (C₁-C₄)Alkoxy und (C₁-C₄)Haloalkoxy substituiert ist, und im Falle cyclischer Reste auch (C₁-C₄)Alkyl und (C₁-C₄)Haloalkyl substituiert ist, und
   B* eine divalente Gruppe der Formel -CO-, -CO-O-, -CO-NR'-, -S(O)ₚ- oder -S(O)ₚ-O-, wobei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 4 C-Atomen ist,
   bedeuten,
wobei -B*-A* inklusive Substituenten 1 bis 12, vorzugsweise 1 bis 10 C-Atome aufweist und Heterocyclyl allgemein wie zu R¹ und R² definiert ist,
bedeutet.

Auch bedeutet vorzugsweise
R³ Wasserstoff, Amino, Formyl, (C₁-C₄)Alkyl, Cyano-(C₁-C₄)alkyl, (C₁-C₄)Haloalkyl, Mono-, Di- oder Poly-hydroxy-(C₁-C₄)alkyl, Hydroxy-poly-[(C₂-C₄)alkylenoxy]-(C₁-C₄)alkyl, Mono-, Di- oder Poly-(C₁-C₄)alkoxy-(C₁-C₄)alkyl, (C₁-C₄)Alkoxy-poly-[(C₂-C₄)alkylenoxy]-(C₁-C₄)alkyl, (C₁-C₄) Haloalkoxy-(C₁-C₄)alkyl, (C₂-C₆)Alkenyl, (C₂-C₆)Haloalkenyl, (C₂-C₆)Alkinyl, (C₂-C₆)Haloalkinyl oder Aminocarbonyl oder (C₁-C₄)Alkylamino-(C₁-C₄)alkyl, Di-[(C₁-C₄)alkyl]-amino-(C₁-C₄)alkyl, (C₁-C₄)Alkylamino-carbonyl, Di(C₁-C₄)alkylamino-carbonyl, (C₁-C₅)Alkanoyl, [(C₂-C₄)Alkenyl]carbonyl, [(C₁-C₄)Alkoxy]carbonyl, (C₂-C₄)Alkenyloxy-carbonyl, Aminocarbonyl-(C₁-C₄)alkyl, (C₁-C₄)Alkylamino-carbonyl-(C₁-C₄)alkyl, Di[(C₁-C₄)alkyl]amino-carbonyl-(C₁-C₄)alkyl, (C₁-C₄)Alkoxy-carbonyl-(C1-C₄)alkyl, (C₁-C₅)Alkanoyl-(C₁-C₄)alkyl, [(C₂-C₄)Alkenyl]carbonyl-(C₁-C₄)alkyl, [(C₁-C₄)Alkoxy]carbonyl-(C₁-C₄)alkyl oder (C₂-C₄)Alkenyloxy-carbonyl-(C₁-C₄)alkyl, wobei jeder der letztgenannten 16 Reste unsubstituiert oder durch einen oder mehrere Reste aus der Gruppe Halogen, CN, (C₁-C₄)Alkoxy und (C₁-C₄)Haloalkoxy substituiert ist, oder (C₃-C₆)Cycloalkyl, (C₃-C₆)Cycloalkylamino-(C₁-C₄)alkyl, Phenyl, Phenylcarbonyl, Phenoxycarbonyl, Phenylcarbonyl-(C₁-C₄)alkyl, Phenoxy-(C₁-C₄)alkyl, Phenyl-(C₁-C₄)alkyl, Heterocyclyl, Heterocyclyl-(C₁-C₄)alkyl oder einen der letztgenannten 10 Reste, der im acyclischen Teil oder, vorzugsweise, im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl und (C₁-C₄)Alkoxy substituiert ist, wobei Heterocyclyl in den Resten jeweils 3 bis 9 Ringatome und 1 bis 3 Heteroringatome aus der Gruppe N, O und S enthält.

Dabei sind Verbindungen (1) bevorzugt, worin
R³ Wasserstoff, (C₁-C₆)Alkyl, (C₃-C₆)Cycloalkyl oder Phenyl, wobei jeder der drei letztgenannten Reste unsubstituiert oder durch einen oder mehrere Reste aus der Gruppe Halogen, Hydroxy, Amino, Formyl, Cyano, Thiocyanato, (C₁-C₄)Alkoxy, (C₁-C₄)Haloalkoxy, (C₁-C₄)Alkythio, Mono(C₁-C₄)alkylamino, Di(C₁-C₄)alkylamino und Phenyl, das unsubstituiert oder durch einen oder mehrere Reste aus der Gruppe Halogen, NO₂ (C₁-C₄)Alkyl, (C₁-C₄)Haloalkyl, (C₁-C₄)Alkoxy und (C₁-C₄)Haloalkoxy substituiert ist, und im Falle cyclischer Reste auch (C₁-C₄)Alkyl und (C₁-C₄)Haloalkyl substituiert ist,
   oder einen Acylrest der Formel -B*-A*, worin
   A* Wasserstoff oder (C₁-C₆)Alkyl oder Phenyl, wobei jeder der beiden letztgenannten Reste unsubstituiert oder durch einen oder mehrere Reste aus der Gruppe Halogen, (C₁-C₄)Alkoxy, (C₁-C₄)Haloalkoxy, Phenyl und (C₃-C₆)Cycloalkyl, wobei jeder der letztgenannten 2 Reste unsubstituiert oder durch einen oder mehrere Reste aus der Gruppe Halogen, (C₁-C₄)Alkyl, (C₁-C₄)Haloalkyl, (C₁-C₄)Alkoxy und (C₁-C₄)Haloalkoxy substituiert ist, und im Falle cyclischer Reste auch (C₁-C₄)Alkyl und (C₁-C₄)Haloalkyl substituiert ist, und
   B* eine divalente Gruppe der Formel -CO-, -CO-O-, -S(O)ₚ-, wobei p = 0,1 oder 2 ist und R' Wasserstoff oder Alkyl mit 1 bis 4 C-Atomen bedeutet, bedeuten,
   wobei -B*-A* inklusive Substituenten 1 bis 12, vorzugsweise 1 bis 10 C-Atome aufweist,
bedeutet.

Beispiele für bevorzugte Acylreste der Formel -B*-A* sind (C₁-C₅)Alkanoyl, [(C₁-C₄)Haloalkyl]carbonyl, [(C₂-C₄)Alkenyl]carbonyl, [(C₂-C₄)Haloalkenyl]carbonyl, (C₁-C₄)Alkoxy-(C₁-C₄)alkyl-carbonyl, [(C₁-C₄)Alkoxy]carbonyl, [(C₁-C₄)Haloalkoxy]carbonyl, [(C₂-C₄)Haloalkenyloxy]carbonyl, [(C₁-C₄)Haloalkoxy]carbonyl, Phenylcarbonyl, Phenoxycarbonyl, Benzylcarbonyl oder Benzyloxycarbonyl, wobei jeder der letztgenannten 4 Reste im Phenylring unsubstituiert oder durch einen oder mehrere Reste aus der Gruppe Halogen, CN, SCN, NO₂ (C₁-C₄)Alkyl, (C₁-C₄)Haloalkyl, (C₁-C₄)Alkoxy und (C₁-C₄)Haloalkoxy substituiert ist, oder (C₁-C₄)Alkylsulfonyl oder (C₁-C₄)Haloalkylsulfonyl.

Besonders bevorzugt bedeutet
R³ Wasserstoff, Amino, Formyl, (C₁-C₄)Alkyl, Cyano-(C₁-C₄)alkyl, (C₁-C₄)Alkoxy-(C₁-C₄)alkyl, (C₂-C₆)Alkenyl, Halo-(C₂-C₆)alkenyl, (C₂-C₆)Alkinyl oder Phenyl, (C₁-C₄)Alkylcarbonyl, (C₁-C₄)Alkoxycarbonyl, Aminocarbonyl, Phenoxy-(C₁-C₄)alkyl, Phenyl-(C₁-C₄)alkyl oder einen der letztgenannten 6 Reste, der im acyclischen Teil oder, vorzugsweise, im cyclischen Teil durch einen oder mehrere Reste aus der Gruppe Halogen, (C₁-C₄)Alkyl und (C₁-C₄)Alkoxy substituiert ist;
   insbesondere Wasserstoff, Methyl, Ethyl, Acetyl, Methoxyacetyl, Formyl, Methoxymethyl, Methoxyethyl, Benzyl, 4-Chlorbenzyl, Benzoyl, Phenyl oder 4-Chlorphenyl;
   ganz besonders Wasserstoff.

Von besonderem Interesse sind auch Verbindungen (I), worin
R⁴ einen Rest der Formel -Z¹-R⁶, wobei Z¹ und R⁶ wie unten angegeben definiert sind,
R⁵ jeweils unabhängig voneinander Halogen, CN, SCN, NO₂, einen Rest der Formel -Z²-R⁷, wobei Z² und R⁷ wie unten angegeben definiert sind,
   oder
   zwei benachbarte Reste R⁵ gemeinsam einen ankondensierten Cyclus mit 4 bis 6 Ringatomen, der carbocyclisch ist oder 1 bis 3 Heteroringatome aus der Gruppe O, S und N enthält und der unsubstituiert oder durch einen oder mehrere Reste aus der Gruppe Halogen, (C₁-C₄)Alkyl und Oxo substituiert ist,
   und
R⁶ und R⁷, R⁸, R⁹ jeweils unabhängig voneinander Wasserstoff, ausgenommen R⁷ = Wasserstoff, wenn Z² = direkte Bindung, oder einen acyclischen Kohlenwasserstoffrest mit 1 bis 10 C-Atomen, vorzugsweise (C₁-C₆)Alkyl, (C₂-C₆)Alkenyl oder (C₂C₆)Alkinyl, oder einen cyclischen Kohlenwasserstoffrest mit 3 bis 6 C-Atomen, vorzugsweise (C₃-C₆)Cycloalkyl oder (C₅-C₆)Cycloalkenyl, oder einen heterocyclischen Rest, vorzugsweise mit 3 bis 6 Ringatomen und 1 bis 3 Heteroringatomen aus der Gruppe N, O und S, wobei jeder der letztgenannten kohlenstoffhaltigen Reste unsubstituiert oder durch einen oder mehrere Reste aus der Gruppe Halogen, Hydroxy, Amino, Nitro, Formyl, Aminocarbonyl, Carboxy, Sulfonyl, Cyano, Thiocyanato, (C₁-C₆)Alkoxy, (C₂-C₆)Alkenyloxy, (C₂-C₆)Alkinyloxy, (C₁-C₆)Alkoxypoly(alkylenoxy), Hydroxy-poly(alkylenoxy), (C₁-C₆)Alkylthio, Mono- und Di[(C₁-C₆)alkyl]amino, [(C₁-C₆)Alkyl]carbonyl, [(C₂-C₆)Alkenyl]carbonyl, [(C₂-C₆)Alkinyl]carbonyl, [(C₁-C₆)Alkoxy]carbonyl, [(C₂-C₆)Alkenyloxy]carbonyl, [(C₂-C₆)Alkinyloxy]carbonyl, Mono- und Di-[(C₁-C₆)alkyl]amino-carbonyl, Phenyl, Phenoxy, (C₃-C₆)Cycloalkyl, Phenylcarbonyl, Phenoxycarbonyl, Heterocyclyl, (C₁-C₄)Alkylsulfonyl und im Falle cyclischer Reste auch (C₁-C₄)Alkyl substituiert ist,
   wobei jeder der letztgenannten 24 Substituenten unsubstituiert oder durch einen oder mehrere Reste aus der Gruppe Halogen, CN, SCN, NO₂, (C₁-C₄)Alkoxy, (C₁-C₄)Alkylthio, (C₁-C₄)Haloalkoxy, Formyl, (C₁-C₄)Alkylcarbonyl, [(C₁-C₄)Haloalkyl]carbonyl, [(C₁-C₄)Alkoxy]carbonyl, [(C₁-C₄)Haloalkoxy]carbonyl und im Falle cyclischer Reste auch (C₁-C₄)Alkyl und (C₁-C₄)Haloalkyl substituiert ist, und vorzugsweise
R⁶ und R⁷, R⁸, R⁹ jeweils unabhängig voneinander Wasserstoff, ausgenommen R⁷ = Wasserstoff, wenn Z² = direkte Bindung, oder einen acyclischen Kohlenwasserstoffrest mit 1 bis 10 C-Atomen, vorzugsweise (C₁-C₆)Alkyl, (C₂-C₆)Alkenyl oder (C₂-C₆)Alkinyl, oder einen cyclischen Kohlenwasserstoffrest mit 3 bis 6 C-Atomen, vorzugsweise (C₃-C₆)Cycloalkyl oder (C₅-C₆)Cycloalkenyl, oder einen heterocyclischen Rest, vorzugsweise mit 3 bis 6 Ringatomen und 1 bis 3 Heteroringatomen aus der Gruppe N, O und S, wobei jeder der letztgenannten kohlenstoffhaltigen Reste unsubstituiert oder durch einen oder mehrere Reste aus der Gruppe Halogen, Hydroxy, Amino, Nitro, Formyl, Carboxy, Sulfonyl, Cyano, Thiocyanato, (C₁-C₄)Alkoxy, (C₁-C₄)Alkoxy-(C₁-C₄)alkoxy, Hydroxy-(C₁-C₄)alkoxy, (C₁-C₄)Alkoxy-poly(alkylenoxy), Hydroxy-poly(alkylenoxy), (C₁-C₄)Haloalkoxy, (C₁-C₄)Alkylthio, (C₁-C₄)Haloalkylthio, Mono(C₁-C₄)alkylamino, Di(C₁-C₄)alkylamino, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, Phenyl, Phenoxy, (C₃-C₆)Cycloalkyl, Phenylcarbonyl, Phenoxycarbonyl, Heterocyclyl, wobei jeder der letztgenannten 6 Reste unsubstituiert oder durch einen oder mehrere Reste aus der Gruppe Halogen, CN, SCN, NO₂, (C₁-C₄)Alkyl, (C₁-C₄)Haloalkyl, (C₁-C₄)Alkoxy und (C₁-C₄)Haloalkoxy substituiert ist, und (C₁-C₄)Alkylsulfonyl und (C₁-C₄)Haloalkylsulfonyl und im Falle cyclischer Reste auch (C₁-C₄)Alkyl und (C₁-C₄)Haloalkyl substituiert ist, und jeweils
Z¹ und die Gruppen Z² jeweils unabhängig voneinander eine direkte Bindung oder eine divalente Gruppe der Formel -O-, -S(O)ₚ-, -S(O)ₚ-O-, -O-S(O)ₚ-, -CO-, -O-CO-, -CO-O-, -NR'-, -O-NR'-, -NR'-O-, -NR'-CO-, -CO-NR'- bedeuten und dabei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 6 C-Atomen ist, und vorzugsweise
Z¹ und die Gruppen Z² jeweils unabhängig voneinander eine direkte Bindung oder eine divalente Gruppe der Formel -O-, -S-, -SO₂-, -CO-, -O-CO-, -CO-O-, -NR'-, -NR'-CO-, -CO-NR'- bedeuten und dabei R' Wasserstoff, (C₁-C₄)Alkyl oder Alkanoyl mit 1 bis 4 C-Atomen ist,
bedeuten,
wobei Heterocyclyl in den Resten jeweils 3 bis 9, vorzugsweise 3 bis 6 Ringatome, und 1 bis 3 Heteroringatome aus der Gruppe N, O und S aufweist.

Außerdem bevorzugt bedeutet
R⁴ Wasserstoff, Formyl, Carboxy, Cyano, Thiocyanato, Aminocarbonyl, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 6 Ringgliedern, wobei die cyclischen Gruppen in den letztgenannten 3 Resten unsubstituiert oder durch einen oder mehrere, vorzugsweise bis zu drei Reste, aus der Gruppe (C₁-C₄)Alkyl, Halogen und Cyano substituiert sind, oder
   Phenyl, Phenoxy, Phenylcarbonyl, Phenylcarbonyl-(C₁-C₄)alkyl, Phenoxycarbonyl, (C₁-C₄)Alkyl-carbonyl, (C₁-C₄)Alkoxy-carbonyl, (C₁-C₄)Alkylamino-carbonyl, (C₁-C₄)Alkoxy-carbonyl-(C₁-C₄)alkyl, (C₁-C₄)Alkylamino-carbonyl-(C₁-C₄)alkyl, Phenoxy-(C₁-C₄)alkyl, Phenyl-(C₁-C₄)alkyl, Heterocyclyl, Heterocyclylamino, Heterocyclyloxy, Heterocyclylthio, [(C₁-C₅)Alkanoylamino, N-[(C₁-C₅)Alkanoyl]-N-[(C₁-C₄)alkyl]-amino, [(C₂-C₄)Alkenyl]carbonylamino, [(C₂-C₄)Alkinyl]carbonylamino, [(C₁-C₄)Alkoxy]carbonylamino, [(C₂-C₄)Alkenyloxy]carbonylamino, [(C₂-C₄)Alkinyloxy]carbonylamino, Phenylcarbonylamino, Phenoxycarbonylamino, (C₁-C₄)Alkylsulfonyl, (C₂-C₄)Alkenylsulfonyl oder einen der letztgenannten 27 Reste, der im acyclischen Teil oder, vorzugsweise, im cyclischen Teil durch einen oder mehrere Reste aus der Gruppe Halogen, Nitro, Cyano, (C₁-C₄)Alkoxy, (C₁-C₄)Alkylthio, (C₁-C₄)Haloalkoxy, Formyl, (C₁-C₄)Alkyl-carbonyl, (C₁-C₄)Alkoxy-carbonyl, (C₁-C₄)Alkoxy und im Falle cyclischer Teile auch (C₁-C₄)Alkyl und (C₁-C₄)Haloalkyl substituiert ist,
   wobei Heterocyclyl in den Resten jeweils 3 bis 9 Ringatome und 1 bis 3 Heteroringatome aus der Gruppe N, O und S aufweist.

Weiter bevorzugt bedeutet:
R⁴ Wasserstoff, (C₁-C₄)Alkyl, Cyano-(C₁₋C₄)alkyl, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)Alkenyl, (C₂-C₆)Alkinyl, (C₁-C₄)Alkylamino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere, vorzugsweise bis zu drei Reste, aus der Gruppe (C₁-C₄)Alkyl, Halegen und Cyano substituiert sind, oder
   Phenyl, Phenoxy-(C₁-C₄)alkyl, Phenyl-(C₁-C₄)alkyl, Heterocyclyl, oder einen der letztgenannten 4 Reste, der im acyclischen Teil oder, vorzugsweise, im cyclischen Teil durch einen oder mehrere Reste aus der Gruppe Halogen, Nitro, Cyano, (C₁-C₄)Alkoxy, (C₁-C₄)Alkylthio, (C₁-C₄)Haloalkoxy, (C₁-C₄)Alkylcarbonyl, (C₁-C₄)Alkoxy-carbonyl, (C₁-C₄)Alkoxy und im Falle cyclischer Teile auch (C₁-C₄)Alkyl und (C₁-C₄)Haloalkyl substituiert ist,
   wobei Heterocyclyl in den Resten jeweils 3 bis 9 Ringatome und 1 bis 3 Heteroringatome aus der Gruppe N, O und S enthält;
insbesondere R⁴ = Wasserstoff oder (C₁-C₄)Alkyl.

Von besonderem Interesse sind außerdem Verbindungen (I), worin
R⁵ wenn n = 1 ist, und die Reste R⁵, jeweils unabhängig voneinander, wenn n größer als 1 ist, Halogen, Hydroxy, Amino, Nitro, Formyl, Carboxy, Cyano, Thiocyanato, Aminocarbonyl, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁₋C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 6 Ringgliedern, wobei die cyclischen Gruppen in den letztgenannten 3 Resten unsubstituiert oder durch einen oder mehrere Reste, vorzugsweise bis zu drei Reste, aus der Gruppe (C₁-C₄)Alkyl, Halogen und Cyano substituiert sind, oder Phenyl, Phenoxy, Phenylcarbonyl, Phenylcarbonyloxy, Phenylcarbonyl-(C₁-C₄)alkyl, Phenoxycarbonyl, (C₁-C₄)Alkyl-carbonyl, (C₁-C₄)Alkoxy-carbonyl, (C₁-C₄)Alkylamino-carbonyl, (C₁-C₄)Alkoxy-carbonyl-(C₁-C₄)alkyl, (C₁-C₄)Alkylamino-carbonyl-(C₁-C₄)alkyl, Phenoxy-(C₁-C₄)alkyl, Phenyl-(C₁-C₄)alkyl, Heterocyclyl, Heterocyclylamino, Heterocyclyloxy, Heterocyclylthio, (C₁-C₅)Alkanoylamino, N-[(C₁-C₅)Alkanoyl]-N-[(C₁-C₄)alkyl]-amino, [(C₂-C₄)Alkenyl]carbonylamino, [(C₂-C₄)Alkinyl]carbonylamino, [(C₁-C₄)Alkoxy]carbonylamino, [(C₂-C₄)Alkenyloxy]carbonylamino, [(C₂-C₄)Alkinyloxy]carbonylamino, Phenylcarbonylamino, Phenoxycarbonylamino, (C₁-C₄)Alkylsulfonyl, (C₂-C₄)Alkenylsulfonyl oder einen der letztgenannten 28 Reste, der im acyclischen Teil oder, vorzugsweise, im cyclischen Teil durch einen oder mehrere Reste aus der Gruppe Halogen, Nitro, Cyano, (C₁-C₄)Alkoxy, (C₁-C₄)Alkylthio, (C₁-C₄)Haloalkoxy, Formyl, (C₁-C₄)Alkyl-carbonyl, (C₁-C₄)Alkoxy-carbonyl, (C₁-C₄)Alkoxy und im Falle cyclischer Teile auch (C₁-C₄)Alkyl und (C₁-C₄)Haloalkyl substituiert ist,
   wobei Heterocyclyl in den Resten jeweils 3 bis 9 Ringatome und 1 bis 3 Heteroringatome aus der Gruppe N, O und S enthält,
   oder
   zwei benachbarte Reste R⁵ gemeinsam einen ankondensierten Cyclus mit 4 bis 6 Ringatomen, der carbocyclisch ist oder 1 bis 3 Heteroringatome aus der Gruppe O S und N enthält und der unsubstituiert oder durch einen oder mehrere Reste aus der Gruppe Halogen, (C₁-C₄)Alkyl und Oxo substituiert ist,
bedeutet bzw. bedeuten.

Weiter bevorzugt bedeutet:
R⁵ wenn n =1 ist, und die Reste R⁵, jeweils unabhängig voneinander, wenn n größter als 1 ist, Halogen, Hydroxy, Amino, Nitro, Formyl, Carboxy, Cyano, Thiocyanato, Aminocarbonyl, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, (C₂-C₆)Alkenyl, (C₂-C₆)Alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste, vorzugsweise bis zu drei Reste, aus der Gruppe (C₁-C₄)Alkyl, Halogen und Cyano substituiert sind, oder
   Phenyl, Phenoxy, Phenylcarbonyl, Phenylcarbonyloxy, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alkyl-carbonyl, (C₁-C₄)Alkoxy-carbonyl, (C₁-C₄)Alkylaminocarbonyl, Phenoxy-(C₁-C₄)alkyl, Phenyl(C₁-C₄)alkyl, Heterocyclyl, Heterocyclylamino, Heterocyclyloxy, Heterocyclylthio, (C₁-C₅)Alkanoylamino, N-[(C₁-C₅)Alkanoyl]-N-[(C₁-C₄)alkyl]-amino, Phenylcarbonylamino, Phenoxycarbonylamino, (C₁-C₄)Alkylsulfonyl, (C₂-C₄)Alkenylsulfonyl oder einen der letztgenannten 22 Reste, der im acyclischen Teil oder, vorzugsweise, im cyclischen Teil durch einen oder mehrere Reste aus der Gruppe Halogen, Nitro, Cyano, (C₁-C₄)Alkoxy, (C₁-C₄)Alkylthio, (C₁-C₄)Alkylcarbonyl, (C₁-C₄)Alkoxy-carbonyl, (C₁-C₄)Alkoxy und im Falle cyclischer Teile auch (C₁-C₄)Alkyl und (C₁-C₄)Haloalkyl substituiert ist,
   wobei Heterocyclyl in den Resten jeweils 3 bis 6 Ringatome und 1 bis 3 Heteroringatome aus der Gruppe N, O und S enthält,
   oder
   zwei benachbarte Reste R⁵ gemeinsam einen ankondensierten Cyclus mit 4 bis 6 Ringatomen, der carbocyclisch ist oder 1 bis 3 Heteroringatome aus der Gruppe O S und N enthält und der unsubstituiert oder durch einen oder mehrere Reste aus der Gruppe Halogen, (C₁-C₄)Alkyl und Oxo substituiert ist,

Weiter bevorzugt bedeutet:
R⁵ wenn n =1 ist, und die Reste R⁵, jeweils unabhängig voneinander, wenn n größer als 1 ist, Halogen, Hydroxy, Amino, Nitro, Formyl, Cyano, Aminocarbonyl, (C₁-C₄)Monoalkylaminocarbonyl, (C₁-C₄)Dialkylaminocarbonyl, (C₁-C₄)Alkyl, Cyano-(C₁-C₄)alkyl, (C₁-C₄)Alkoxy, (C₁-C₄)Alkylamino, Halo-(C₁-C₄)alkyl, Hydroxy-(C₁-C₄)alkyl, (C₁-C₄)Alkoxy-(C₁-C₄)alkyl, (C₂-C₄)Alkenyl, (C₂-C₄)Alkinyl, (C₁-C₄)Alkylamino-(C₁-C₄)alkyl, Di-[(C₁-C₄)alkyl]-amino-(C₁-C₄)alkyl, oder [(C₁-C₄)Alkyl]carbonyl, [(C₁-C₄)Haloalkyl]carbonyl, [(C₁-C₄)Alkoxy-(C₁-C₄)alkyl]carbonyl, Formylamino, [(C₁-C₄)Alkyl]carbonylamino, [(C₁-C₄)Haloalkyl]carbonylamino,
   Phenyl, Phenoxy, Phenylcarbonyl, Phenylcarbonylamino, Heterocyclyl oder einen der letztgenannten 5 Reste, der durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist;

insbesondere R⁵ = Halogen, vorzugsweise F, Cl oder Br, Hydroxy, Formyl, Cyano, (C₁-C₄)Alkyl, z. B. Methyl, Ethyl oder Isopropyl, oder (C₁-C₄)Alkoxy, z. B. Methoxy, Ethoxy oder Isopropoxy, oder (C₁-C₄)Alkylamino, (C₁-C₄)Haloalkyl, (C₂-C₄)Alkenyl, (C₂-C₄)Alkinyl, [(C₁-C₄)Alkyl]carbonyl, z. B. Acetyl, [(C₁-C₄)Haloalkyl]carbonyl, z. B. Trifluoracetyl, oder Formylamino, [(C₁-C₄)Alkyl]carbonylamino oder Benzoylamino.

Die besonders bevorzugten Bedeutungen für R⁶ und R⁷ ergeben sich entsprechend aus den bevorzugten Bedeutungen für R⁴ und R⁵.

Von besonderem Interesse sind auch Verbindungen (I), worin
R⁸ und R⁹ jeweils unabhängig voneinander Wasserstoff, Formyl, Carboxy, Cyano, Thiocyanato, Aminocarbonyl, (C₁-C₄)Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 6 Ringgliedern, wobei die cyclischen Gruppen in den letztgenannten 3 Resten unsubstituiert oder durch einen oder mehrere, vorzugsweise bis zu drei Reste, aus der Gruppe (C₁-C₄)Alkyl, Halogen und Cyano substituiert sind, oder Phenyl, Phenoxy, Phenylcarbonyl, Phenylcarbonyl(C₁-C₄)alkyl, Phenoxycarbonyl, (C₁-C₄)Alkyl-carbonyl, (C₁-C₄)Alkoxy-carbonyl, (C₁-C₄)Alkylamino-carbonyl, (C₁-C₄)Alkoxy-carbonyl-(C₁-C₄)alkyl, (C₁-C₄)Alkylamino-carbonyl-(C₁-C₄)alkyl, Phenoxy-(C₁-C₄)alkyl, Phenyl-(C₁-C₄)alkyl, Heterocyclyl-, Heterocyclylamino, Heterocyclyloxy, Heterocyclylthio, [(C₁-C₅)Alkanoylamino, N-[(C₁-C₅)Alkanoyl]-N-[(C₁-C₄)alkyl]-amino, [(C₂-C₄)Alkenyl]carbonylamino, [(C₂-C₄)Alkinyl]carbonylamino, [(C₁-C₄)Alkoxy]carbonylamino, [(C₂-C₄)Alkenyloxy]carbonylamino, [(C₂-C₄)Alkinyloxy]carbonylamino, Phenylcarbonylamino, Phenoxycarbonylamino, (C₁-C₄)Alkylsulfonyl, (C₂-C₄)Alkenylsulfonyl
oder einen der letztgenannten 27 Reste, der im acyclischen Teil oder, vorzugsweise, im cyclischen Teil durch einen oder mehrere Reste aus der Gruppe Halogen, Nitro, Cyano, (C₁-C₄)Alkoxy, (C₁-C₄)Alkylthio, (C₁-C₄)Haloalkoxy, Formyl, (C₁-C₄)Alkyl-carbonyl, (C₁-C₄)Alkoxy-carbonyl, (C₁-C₄)Alkoxy und im Falle cyclischer Teile auch (C₁-C₄)Alkyl und (C₁-C₄)Haloalkyl substituiert ist,
bedeuten, wobei Heterocyclyl in den Resten vorzugsweise jeweils 3 bis 9 Ringatome und 1 bis 3 Heteroringatome aus der Gruppe N, O und S aufweist.

Weiter bevorzugt bedeuten R⁸ und R⁹ jeweils unabhängig voneinander Wasserstoff, (C₁-C₄)Alkyl, Cyano-(C₁-C₄)alkyl, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₆)Alkenyl, (C₂-C₆)Alkinyl, (C₁-C₄)Alkylamino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern,
wobei die cyclischen Gruppen in den letztgenannten 3 Resten unsubstituiert oder durch einen oder mehrere, vorzugsweise bis zu drei Reste, aus der Gruppe (C₁-C₄)Alkyl, Halogen und Cyano substituiert sind, oder
Phenyl, Phenoxy-(C₁-C₄)alkyl, Phenyl-(C₁-C₄)alkyl, Heterocyclyl, oder einen der letztgenannten 4 Reste, der im acyclischen Teil oder, vorzugsweise, im cyclischen Teil durch einen oder mehrere Reste aus der Gruppe Halogen, Nitro, Cyano, (C₁-C₄)Alkoxy, (C₁-C₄)Alkylthio, (C₁-C₄)Haloalkoxy, (C₁-C₄)Alkyl-carbonyl, (C₁-C₄)Alkoxycarbonyl, (C₁-C₄)Alkoxy und im Falle cyclischer Teile auch (C₁-C₄)Alkyl und (C₁-C₄)Haloalkyl substituiert ist,
wobei Heterocyclyl in den Resten vorzugsweise jeweils 3 bis 9 Ringatome und 1 bis 3 Heteroringatome aus der Gruppe N, O und S enthält;
insbesondere R⁸ und R⁹ unabhängig voneinander Wasserstoff oder (C₁-C₄)Alkyl.

Von besonderem Interesse sind auch Verbindungen (I), worin
X¹, X², X³, X⁴ jeweils unabhängig voneinander ein Kohlenstoffatom, das mit einem Wasserstoffatom oder einer der definierten Substituenten R⁵ substituiert ist, oder ein Stickstoffatom oder zwei benachbarte Symbole X¹, X², X³ und X⁴ jeweils gemeinsam eine divalente Gruppe der Formel -O-, -S-, -NH- oder -NR-, worin R wie für R³ definiert ist und vorzugsweise Wasserstoff oder (C₁-C₄)Alkyl bedeutet, vorausgesetzt die Gruppen X¹, X², X³, X⁴ bilden zusammen mit der verknüpften C₂-Einheit des ankondensierten Rings einen carbocyclischen oder heterocyclischen aromatischen fünfgliedrigen oder sechsgliedrigen Ring,
(Y¹)ₘ m divalente Gruppen Y¹, wobei jede Gruppe Y¹ unabhängig von anderen Resten Y¹ eine Gruppe der Formel -O-, -CO-, -C(=NR*)-, -S(O)q-, -NR*- oder -N(O)-, wobei q = 0, 1 oder 2 ist und R* wie für R³ definiert ist und vorzugsweise Wasserstoff, (C₁-C₄)Alkyl, Benzyl oder Phenyl bedeutet, insbesondere Wasserstoff, (C₁-C₄)Alkyl oder Phenyl ist, oder eine Gruppe der bereits definierten Formel CR⁸R⁹ bedeutet, und
Y¹ vorzugsweise eine Gruppe der Formel CR⁸R⁹ bedeutet, insbesondere CH₂, CH(CH₃), CH(C₂H₅), CH(CH₃)₂ oder CH(C₆H₅) und m = 0, 1, 2 oder 3, insbesondere m = 1 oder 2 bedeuten und
(Y¹)ₘ vorzugweise CH₂, CH₂CH₂, CH₂CH₂CH₂, CH(CH₃), CH(CH₃)CH₂, CH₂CH(CH₃), CH(CH₃)₂CH₂, CH(C₂H₅)CH₂, oder CH₂CH(C₆H₅),
Y² eine Gruppe wie für Y¹ definiert oder eine direkte Bindung, vorzugsweise eine direkte Bindung oder eine Gruppe der Formel -O-, -S-, CH₂, CH(CH₃) oder (C₁-C₄)Alkylamino, beispielsweise N(CH₃), N(C₂H₅), N(n-C₃H₇) oder N(i-C₃H₇), oder N(CH₂C₆H₅) oder N(C₆H₅),
wobei zwei benachbarte Gruppen der Symbolpaare Y¹ und Y¹ oder der Symbolpaare Y¹ und Y² nicht heteroatomige Gruppen derselben Bedeutung darstellen und wobei die Gruppen (Y¹)ₘ und Y² zusammen mit der verknüpften C₂-Einheit des aromatischen Rings und dem mit R⁴ verknüpften C-Atom einen ankondensierten carbocyclischen oder heterocyclischen nicht aromatischen viergliedrigen bis achtgliedrigen Ring bilden,
bedeuten.

Im Falle, dass ein Paar benachbarter Symbole X zusammen eine divalente Gruppe der genannten Formel -O-, -S-, -NH- oder -NR- bedeuten, sind die Doppelbindungen im heteroaromatischen Rest fixiert. Die Formel (I) umfasst dann die Substrukturen (I-A) und (I-B):

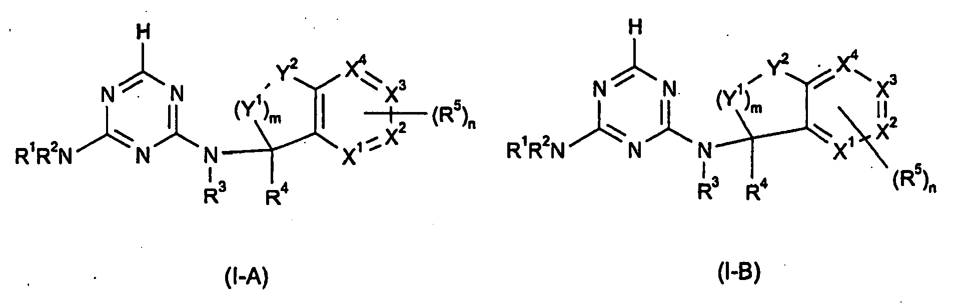

Als aromatische carbocyclische oder heterocyclische Ringe im bicyclischen System kommen als Basis beispielsweise folgende in Frage:
Benzol, Pyridin, Pyrimidin, Pyrazin, Pyridazin, Pyrrol, Pyrazol, Imidazol, Furan, Thiazol, Oxazol, Isoxazol, Thiophen, Thiazol.

Bevorzugt sind auch Verbindungen der Formel (I) und deren Salze, worin ein oder mehrere oder alle der Reste und Symbole aus der Gruppe R¹, R², R³, R⁴, R⁵, R⁶, R⁷, R⁸, R⁹, X¹, X², X³, X⁴, Y¹, Y², n und m jeweils eine der genannten bevorzugten Bedeutungen oder besonders bevorzugten haben, insbesondere auch Bedeutungen haben, wie sie in den Resten der weiter unten genannten Tabellenbeispiele vorkommen.

Gegenstand der vorliegenden Erfindung ist auch ein Verfahren zur Herstellung der Verbindungen der allgemeinen Formel (I) oder deren Salze, dadurch gekennzeichnet, dass man
a) eine Verbindung der Formel (II),

   H-R¹³ (II)

   worin R¹³ eine funktionelle Gruppe aus der Gruppe der Carbonsäureester, Carbonsäureorthoester, Carbonsäurechloride, Carbonsäureamide und Carbonsäureanhydride bedeutet,
   mit einem Biguanid der Formel (III) oder einem Säureadditionssalz hiervon 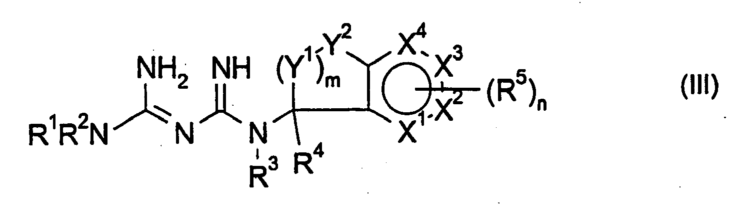 umsetzt oder
b) eine verbindung der formel (IV), 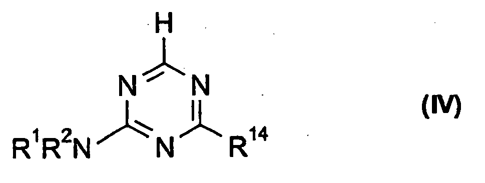 worin R¹⁴ einen austauschfähigen Rest oder eine Abgangsgruppe, z.B. Chlor, Trichlormethyl, (C₁-C₄)Alkylsulfonyl und unsubstituiertes oder substituiertes Phenyl-(C₁-C₄)alkylsulfonyl oder (C₁-C₄)Alkylphenylsulfonyl, bedeutet, mit einem geeigneten Amin der Formel (V) oder einem Säureadditionssalz hiervon 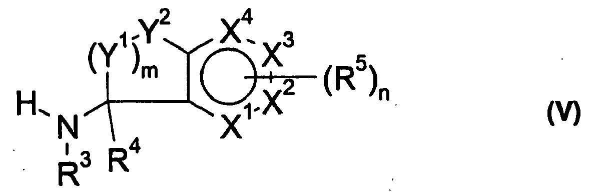 umsetzt oder
c) ein Diamino-1,3,5-triazin der Formel (V1) mit einem Isocyanat der Formel (VII) 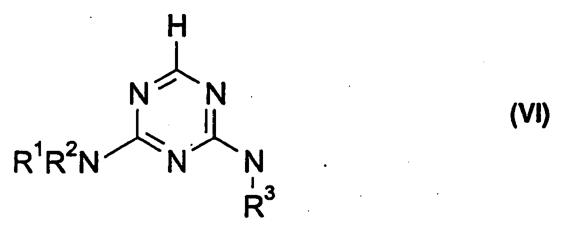 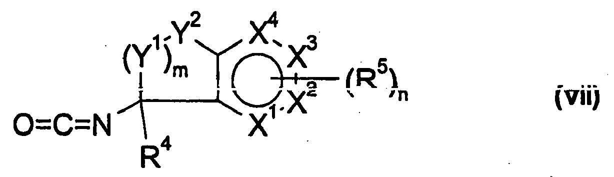 umsetzt oder
d) bei einem Triazin der Struktur (VIII) den Rest R¹⁵, der eine Abgangsgruppe oder ein abspaltbarer Rest ist, mit geeigneten Verfahren, wie beispielsweise durch Hydrierung eines durch Hydrierung abspaltbaren Restes oder einer Abgangsgruppe R¹⁵ , wenn diese beispielsweise Chlor oder einen über Schwefel gebundenen Rest bedeutet, abspaltet, 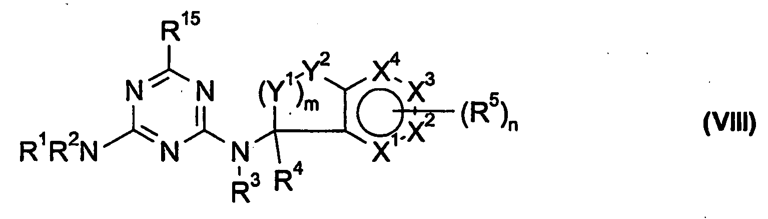
wobei in den Formeln (II), (III), (IV), (V), (VI), (VII) und (VIII) die Reste R¹, R², R³, R⁴, R⁵, X¹, X², X³, X⁴, Y¹ und Y² und die Symbole m und n wie in der Formel (I) definiert sind.

Die Umsetzung der Verbindungen der Formel (II) und (III) erfolgt vorzugsweise basenkatalysiert in einem inerten organischen Lösungsmittel; wie z.B. Tetrahydrofuran (THF), Dioxan, Acetonitril, Dimethylformamid (DMF), Methanol und Ethanol, bei Temperaturen zwischen -10 °C und dem Siedepunkt des Lösungsmittel, vorzugsweise bei 20 °C bis 60 °C; falls Säureadditionssalze der Formel (III) verwendet werden, setzt man diese in der Regel mit Hilfe einer Base in situ frei. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei beispielsweise im Bereich von 0,1 bis 5 Moläquivalenten bezogen auf die Verbindung der Formel (III) eingsetzt. Die Verbindung der Formel (II) kann im Verhältnis zur Verbindung der Formel (III) beispielsweise äquimolar oder mit bis zu 10 Moläquivalenten Überschuß eingesetzt werden. Analoge Verfahren sind aus der Literatur bekannt (vergleiche: Shapiro, S. L.; Parrino, V. A.; Geiger, K.; Kobrin, S.; Freedman, L.; J Am Chem Soc, 1957, 79, 5664).

Die Umsetzung der Verbindungen der Formel (IV) und (V) erfolgt vorzugsweise basenkatatysiert in einem inerten organischen Lösungsmittel, wie z.B. THF, Dioxan, Acetonitril, DMF, Methanol und Ethanol, bei Temperaturen zwischen -10 °C und dem Siedepunkt des jeweiligen Lösungsmittels oder Lösungsmittelgemisches, vorzugsweise bei 20 °C bis 150 °C, insbesondere 20 °C bis 60 °C (sofern damit schon eine Reaktion einsetzt), wobei die Verbindung (V), falls als Säureadditionssalz eingesetzt, gegebenenfalls in situ mit einer Base freigesetzt wird. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in der Regel im Bereich von 1 bis 3 Moläquivalenten bezogen auf die Verbindung der Formel (IV) eingesetzt; die Verbindung der Formel (IV) kann beispielsweise äquimolar zur Verbindung der Formel (V) oder mit bis zu 2 Moläquivalenten Überschuß eingesetzt werden. Analoge Verfahren sind aus der Literatur bekannt (vgl. Comprehensive Heterocyclic Chemistry, A.R. Katritzky, C.W. Rees, Pergamon Press, Oxford, New York, 1984, Vol.3; Part 2B; ISBN 0-08-030703-5, S. 482).

Die Umsetzung der Diamino-1,3,5-triazine der Formel (VI) mit Isocyanaten der Formel (VII) erfolgt vorzugsweise basenkatalysiert in einem inerten organischen Lösungsmittel, wie z.B. Tetrahydrofuran (THF), Dioxan, Acetonitril, Dimethylformamid (DMF) bei Temperaturen zwischen -10 °C und dem Siedepunkt des Lösungsmittel, vorzugsweise bei 20 °C bis 60 °C. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei beispielweise im Bereich von 0,5 bis 3 Moläquivalenten bezogen auf die Verbindung der Formel (VI) eingesetzt. Die Verbindung der Formel (VII) kann im Verhältnis zur Verbindung der Formel (VI) beispielsweise äquimolar oder mit geringem Überschuß eingesetzt werden. Grundsätzlich sind die entsprechenden Verfahren für acyclische und aromatische Derivate aus der Literatur bekannt (vergl. B. Singh; Heterocycles, 1993, 34,S. 929 - 935).

Die Abspaltung des Restes R¹⁵ der Diamino-1,3,5-triazine der Formel (VIII) erfolgt vorzugsweise durch eine gegebenenfalls mit Säuren katalysierte Hydrierung unter zur Hilfenahme eines geeigneten Hydrierkatalysators wie beispielsweise Raney-Nickel oder Edelmetallkatalysatoren wie Palladium oder Platin auf einem geeigneten Träger wie beispielsweise A-Kohle in einem inerten organischen Lösungsmittel, wie z.B. Tetrahydrofuran (THF), Dioxan, geeigneten Alkoholen wie Ethanol, Essigsäure oder auch Mischungen davon bei Temperaturen zwischen -10 °C und dem Siedepunkt des Lösungsmittel, vorzugsweise bei 20 °C bis 60 °C, wobei in dafür geeigneten Apparaturen Wasserstoff in das Reaktionsgemisch eingeleitet wird oder das Reaktionsgemisch einer Wasserstoffatmosphäre unter erhöhtem Druck ausgesetzt wird. Entsprechende Verfahren sind aus der Literatur bekannt (Hirt, R.; Nidecker, H.; Berchtold, R.; Helv. Chim. Acta.; 1950, 33, 1365.).

Die Ausgangsverbindungen der Formeln (II), (III), (IV), (V), (VI), (VII) und (VIII) sind entweder kommerziell erhältlich oder können nach oder analog literaturbekannten Verfahren hergestellt werden. Die Verbindungen können beispielsweise auch nach einem der nachfolgend beschriebenen Verfahren hergestellt werden.

Die Verbindungen der Formel (II) können gegebenenfalls durch Reaktion von Cyanoguanidin mit Aminen der Formel (V) oder Salze hiervon hergestellt werden. Hierzu können beispielsweise beide Komponenten in einem inerten Lösungsmittel wie Dichlorbenzol auf 100 bis 190 °C erhitzt werden, wobei die anfallenenden Biguanidine als Salze dann durch Absaugen isoliert werden können. Entsprechende Verfahren sind bespielsweise durch S.L. Shapiro, V.A. Parrino, L. Freedmann; JACS 81, (1959), S. 3728 oder H.M. Eisa, A.S. Tantawy, M.M. El-Kerdawy; Pharmazie 46, (1991) S. 182 ff. beschrieben. Die Umsetzungen können gegebenfalls durch Zusatz von Metallsalzen wie Kupfer(II) sulfat. Zink(II) chlorid oder Einsen(III) chlorid (T Suyama, T. Soga, K. Miauchi; NIPPON KAGAKU KAISHI (1989), (5), Seite 884-887) katalysiert werden, wobei die Reaktion dann zumeist bei niederigeren Temperaturen im Bereich von 50 °C bis Rückfluß der betreffenden Lösungsmittel erfolgen kann. Gegebenfalls kann die Reaktion dann auch in einer Vielzahl von Lösungmitteln wie THF, Dioxan, Alkohlen oer Aethem durchgeführt werden.

Die Amine entsprechende der Formel (V) können beispielsweise auch hergestellt werden durch. Hydrierung der entsprechenden Oxime, die ihrereseits aus den entsprechenden Ketonen hergestellt werden können. Beispielsweise beschreiben A.B. Sen, S.B. Singh; J. Ind. Chem. Soc.; 43 (1966), Seite 521 ein entsprechendes Verfahren, wobei in diesem Falle die Umsetzung von Natrium zu Natriumethanolat als Wasserstoffquelle genutzt wird. Weiterhin beschreiben Sarges et al.; J.Med.Chem.; 16, (1973), Seite 1003,1008 ein Verfahren zur Umwandlung eines Ketons in das Oxim und dessen Paladium-katalytische Hydrierung zu einem entsprechenden Aminhydochlorid. Auch sind Hydrierungen mit Raney-Nickel (vergl.: D. Barbry, D. Couturier, N. Abdellatifi, D. Lesieur, C. Lespagnol; J. Heterocycl. Chem.; 28, (1991), Seite 449) oder Hydrierungen mit Borwasserstoff-Verbindungen (A.K. Gosh.S.P. McKee, W.M. Sanders; Tetrahedron Lett. 32, (1991), Seite 711-714) und andere Verfahren bei entsprechenden bicyclischen Derivaten beschrieben und können zur Synthese von Aminen entsprechend der Formel (V) angewendet werden.

Die Verbindung der Formel (IV), oder eine direkte Vorstufe davon, läßt sich beispielweise wie folgt herstellen:
1. Durch Reaktion einer Verbindung der Formel (II) mit einem Amidinothioharnstoff-Derivat der Formel (IX), 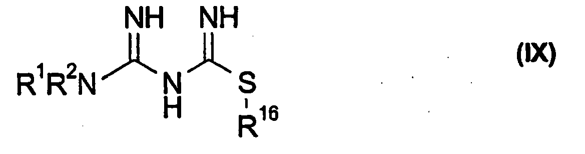 worin R¹⁶ (C₁-C₄)-Alkyl oder Phenyl-(C₁-C₄)-alkyl bedeutet und R¹ und R² wie in Formel (I) definiert sind, werden Verbindungen der Formel (IV) erhalten, in denen R¹⁴ = -SR¹⁶ bedeutet.
2. Durch Umsetzung eines Amidins der Formel (X) oder eines Säureadditionssalzes davon, 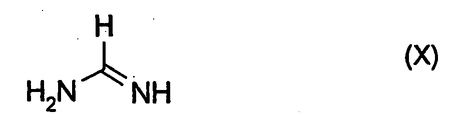 mit einem N-Cyanodithioiminocarbonat der Formel (XI), 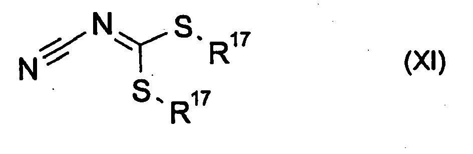 worin R¹⁷ (C₁-C₄)-Alkyl oder Phenyl-(C₁-C₄)-alkyl bedeutet, werden Verbindungen der Formel (IV) erhalten, worin R¹⁴ =-S-R¹⁷ bedeutet.
3. Durch Umsetzung eines Alkali-dicyanamids mit einem Carbonsäurederivat der genannten Formel (II) werden Verbindungen der Formel (VI) erhaltene, worin R¹⁴ = NH₂ bedeutet,
   Gegebenenfalls können analog den vorstehenden Verfahren unter 1.- 3. auch Zwischenprodukte der Formel (XII), 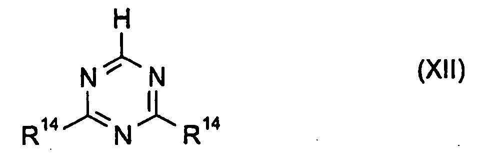 mit 2 austauschfähigen Gruppen R¹⁴ hergestellt werden (vgl. Formel (IV)) und die austauschfähigen Gruppen nacheinander mit geeigneten Aminen oder Ammoniak substituiert werden, um analog allgemein bekannten Verfahrensweisen zu Verbindungen der Formel (I) zu gelangen. Ebenso können auch kommerziell erhältliche Verbindungen oder nach anderen Verfahen herstellbare Verbindungen der Formel (XII) entsprechend modifiziert werden.
   Gegebenfalls können analog den vorstehenden Verfahren unter 1.- 3. erhaltene Zwischenprodukte der Formel (IV) oder (XII), worin R¹⁴ (C₁-C₄)-Alkylthio oder Phenyl-(C₁-C₄)-alkylthio bedeutet, durch eine Chlorierung oder Oxidation in reaktionsfähigere Derivate der Formeln (IV) oder (XII) umgewandelt werden.

Die Umsetzung der Carbonsäurederivate der Formel (II) mit den Amidinothiohamstoff-Derivaten der Formel (VIII) erfolgt vorzugsweise basenkatalysiert in einem organischen Lösungsmittel, wie z.B. Aceton, THF, Dioxan, Acetonitril, DMF, Methanol, Ethanol, bei Temperaturen von -10 °C bis zum Siedepunkt des Lösungsmittel, vorzugsweise bei 0 °C bis 20 °C. Die Umsetzung kann aber auch in Wasser oder in wässrigen Lösungsmitttelgemischen mit einem oder mehreren der obengenannten organischen Lösungsmitteln erfolgen. Falls (VI) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei im Bereich von 1 bis 10 Moläquivalenten bezogen auf die Verbindung der Formel (VIII) eingesetzt. Verbindungen der Formel (II) und (VIII) können beispielsweise äquimolar oder mit bis zu 2 Moläquivalenten Überschuß an Verbindung der Formel (II) eingesetzt werden. Analoge Verfahren sind literaturbekannt (vergl.: H. Eilingsfeld, H. Scheuermann, Chem. Ber.; 1967, 100, 1874).

Die Umsetzung der Amidine der Formel (IX) mit den N-Cyanodithioiminocarbonaten der Formel (X) erfolgt vorzugsweise basenkatalysiert in einem inerten organischen Lösungsmittel, wie z.B. Acetonitril, DMF, Dimethylacetamid (DMA), N-Methylpyrrolidon (NMP), Methanol und Ethanol, bei Temperaturen von -10 °C bis zum Siedepunkt des Lösungsmittels, vorzugsweise bei 20 °C bis 80 °C. Falls (VII) als Säureadditionssalz eingesetzt wird, kann es gegebenenfalls in situ mit einer Base freigesetzt werden. Als Basen bzw. basische Katalysatoren eignen sich Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in Bereich von 1 bis 3 Moläquivalenten bezogen auf die Verbindung der Formel (X) eingesetzt, Verbindungen der Formel (IX) und (X) können in der Regel äquimolar oder mit 2 Moläquvalenten Überschuß an Verbindung der Formel (II) eingesetzt werden. Analoge Verfahren sind literaturbekannt (vergl.: T.A. Riley, W.J. Henney, N.K. Dalley, B.E. Wilson, R.K. Robins; J. Heterocyclic Chem.; 1986, 23 (6), 1706-1714).

Die Herstellung von Zwischenprodukten der Formel (XII) mit R¹⁴ = Chlor kann durch Reaktion von Alkali-dicyanamid mit einem Carbonsäurederivat der Formel (II), wobei dann R¹³ bevorzugt die funktionelle Gruppe Carbonsäurechlorid oder Carbonsäureamid bedeutet, erfolgen. Die Umsetzung der Reaktionskomponenten erfolgt beispielsweise säurekatalysiert in einem inerten organischen Lösungsmittel wie z.B. Toluol, Chlorbenzol, chlorierten Kohlenwasserstoffen bei Temperaturen zwischen -10 °C und dem Siedepunkt des Lösungsmittels, vorzugsweise bei 20 °C bis 80 °C, wobei die entstehenden Intermediate in situ mit einem geeigneten Chlorierungsreagenz wie beispielsweise Phosphoroxychlorid chloriert werden können. Geeignete Säuren sind z.B. Halogenwasserstoffsäuren, wie HCl, oder auch Lewis-Säuren, wie z.B. AlCl₃ oder BF₃ (vergl. US-A-5095113, DuPont und dort zitierte Literatur).

Zwischenprodukte der Formel (IV) oder (XII), worin R¹⁴ = (C₁-C₄)Alkylmercapto oder unsubstituiertes Phenyl-(C₁-C₄)-alkylmercapto ist, können in einem inerten organischen Lösungsmittel wie z.B. Toluol, Chlorbenzol, chlorierten Kohlenwasserstoffen oder anderen bei Temperaturen zwischen -40 °C und dem Siedepunkt des Lösungsmittel, vorzugsweise bei 20 °C bis 80 °C, mit einem geeigneten Chlorierungsreagenz wie z.B. elementarem Chlor oder Phosporoxychlorid zu reaktionsfähigeren Chlortriazinen der Formel (IV) oder (X), worin R¹⁴ = Cl ist, überführt werden (vgl. J.K. Chakrabarti, D.E. Tupper, Tetrahedron 1975, 31(16), 1879-1882)

Zwischenprodukte der Formel (IV) oder (XII), wobei R¹⁴= (C₁-C₄)Alkylmercapto oder unsubstituiertes oder substituiertes Phenyl-(C₁-C₄)-alkylmercapto oder (C₁-C₄)Alkylphenylthio ist, können in einem geeigneten Lösungsmittel wie z.B. chlorierten Kohlenwasserstoffen, Essigsäure, Wasser, Alkoholen, Aceton oder Mischungen hiervon bei Temperaturen zwischen 0 °C und dem Siedepunkt des Lösungsmittels, vorzugsweise von 20 °C bis 80 °C, mit einem geeigneten Oxidationssreagenz wie z.B. m-Chlorperbenzoesäure, Wasserstoffperoxid, Kaliumperoxomonosulfat oxidiert werden (vergl.: T.A. Riley, W.J. Henney, N.K. Dalley, B.E. Wilson, R.K. Robins; J. Heterocyclic Chem.; 1986, 23 (6), 1706-1714).

Verbindungen analog der Formel (IV) erhält man auch durch selektive nucleophile Substitution einer austauschfähigen Gruppen bei Verbindungen analog der Formel (XII), wobei R¹⁴ beispielsweise für Halogen, Perhalomethyl, (C₁-C₄)Alkylsulfinyl, (C₁-C₄)Alkylsulfonyl oder andere literaturbekannte Abgangsgruppen steht, in einem geeigneten Lösungsmittel wie z .B. THF, Dioxan, Alkohole, DMF oder Acetonitril oder Mischungen hiervon bei Temperaturen zwischen -10 °C und dem Siedepunkt des Lösungsmittels, vorzugsweise bei 10 °C bis 25 °C, gegebenfalls unter basischen Bedingungen. Als Basen eignen sich dabei Alkalihydroxide, Alkalihydride, Alkalicarbonate, Alkalialkoholate, Erdalkalihydroxide, Erdalkalihydride, Erdalkalicarbonate oder organische Basen wie Triethylamin oder 1,8-Diazabicyclo[5.4.0]undec-7-en (DBU). Die jeweilige Base wird dabei in Bereich von 1 bis 3 Moläquivalenten bezogen auf die Verbindung der Formel (XII) eingesetzt; das Nucleophil wird in der Regel äquimolar bis zu 2 Moläquivalenten im Überschuß eingesetzt und kann gegebenenfalls auch selbst als Base gebraucht werden. Analoge Verfahren sind literaturbekannt (vergl.: V.I. Kaelarev, Dibi Ammar, A.F. Lunin; Ximinya Geterosikl. Soedin:, 1985, N11, 1557 -1563).

Eine Kollektion aus Verbindungen (I), die nach den obengenannten Verfahren synthetisiert werden können, können zusätzlich in parallelisierter Weise hergestellt werden , wobei dies in manueller, teilweise automatisierter oder vollständig automatisierter Weise geschehen kann. Dabei ist es möglich, sowohl die Reaktionsdurchführung die Aufarbeitung oder die Reinigung der Produkte bzw. Zwischenstufen zu automatisieren. Insgesamt wird hierunter eine Vorgehensweise verstanden, wie sie beispielsweise durch S. H. DeWitt in "Annual Reports in Combinatorial Chemistry and Molecular Diversity: Automated Synthesis", Band 1, Verlag Escom, 1997, Seite 69 bis 77 beschrieben wird.
Zur parallelisierten Reaktionsdurchführung und Aufarbeitung können eine Reihe von im Handel erhältlichen Geräten verwendet werden wie sie beispielsweise von den Firmen Stern Corporation, Woodrolfe Road, Tollesbury, Essex, CM9 8SE, England oder H + P Labortechnik GmbH, Bruckmannring 28, 85764 Oberschleißheim, Deutschland angeboten werden. Für die parallelisierte Aufreinigung von Verbindungen (I) oder von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA. Die aufgeführten Apparaturen ermöglichen eine modulare Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise von Roboter bedient werden. Derartige Automationssysteme können zum Beispiel von der Firma Zymark Corporation, Zymark Center, Hopkinton, MA 01748, USA bezogen werden.
Neben den beschriebenen Methoden kann die Herstellung von Verbindungen (I) vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepaßten Synthese an ein Syntheseharz gebunden. Festphasen unterstützte Synthesemethoden sind in der Fachliteratur hinreichend beschrieben, z. B.: Barry A. Bunin in "The Combinatorial Index", Verlag Academic Press, 1998.
Die Verwendung von Festphasen unterstützten Synthesemethoden erlaubt eine Reihe von literaturbekannten Protokollen, die wiederum manuell oder automatisiert ausgeführt werden können. Zum Beispiel kann die "Teebeutelmethode" (Houghten, US 4,631,211; Houghten et al., Proc. Natl. Acad. Sci., 1985, 82, 5131 - 5135) mit Produkten der Firma IRORI, 11149 North Torrey Pines Road, La Jolla, CA 92037, USA teilweise automatisiert werden. Die Automatisierung von Festphasen unterstützter Parallelsynthese gelingt beispielsweise durch Apparaturen der Firmen Argonaut Technologies, Inc., 887 Industrial Road, San Carlos, CA 94070, USA oder MultiSynTech GmbH, Wullener Feld 4, 58454 Witten, Deutschland.

Die Herstellung gemäß der hier beschriebenen Verfahren liefert Verbindungen (1) in Form von Substanzkollektionen oder -bibliotheken, also Bibliotheken der Verbindungen (1), die mindestens zwei Verbindungen (I) enthalten und deren Vorprodukten.

Zur Herstellung der Säureadditionssalze der Verbindungen der Formel (I) kommen folgende Säuren in Frage: Halogenwasserstoffsäuren wie Chlorwasserstoffsäure oder Bromwasserstoffsäure, weiterhin Phosphorsäure, Salpetersäure, Schwefelsäure, mono- oder bifunktionelle Carbonsäuren und Hydroxycarbonsäuren wie Essigsäure, Maleinsäure, Bernsteinsäure, Fumarsäure, Weinsäure, Citronensäure, Salicylsäure, Sorbinsäure oder Milchsäure, sowie Sulfonsäuren wie p-Toluolsulfonsäure oder 1,5-Naphtalindisulfonsäure. Die Säureadditionsverbindungen der Formel (I) können in einfacher Weise nach den üblichen Salzbildungsmethoden, z.B. durch Lösen einer Verbindung der Formel (I) in einem geeigneten organischen Lösungsmittel wie z.B. Methanol, Aceton, Methylenchlorid oder Benzin und Hinzufügen der Säure bei Temperaturen von 0 bis 100 °C erhalten werden und in bekannter Weise, z.B. durch Abfiltrieren, isoliert und gegebenfalls durch Waschen mit einem inerten organischen Lösemittel gereinigt werden.
Die Basenadditionssalze der Verbindungen der Formel (I) werden vorzugsweise in inerten polaren Lösungsmitteln wie z.B. Wasser, Methanol oder Aceton bei Temperaturen von 0 bis 100 °C hergestellt. Geeignete Basen zur Herstellung der erfindungsgemäßen Salze sind beispielsweise Alkalicarbonate, wie Kaliumcarbonat, Alkali- und Erdalkalihydroxide, z.B. NaOH oder KOH, Alkali- und Erdalkalihydride, z.B. NaH, Alkali- und Erdalalkoholate, z.B. Natriummethanolat, Kalium-tert.Butylat, oder Ammoniak oder Ethanolamin.

Mit den in den vorstehenden Verfahrensvarianten bezeichneten "inerten Lösungsmitteln" sind jeweils Lösungsmittel gemeint, die unter den jeweiligen Reaktionsbedingungen inert sind jedoch nicht unter beliebigen Reaktionsbedingungen inert sein müssen.

Die erfindungsgemäßen Verbindungen der Formel (I) und deren Salze, weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf oder Nachauflaufverfahren ausgebracht werden.
Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.
Auf der Seite der monokotylen Unkrautarten werden z.B.
Agrostis, Alopecurus, Apera, Avena, Brachicaria, Bromus, Dactyloctenium, Digitaria, Echinochloa, Eleocharis, Eleusine, Festuca, Fimbristylis, Ischaemum, Lolium, Monochoria, Panicum, Paspalum, Phalaris, Phleum, Poa, Sagittaria, Scirpus, Setaria, Sphenoclea sowie Cyperusarten vorwiegend aus der annuellen Gruppe und auf Seiten der perennierenden Spezies Agropyron, Cynodon, Imperata sowie Sorghum und auch ausdauernde Cyperusarten gut erfaßt.
Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Außerdem wird herbizide Wirkung bei dikotylen Unkräutern wie Ambrosia, Anthemis, Carduus, Centaurea, Chenopodium, Cirsium, Convolvulus, Datura, Emex, Galeopsis, Galinsoga, Lepidium, Lindernia, Papaver, Portlaca, Polygonum, Ranunculus, Rorippa, Rotala, Seneceio, Sesbania, Solanum, Sonchus, Taraxacum, Trifolium, Urtica und Xanthium erzielt.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen,- Gerste, Roggen, Reis, Mais, Zuckerrübe, Zuckerrohr, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Die erfindungsgemäßen Verbindungen der Formel (I) oder deren Salze können wegen der breiten Wirksamkeit gegen unerwünschten Pflanzenwuchs auch in Baumkulturen, im Weinbau und in Kulturen zur Erzeugung von Nüssen eingesetzt werden. Weiterhin ist die Anwendung im industriellen Bereich möglich, wenn auf industriellen Freiflächen oder beispielsweise auf Fahrwegen oder Bahnstrecken der Pflanzenwuchs verhindert werden soll.

Darüberhinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Emteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von bekannten oder noch zu entwickelnden gentechnisch veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Emteguts bekannt.

Bevorzugt ist die Anwendung der erfindungsgemäßen Verbindungen der Formel (I) oder deren Salze in wirtschaftlich bedeutenden transgenen Kulturen von Nutz-und Zierpflanzen, z. B. von Getreide wie Weizen, Gerste, Roggen, Hafer, Hirse, Reis, Maniok und Mais oder auch Kulturen von Zuckerrübe, Baumwolle, Soja, Raps, Kartoffel, Tomate, Erbse und anderen Gemüsesorten.

Vorzugsweise können die Verbindungen der Formel (I) als Herbizide in Nutzpflanzenkülturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der
   in den Pflanzen synthetisierten Stärke (z. B. WO 92111376, WO 92/14827, WO 91/19806),
- transgene Kulturpflanzen, welche gegen bestimmte Herbizide vom Typ
   Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
Zahlreiche molekularbiologische Techniken,' mit denen neue transgene Pflanzen mit veränderten Eigenschaften hergestellt werden können, sind im Prinzip bekannt; siehe z.B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996 oder Christou, "Trends in Plant Science" 1 (1996) 423-431).
Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe der obengenannten Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden.
Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet
Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweise, aber nicht vollkommen identisch sind.
Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Weiter et al., Proc. Natl. Acad. Sci. USA 85 (1988), 846-850; Sonnewald et al., Plant J. 1 (1991), 95-106).
Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h. sowohl monokotyle als auch dikotyle Pflanzen.
So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen oder mutanten Kulturen eingesetzt werden, welche gegen Herbizide aus der Gruppe der Sulfonylharnstoffe, Imidazolinone, Glufosinate-ammonium oder Glyphosate-isopropylammonium oder analoger Wirkstoffe resistent sind.
Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (1) enthalten.
Die Verbindungen der Formel (1) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.
Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers" 2nd Ed Darland Books Caldwell N.J, H.v Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emufsifiers Annual", MC Publ. Corp., Ridgewood N.J.; Sisley and Wood, "Encyclopedia of . Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenem, Düngemitteln und/oder Wachstumsregulatoren herstellen, z.B. in Form einer Fertigformulierung oder als Tankmix.
Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkypolyether, Sorbitanester wie z.B. Sorbitanfettsäureester oder Polyoxethylensorbitanester wie z.B. Polyoxyethylensorbitanfettsäureester.
Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.
Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.
Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.
Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.
Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischem und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57. Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.
Die agrochemischen Zubereitungen enthalten in der Regel 0,1 bis 99 Gew.-%, insbesondere 0,1 bis 95 Gew.%, Wirkstoff der Formel (I).
In Spritzpulvern beträgt die Wirkstoffkonzentration z.B. etwa 10 bis 90 Gew.%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.% betragen. Staubförmige Formulierungen enthalten 1 bis 30 Gew.-% Wirkstoff, vorzugsweise meistens 5 bis 20 Gew.% an Wirkstoff, versprühbare Lösungen enthalten etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.% .
Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.
Die Verbindungen der Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenem, Düngemitteln und/oder Wachstumsregulatoren kombiniert eingesetzt werden, z. B. als Fertigformulierung oder als Tankmischungen.
Als Kombinationspartner für die erfindungsgemäßen Wirkstoffe in Mischungsformulierungen oder im Tank-Mix sind beispielsweise bekannte Wirkstoffe einsetzbar, wie sie in z.B. aus Weed Research 26,441-445 (1986), oder "The Pesticide Manual", 12the edition, The British Crop Protection Council and the Royal Soc. of Chemistry, 2000/2001 und dort zitierter Literatur beschrieben sind. Als bekannte Herbizide oder Pflanzenwachstumsregulatoren, die mit den Verbindungen der Formel (I) kombiniert werden können, kommen beispielsweise folgende Wirkstoffe in Frage; die Verbindungen sind nachstehend entweder mit dem "common name" (meist gemäß englischer Schreibweise) nach der International Organization for Standardization (ISO) oder mit dem chemischen Namen, ggf. zusammen mit einer üblichen Codenummer bezeichnet:
acetochlor, acifluofen(-sodium):aclonifen: AKH 7088.d.h.[[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim(-sodium); ametryn; amidochlor, amidosulfuron; amitrol; AMS, d.h. Ammoniumsulfamat; anilofos; asulam; atrazin; azafenidin, azimsulfurone (DPX-A8947); aziprotryn; barban; BAS 516 H,d.h. 5-Fluor-2-phenyl-4H-3,1-benzoxazin-4-on; beflubutamid, benazolin(-ethyl); benfluralin; benfuresate; bensulfuron(-methyl); bensulide; bentazone; benzobicyclon, benzofenap; benzofluor; benzoylprop(-ethyl); benzthiazuron; bialaphos; bifenox; bispyribac(-sodium), bromacil; bromobutide; bromofenoxim; bromoxynil; bromuron; buminafos; busoxinone; butachlor; butafenacil, butamifos; butenachlor; buthidazole; butralin; butroxydim, butylate; cafenstrole (CH-900); carbetamide; carfentrazone(-ethyl) (ICI-A0051); caloxydim, CDAA, d.h. 2-Chlor-N,N-di-2-propenylacetamid; CDEC, d.h. Diethyldithiocarbaminsäure-2-chlorallylester, chlomethoxyfen; chloramben; chlorazifop-butyl, chlormesulon (ICI-A0051); chlorbromuron; chlorbufam; chlorfenac; chlorflurecol-methyl; chloridazon; chlorimuron(-ethyl); chlomitrofen; chlorotoluron; chloroxuron; chlorpropham; chlorsulfuron; chlorthal-dimethyl; chlorthiamid; chlortoluron, cinmethylin; cinosulfuron; clethodim; cinidon(-methyl), clefoxydim, clodinafop und dessen Esterderivate (z.B. clodinafoppropargyl); clomazone; clomeprop; cloproxydim; clopyralid; clopyrasulfuron(-methyl), cloransulam(-methyl), cumyluron (JC 940); cyanazine; cycloate; cyclosulfamuron (AC 104); cycloxydim; cycluron; cyhalofop und dessen Esterderivate (z.B. Butylester, DEH-112); cyperquat; cyprazine; cyprazole; daimuron; 2,4-D, 2,4-DB; 2,4-DB, dalapon; desmedipham; desmetryn; di-allate; dicamba; dichlobenil; dichlorprop; diclofop und dessen Ester wie diclofop-methyl; diclosulam, diethatyl(-ethyl); difenoxuron; difenzoquat; diflufenican; diflufenzopyr, dimefuron; dimepiperate, dimethachlor; dimethametryn; dimethenamid (SAN-582H); dimethazone, dimexyflam, dimethipin; dimetrasulfuron, dinitramine; dinoseb; dinoterb; diphenamid; dipropetryn; diquat; dithiopyr; diuron; DNOC; eglinazine-ethyl; EL 77, d.h. 5-Cyano-1-(1,1-dimethylethyl)-N-methyl-1H-pyrazole-4-carboxamid; endothal; epoprodan, EPTC; esprocarb; ethalfluralin; ethametsulfuron-methyl; ethidimuron; ethiozin; ethofumesate; ethoxysulfuron, etobenzanid (HW 52); F5231, d.h. N-[2-Chlor-4-fluor-5-[4-(3-fluorpropyl)-4,5-dihydro-5-oxo-1 H-tetrazol-1 -yl]-phenyl]-ethansulfonamid; ethoxyfen und dessen Ester (z.B. Ethylester, HN-252); fenoprop; fenoxan, fenoxaprop und fenoxaprop-P sowie deren Ester.z.B. fenoxaprop-P-ethyl und fenoxaprop-ethyl; fenoxydim; fentrazamide, fenuron; flamprop(-methyl oder -isopropyl oder -isopropyl-L); flazasulfuron; floazulate, florasulam, fluazifop und fluazifop-P und deren Ester, z.B. fluazifop-butyl und fluazifop-P-butyl; flucarbazone(-sodium), fluchloralin; flumetsulam; flumeturon; flumiclorac(-pentyl), flumioxazin (S-482); flumipropyn; fluometuron, fluorochloridone, fluorodifen; fluoroglycofen(-ethyl); flupoxam (KNW-739); flupropacil (UBIC-4243); flupyrsulfuron(-methyl oder -sodium), flurenol(-butyl), fluridone; flurochloridone; fluroxypyr(-meptyl); flurprimidol, flurtamone; fluthiacet(-methyl), fluthiamide, fomesafen; foramsulfuron, fosamine; furyloxyfen; glufosinate(-ammonium); glyphosate(-isopropylammonium); halosafen; halosulfuron(-methyl) und dessen Ester (z.B. Methylester, NC-319); haloxyfop und dessen Ester, haloxyfop-P (= R-haloxyfop) und dessen Ester, hexazinone; imazamethabenz(-methyl); imazapyr; imazaquin und Salze wie das Ammoniumsalz; imazamethapyr, imazamox, imazapic, imazethamethapyr; imazethapyr; imazosulfuron; indanofan, iodosulfuron-methyl-sodium salt, ioxynil; isocarbamid; isopropalin; isoproturon; isouron; isoxaben; isoxachlortole, isoxaflutole, isoxapyrifop; karbutilate; lactofen; lenacil; linuron; MCPA; MCPB; mecoprop; mefenacet; mefluidid; mesosulfuron-methyl, mesotrione, metamitron; metazachlor, methabenzthiazuron; metham; methazole; methoxyphenone; methyldymron; metabenzuron, methobenzuron; metobromuron; (alpha-)metolachlor, metosulam (XRD 511); metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1 - methylethylrphenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; obencarb; oryzalin; oxadiargyl (RP-020630); oxadiazon; oxasulfuron, oxaziclomefone, oxyfluorfen; paraquat; pebulate; pelargonic acid, pendimethalin; pentoxazone, perfluidone; phenisopham; phenmedipham; picloram; picolinafen, piperophos; piributicarb; pirifenop-butyl; pretilachlor; primisulfuron(-methyl); procarbazone-(sodium), procyazine; prodiamine; profluralin; proglinazine(-ethyl); prometon; prometryn; propachlor, propanil; propaquizafop und dessen Ester, propazine; propham; propisochlor, propyzamide; prosulfalin; prosulfocarb; prosulfuron (CGA-152005); prynachlor, pyraflufen(-ethyl), pyrazolinate; pyrazon; pyrazosulfuron(-ethyl); pyrazoxyfen, pyribenzoxim, pyrlbuticarb, pyridafol, pyridate; pyrimidobac(-methyl), pyrithiobac(-sodium) (KIH-2031); pyroxofop und dessen Ester (z.B. Propargylester); quinclorac; quinmerac; quinoclamine, quinofop und dessen Esterderivate, quizalofop und quizalofop-P und deren Esterderivate z.B. quizalofop-ethyl; quizalofop-P-tefuryl und -ethyl; renriduron; rimsulfuron (DPX-E 9636); S 275, d.h. 2-[4-Chlor-2-fluor-5-(2-propynyloxy)-phenyl]-4,5,6,7-tetrahydro-2H-indazol; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester, sulcotrione, sulfentrazon (FMC-97285, F-6285); sulfazuron; sulfometuron(-methyl); sulfosate (ICI-A0224); sulfosulfuron, TCA; tebutam (GCP-5544); tebuthiuron; tepraloxydim, terbacil; terbucarb; terbuchlor terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thenylchlor (NSK-850); thiafluamide, thiazafluron; thiazopyr (Mon-13200); thidiazimin (SN-24085); thifensulfuron(-methyl); thiobencarb; tiocarbazil; tralkoxydim; tri-allate; triasulfuron; triaziflam, triazofenamide; tribenuron(-methyl); triclopyr; tridiphane; trietazine; trifluralin; triflusulfuron und Ester (z.B. Methylester, DPX-66037); trimeturon; tritosulfuron, tsitodef, vemolate; WL 110547, d.h. 5-Phenoxy-1-[3-(trifluormethyl)-phenyl]-1H-tetrazol; UBH-509; D-489; LS 82-556; KPP-300; NC-324; NC-330; KH-218; DPX-N8189; SC-0774; DOWCO-535; DK-8910; V-53482; PP-600; MBH-001; KIH-9201; ET-751; KIH-6127 und KIH-2023.

Von besonderem Interesse ist die selektive Bekämpfung von Schadpflanzen in Kulturen von Nutz und Zierpflanzen. Obgleich die erfindungsgemäßen Verbindungen (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen (I) von besonderem Interesse, weiche die Verbindungen (I) bzw. deren Kombinationen mit anderen Herbiziden oder Pestiziden und Safenem enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen. Gerste. Roggen. Mais. Reis. Hirse). Zuckerrübe. Zuckerrohr. Raps. Baumwolle und Soja, vorzugsweise Getreide.

Folgende Gruppen von Verbindungen kommen beispielsweise als Safener für die Verbindungen (I) und deren Kombinationen mit weiteren Pestiziden in Frage:
a) Verbindungen vom Typ der Dichlorphenylpyrazolin-3-carbonsäure, vorzugsweise Verbindungen wie 1-(2,4-Dichlorphenyl)-5-(ethoxycarbonyl)-5-methyl-2-pyrazolin-3-carbonsäureethylester (S1-1) ("Mefenpyr-diethyl", "The Pesticide Manual", 12th edition, 2000, Nr. 492, S. 594-595), und verwandte Verbindungen, wie sie in der WO 91/07874 beschrieben sind,
b) Derivate der Dichlorphenylpyrazolcarbonsäure, vorzugsweise Verbindungen wie 1-(2,4-Dichlorphenyl)-5-methyl-pyrazol-3-carbonsäureethylester (S1-2), 1-(2,4-Dichlorphenyl)-5-isopropyl-pyrazol-3-carbonsäureethylester (51-3), 1-(2,4-Dichlorphenyl)-5-(1,1-dimethyl-ethyl)pyrazol-3-carbonsäureethyl-ester (S1-4), 1-(2,4-Dichlorphenyl)-5-phenyl-pyrazol-3-carbonsäureethylester (S1-5) und verwandte Verbindungen, wie sie in EP-A-333 131 und EP-A-269 806 beschrieben sind.
c) Verbindungen vom Typ der Triazolcarbonsäuren, vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-6), und verwandte Verbindungen (siehe EP-A-174 562 und EP-A-346 620);
d) Verbindungen vom Typ der 5-Benzyl- oder 5-Phenyl-2-isoxazolin-3-carbonsäure, oder der 5,5-Diphenyl-2-isoxazolin-3-carbonsäure vorzugsweise Verbindungen wie 5-(2,4-Dichlorbenzyl)-2-isoxazolin-3-carbonsäureethylester (S1-7) oder 5-Phenyl-2-isoxazolin-3-carbonsäureethylester (S1-8) und verwandte Verbindungen, wie sie in WO 91/08202 beschrieben sind, bzw. der 5,5-Diphenyl-2-isoxazolin-carbonsäureethylester (S1-9) ("Isoxadifen-ethyl") oder -n-propylester (S1-10) oder der 5-(4-Fluorpheny)-5-phenyl-2-isoxazolin-3-carbonsäureethylester (S1-11), wie sie in der deutschen Patentanmeldung (WO-A-95/07897) beschrieben sind.
e) Verbindungen vom Typ der 8-Chinolinoxyessigsäure (S2), vorzugsweise (5-Chlor-8-chinolinoxy)-essigsäure-(1-methyl-hex-1-yl)-ester (Common name "Cloquintocet-mexyl" (S2-1) (siehe "The Pesticide Manual", 12th edition, 2000, Nr. 195, S 195-196)
   (5-Chlor-8-chinolinoxy)-essigsäure-(1,3-dimethyl-but-1-yl)-ester (S2-2),
   (5-Chlor-8-chinolinoxy)-essigsäure-4-allyl-oxy-butylester (S2-3),
   (5-Chlor-8-chinolinoxy)-essigsäure-1-allyloxy-prop-2-ylester (S2-4),
   (5-Chlor-8-chinolinoxy)-essigsäureethylester (S2-5),
   (5-Chlor-8-chinolinoxy)-essigsäuremethylester (S2-6),
   (5-Chlor-8-chinolinoxy)-essigsäureallylester (S2-7),
   (5-Chlor-8-chinolinoxy)-essigsäure-2-(2-propyliden-iminoxy)-1-ethylester (S2-8),
   (5-Chlor-8-chinolinoxy)-essigsäure-2-oxo-prop-1-ylester (S2-9)
   und verwandte Verbindungen, wie sie in EP-A-86 750, EP-A-94 349 und EP-A-191 736 oder EP-A-0 492 366 beschrieben sind.
f) Verbindungen vom Typ der (5-Chlor-8-chinolinoxy)-malonsäure, vorzugsweise Verbindungen wie (5-Chlor-8-chinolinoxy)-malonsäure-diethylester,
   (5-Chlor-8-chinolinoxyhmalonsäurediallylester,
   (5-Chlor-8-chinolinoxy)-malonsäure-methyl-ethylester und verwandte Verbindungen,
   wie sie in EP-A-0 582 198 beschrieben sind.
g) Wirkstoffe vom Typ der Phenoxyessig- bzw. -propionsäurederivate bzw. der aromatischen Carbonsäuren, wie z.B. 2,4-Dichlorphenoxyessigsäure(ester) (2,4-D), 4-Chlor-2-methyl-phenoxy-propionester (Mecoprop), MCPA oder
   3,6-Dichlor-2-methoxy-benzoesäure(ester) (Dicamba).
h) Wirkstoffe vom Typ der Pyrimidine, die als bodenwirksame Safener in Reis angewendet werden, wie z. B. "Fenclorim" ("The Pesticide Manual", 12th edition, 2000, Nr. 325, S. 386-387) (= 4,6-Dichlor-2-phenylpyrimidin), das als Safener für Pretilachlor in gesätem Reis bekannt ist,
i) Wirkstoffe vom Typ der Dichloracetamide, die häufig als Vorauflaufsafener (bodenwirksame Safener) angewendet werden, wie z. B. "Dichlormid" ("The Pesticide Manual", 12th edition, 2000, Nr. 225, S. 270-271) (= N,N-Diallyl-2,2-dichloracetamid),
   "R-29148" (= 3-Dichloracetyl-2,2,5-trimethyl-1,3-oxazolidin von der Firma Stauffer),
   "Benoxacor" ("The Pesticide Manual", 12th edition, 2000, Nr. 65 S. 74-75) (= 4-Dichloracetyl-3,4-dihydro-3-methyl-2H-1,4-benzoxazin).
   "PPG-1292" (= N-Allyl-N-[(1,3-dioxolan-2-yl)-methyl]-dichloracetamid von der Firma PPG Industries),
   "DK-24" (= N-Allyl-N-[(allylaminocarbonyl)-methyl]-dichloracetamid von der Firma Sagro-Chem),
   "AD-67" oder "MON 4660" (= Dichloracetyl-1-oxa-3-aza-spiro[4,5]decan von der Firma Nitrokemia bzw. Monsanto),
   "Diclonon" oder "BAS145138" oder "LAB145138" (= (= 3-Dichloracetyl-2,5,5-trimethyl-1,3-diazabicyclo[4.3.0]nonan von der Firma BASF) und
   "Furilazol" oder "MON 13900" (siehe "The Pesticide Manual", 12th edition, 2000, Nr. 401, S. 482-483) (= (RS)-3-Dichloracetyl-5-(2-furyl)-2,2-dimethyloxazolidin)
j) Wirkstoffe vom Typ der Dichloracetonderivate, wie z. B.
   "MG 191" (CAS-Reg. Nr. 96420-72-3) (= 2-Dichlormethyl-2-methyl-1,3-dioxotan von der Firma Nitrokemia), das als Safener für Mais bekannt ist,
k) Wirkstoffe vom Typ der Oxyimino-Verbindungen, die als Saatbeizmittel bekannt sind, wie z. B.
   "Oxabetrinil" ("The Pesticide Manual", 12the, edition, 2000, Nr. 577.S. 689) (= (Z)-1,3-Dioxolan-2-ylmethoxyimino(phenyl)acetonitril), das als Saatbeiz-Safener für Hirse gegen Schäden von Metolachlor bekannt ist,
   "Fluxofenim" ("The Pesticide Manual", 12th edition, 2000, Nr. 389, S. 467-468) (= 1-(4-Chlorphenyl)-2,2,2-trifluor-1-ethanon-O-(1,3-dioxolan-2-ylmethyl)-oxim, das als Saatbeiz-Safener für Hirse gegen Schäden von Metolachlor bekannt ist, und "Cyometrinil" oder "-CGA-43089" ("The Pesticide Manual", 12th edition, 2000, Nr. 974, S. 983) (= (Z)-Cyanomethoxyimino(phenyl)acetonitril), das als Saatbeiz-Safener für Hirse gegen Schäden von Metolachlor bekannt ist,
l) Wirkstoffe vom Typ der Thiazolcarbonsäureester, die als Saatbeizmittel bekannt sind, wie z. B.
   "Flurazol" ("The Pesticide Manual", 12th edition, 2000, Nr. 376, S. 450 -451) (= 2-Chlor-4-trifluormethyl-1,3-thiazol-5-carbonsäurebenzylester), das als Saatbeiz-Safener für Hirse gegen Schäden von Alachlor und Metolachlor bekannt ist,
m) Wirkstofe vom Typ der Naphthalindicarbonsäurederivative die als Saatbeizmittel bekannt sind, wie z. B.
   "Naphthalic anhydrid" ("The Pesticide Manual", 12th edition, 2000, Nr. 1249, S. 1009-1010) (= 1,8-Naphthalindicarbonsäureanhydrid), das als Saatbeiz-Safener für Mais gegen Schäden von Thiocarbamatherbiziden bekannt ist,
n) Wirkstoffe vom Typ Chromanessigsäurederivate, wie z. B.
   "CL 304415" (CAS-Reg. Nr. 31541-57-8) (= 2-(4-Carboxy-chroman-4-yl)-essigsäure von der Firma American Cyanamid), das als Safener für Mais gegen Schäden von Imidazolinonen bekannt ist,
o) Wirkstoffe, die neben einer herbiziden Wirkung gegen Schadpflanzen auch Safenerwirkung an Kulturpflanzen wie Reis aufweisen, wie z. B.
   "Dimepiperate" oder "MY-93".("The Pesticide Manual", 12th edition, 2000, Nr. 251, S. 302-303) (= Piperidin-1-thiocarbonsäure-S-1-methyl-1-phenylethylester), das als Safener für Reis gegen Schäden des Herbizids Molinate bekannt ist,
   "Daimuron" oder "SK 23" ("The Pesticide Manual", 12th edition, 2000, Nr. 207, S. 247) (= 1-(1-Methyl-1-phenylethyl)-3-p-tolyl-harnstoff), das als Safener für Reis gegen Schäden des Herbizids Imazosulfuron bekannt ist,
   "Cumyluron" = "JC-940" (= 3-(2-Chlorphenylmethyl)-1-(1-methyl-1-phenyl-ethyl)-harnstoff, siehe JP-A-60087254), das als Safener für Reis gegen Schäden einiger Herbizide bekannt ist,
   "Methoxyphenon" oder "NK 049" (= 3,3'-Dimethyl-4-methoxy-benzophenon), das als Safener für Reis gegen Schäden einiger Herbizide bekannt ist,
   "CSB" (= 1-Brom-4-(chlormethylsulfonyl)-benzol) (CAS-Reg. Nr. 54091-06-4 von Kumiai), das als Safener gegen Schäden einiger Herbizide in Reis bekannt ist,
p) N-Acylsulfonamide der Formel (S3) und ihre Salze, 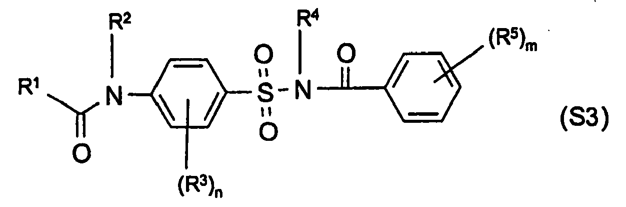 wie sie in WO-A-97/45016 beschrieben sind,
q) Acylsulfamoylbenzoesäureamide der allgemeinen Formel (S4), gegebenenfalls auch in Salzform, 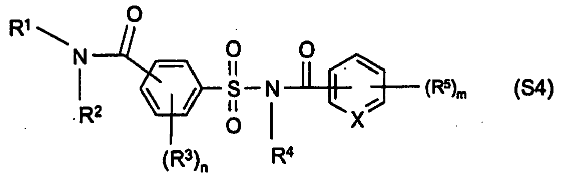 wie sie in der Internationalen Anmeldung Nr. PCT/EP98/06097 beschrieben sind, und
r) Verbindungen der Formel (S5), 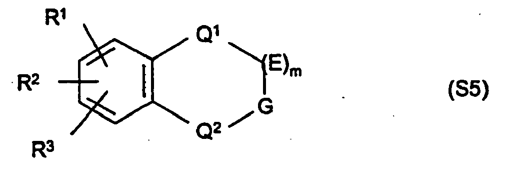 wie sie in der WO-A 98/13 361 beschrieben sind,
   einschließlich der Stereoisomeren und den in der Landwirtschaft gebräuchlichen Salzen.

Von besonderem Interesse sind unter den genannten Safenem sind (S1-1) und (S1-9) und (S2-1), insbesondere (S1-1) und (S1-9).
Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen (I) oder deren Mischungen mit weiteren Herbiziden/Pestiziden formuliert werden und als Fertigformulierung oder Tankmischung mit den Herbiziden bereitgestellt und angewendet werden.
Zur Anwendung werden die in handelsüblicher Form vorliegenden Herbizid- oder Herbizid-Safener-Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha, insbesondere zwischen 0,01 und 1 kg/ha Aktivsubstanz.

### A. Chemische Beispiele

### Beispiel 1

### 2-Amino-4-(5,7-dimethyl-1,2,3,4-tetrahydro-naphthalin-1-amino)-1,3,5-triazin

a) 27,0 g (0,13 mol) 1-Amino-5,7-dimethyl-1,2,3,4-tetrahydro-naphthalinhydrochlorid und 11,5 g (0,13 mol) Cyanoguanidin werden homogenisiert und in 60 ml 1,2-Dichlorbenzol aufgenommen. Diese Mischung wird 150 Minuten auf 140-160 °C erhitzt, hierbei entsteht zunächst ein homogenes Gemisch, welches sich dann aber wieder entmischt. Nach Abkühlen und Zugabe von 100 ml Toluol können nach Absaugen 40,0 g (94 % d.Th mit einer Reinheit größer 90 %) 1-Biguanidino-5,7-dimethyl-1,2,3,4-tetrahydro-naphthalin-hydrochlorid mit einem Schmelzpunkt von 215-216 °C erhalten werden.
b) Zu einer Natriummethanolatlösung aus 1,01 g (0,044 mol) Natrium in 50 ml Methanol werden 3,70 g (0,013 mol) 1-Biguanidino-5,7-dimethyl-1,2,3,4-tetrahydronaphthalin-hydrochlorid gegeben. Nachfolgend fügt man 3,7 g (0,03 mol) Ameisensäureethylester hinzu und rührt 15 Stunden bei 25 °C. Zu der Reaktionsmischung wird Wasser und Methylenchlorid gegeben, die oranische Phase wird abgetrennt, die wässrige Phase noch zweimal mit Methylenchlorid extrahiert. Die organische Phase wird jeweils abgetrennt und gemeinsam mit Natriumsulfat getrocknet. Das Trockenmittel wird abfiltiriert und Methylenchloridphase eingedampft. Man erhält nach säulenchromatographischer Trennung über Kiselgel mit Essigester als Laufmittel 2,3 g (62 % d.Th. bei 90 % Reinheit) N2-(5,7-dimethyl-1,2,3,4-tetrahydro-1-naphtalenyl)-1,3,5-triazine-2,4-diamine mit einem Schmelzpunkt von 178-180 °C.

Die in den Tabellen 1 und 2 beschriebenen Verbindungen erhält man analog zu dem vorstehenden Beispiel 1.

In der Tabelle bedeuten:

| | |
|---|---|
| Nr. | = Beispiel oder Beispielnummer |
| Fp. | = Festpunkt (Schmelzpunkt) in °C |
| Zustand | = Aggregatzustand oder andere Angabe (Konsistenz etc.), |
| Me | = Methyl |
| Et | = Ethyl |
| Pr | = Propyl |
| i-Pr | = Isopropyl |
| X-i-Pr | = sub.-Isopropyl |
| | z.B. F-i-Pr = Fluorisopropyl, d.h. -CF(CH₃)₂ der Formel 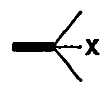 |
| c-Pr | = Cyclopropyl |
| 1-Me-c-Pr | = 1-Methyl-cyclopropyl = 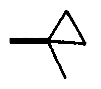 |

| | |
|---|---|
| t-Bu | = tertiär-Butyl |
| Ph | = Phenyl |
| Bz | = Benzyl |
| Ac | = Acetyl |
| Ziffern | = 1. Ziffer vor Substituenten am Bicyclus bedeutet Position des Substituenten Rest X" mit gleicher Nummer n. |
| Morpholino | = -morpholin = 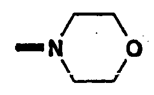 |
| Piperidino | = -piperidin = 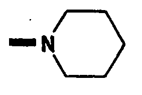 |

### Anmerkung:

Der fette Strich bezeichnet jeweils die Bindungsstelle der Reste.

**Tabelle 1: Verbindungen der Formel (I-A)**

| 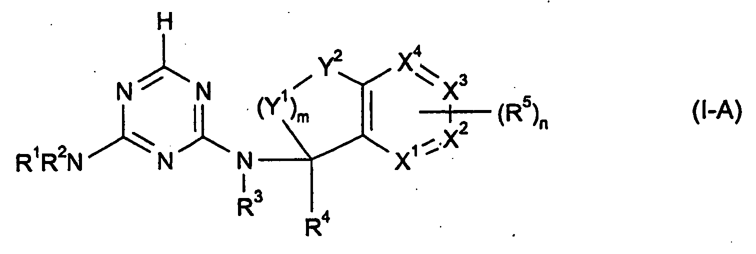 | | | | | | | | |
|---|---|---|---|---|---|---|---|---|
| Nr. | NR¹R² | R³ | R⁴ | -X¹=X²-X³=X⁴- | (R⁵)ₙ | (Y¹)ₘ | Y² | Zustand/Fₚ. (°C) |
| 1 | NH₂ | H | H | -C=C-C=C- | 2-Me, 4-Me | -CH₂-CH₂- | -CH₂- | 178-180 |
| 2 | NH₂ | H | H | -C=C-C=C- | 2-Me, 4-Me | -CMeH-CH₂- | -CH₂- | |
| 3 | NH₂ | H | H | -C=C-S- | 2-Me | -CH₂-CH₂- | -CH₂- | |
| 4 | NH₂ | H | H | -C=C-C=C- | 2-Me, 4-Me | -CH₂-CH₂- | -O- | 119-121 |
| 5 | NH₂ | H | H | -C=C-C=C- | 4-Me | -CH₂-CH₂₋ | -O- | 106-108 |
| 6 | NH₂ | H | H | -C=C-C=C- | 1-Me, 4-Me | -CH₂-CH₂- | -O- | |
| 7 | NH₂ | H | H | -C=C-C=C- | 2-Me, 4-Me | -CH₂-CH₂- | -NMe- | |
| 8 | NH₂ | H | H | -C=C-C=C- | 4-Me | -CH₂-CH₂- | -NMe- | |
| 9 | NHC(=O)-CH₂-CH₂-Cl | H | H | -C=C-C=C- | 2-Me, 4-Me | -CH₂-CH₂- | -NMe- | |
| 10 | NHC(=O)-CH₂-CH₂-Cl | H | H | -C=C-C=C- | 2-Me, 4-Me | -CH₂-CH₂- | -C(=O)- | |
| 11 | NH₂ | H | H | -C=C-C=C- | 2-Me, 4-Me | -CH₂-CH₂- | C(=O)- | |
| 12 | NHC(=O)-CH₂-OCH₃ | H | H | -C=C-C=C- | 2-Me, 4-Me | -CH₂-CH₂- | C(=O)- | |
| 13 | NHC(=O)-CH₂-OCH₃ | H | H | -C=C-C=C- | 2-Me, 4-Me | -CH₂-CH₂- | CHOH | |
| 14 | NH₂ | H | H | -C=C-C=C- | 2-Me, 4-Me | -CH₂-CH₂- | CHOH | |
| 15 | NHC(=O)CH₃ | H | H | -C=C-C=C- | 2-Me, 4-Me | -CH₂-CH₂- | CHOH | |
| 16 | NH₂ | H | H | -C=C-C=C- | 1-Me, 2-Me, 4-Me | -CH₂-CH₂- | -CH₂- | |
| 17 | NHC(=O)-CH₃ | H | H | -C=C-C=C- | 1-Me, 2-Me, 4-Me | -CH₂-CH₂- | -CH₂- | |
| 18 | NH2 | H | H | -C=C-C=C- | 1-Me, 3-Me, 4-Me | -CH₂-CH₂- | -CH₂- | |
| 19 | NH₂ | H | H | -C=C-C=C- | 3-Me, 4-Me | -CH₂-CH₂- | -CH₂- | 208 - 211 |
| 20 | NH₂ | H | H | -C=C-C=C- | 3-F, 4-Me | -CH₂-CH₂- | -CH₂- | |
| 21 | NH₂ | H | H | -C=C-C=C- | 3-Cl, 4-Me | -CH₂-CH₂- | -CH₂- | |
| 22 | NH₂ | H | H | -C=C-C=C- | 2-Et | -CH₂-CH₂- | -CH₂- | |
| 23 | NH₂ | H | H | -C=C-C=C- | 2-Et, 4-Me | -CH₂-CH₂- | -CH₂- | |
| 24 | NH₂ | H | H | -C=C-C=C- | 2-(1'-Fluor)- | -CH₂-CH₂- | -CH₂- | |
| | | | | | Et, 4-Me | | | |
| 25 | NHMe | H | H | -C=C-C=C- | 2-Me, 4-Me | -CH₂-CH₂- | -CH₂- | |
| 26 | NMe₂ | H | H | -C=C-C=C- | 2-Me, 4-Me | -CH₂-CH₂- | -CH₂- | |
| 27 | NMe(CO)Me | H | H | -C=C-C=C- | 2-Me, 4-Me | -CH₂-CH₂- | -CH₂- | |
| 28 | NH₂ | H | H | -C=C-C=C- | 2-F, 4-Me | -CMeH-CH₂- | -CH₂- | |
| 29 | NMeH | H | H | -C=C-C=C- | 2-Me, 4-Me | -CMeH-CH₂- | -CH₂- | |
| 30 | NH₂ | H | H | -C=C-C=C- | 2-Me, 4-Me | -CH₂-CH₂- | - | fester Schaum |
| 31 | NH₂ | H | H | -C=C-C=C- | 3-Me, 4-Me | -CH₂-CH₂- | - | |
| 32 | NH₂ | H | H | -C=C-C=C- | 2-Me, 3-Me | -CH₂-CH₂- | - | fester Schaum |
| 33 | NH₂ | H | H | -C=C-C=C- | 2-Me, 3-Me, 4-Me | -CH₂-CH₂- | - | fester Schaum |
| 34 | NH₂ | H | H | -C=C-C=C- | 1-Me, 4-Me | -CH₂-CH₂- | - | |
| 35 | NH₂ | H | H | -C=C-C=C- | 3-Me, 4-Me | -CH₂- | -O- | |
| 36 | NH₂ | H | H | -C=C-C=C- | 1-Me, 4-Me | -CH₂- | -O- | |
| 37 | NH₂ | H | H | -C=C-C=C- | 2-Me, 4-Me | -CH₂- | -O- | |
| 38 | NH₂ | H | H | -C=C-C=C- | 2-Me | -CH₂- | -O- | 184-190 |
| 39 | NH₂ | H | H | -C=C-C=C- | 2-Me | -CH₂- | -NMe- | |
| 40 | NH₂ | H | H | -C=C-C=C- | 2-Me, 4-Me | -CH₂- | -NMe- | |
| 41 | NH₂ | H | H | -C=C-C=C- | 1-Me, 2-Me | -CH₂- | -NMe- | |
| 42 | NH₂ | H | H | -C=C-C=C- | 2-Me, 3-OMe 4-Me | -CH₂-CH₂- | -NMe- | |
| 43 | NH₂ | H | H | -C=C-C=C-. | 1-Me, 2-Me | -CH₂- | -CHMe- | |
| 44 | NH₂ | H | H | -C=C-C=C- | 2-Me | -CH₂- | -CHMe- | |
| 45 | NH₂ | H | H | -C=C-C=C- | 3-Me | -CH₂- | -CHMe- | |
| 46 | CH₂ | H | H | -C=C-C=C- | 3-Me, 4-Me | -CH₂- | -CHMe- | |
| 47 | NH₂ | H | H | -C=C-C=C- | 1-Me, 3-Me | -CH₂- | -CHMe- | |
| 48 | NH₂ | H | H | -C=C-C=C- | 2-Me | -CHMe- | -CHMe- | |
| 49 | NH₂ | H | H | -C=C-C=C- | 2-Me, 4-Me | -CHMe- | CHMe- | |
| 50 | NH₂ | H | H | -C=C-C=C- | 1-Me, 4-Me | -CHMe- | -CHMe- | |
| 51 | NH₂ | H | H | -C=C-C=C- | 1-Et | -CHMe- | -CHMe- | |
| 52 | NH₂ | H | H | -C=C-C=C- | 2-Et | -CHMe- | -CHMe- | |
| 53 | NH₂ | H | H | -C=C-C=C- | 2-(1'-Fluor)-Et | -CHMe- | -CH₂- | |
| 54 | NH₂ | H | H | -C=C-C=C- | 3-(1'-Fluor)-Et | -CHMe- | -CH₂- | |
| 55 | NH₂ | H | H | -C=C-C=C- | 3-F | -CHMe- | -CH₂- | |
| 56 | NH₂ | H | H | -C=C-C=C- | 1-F | -CHMe- | -CH₂- | |
| 57 | NH₂ | H | H | -C=C-C=C- | 1-Cl | -CHMe- | -CH₂- | |
| 58 | NH₂ | H | H | -C=C-C=C- | 3-Cl | -CHMe- | -CH₂- 177-179 | |
| 59 | NMe₂ | H | H | -C=C-C=C- | 3-F | -CHMe- | -CH₂- | |
| 60 | NHC(=O)Me | H | H | -C=C-C=C- | 3-F | -CHMe- | -CH₂- | |
| 61 | NMeC(=O)Me | H | H | -C=C-C=C- | 3-F | -CHMe- | -CH₂- | |
| 62 | NHC(=O)CH₂-OMe | H | H | -C=C-C=C- | 3-F | -CHMe- | -CH₂- | |
| 63 | NHC(=O)CF₃ | H | H | -C=C-C=C- | 3-F | -CHMe- | -CH₂- | |
| 64 | NHC(=O)CH₂-CH₂-Cl | H | H | -C=C-C=C- | 3-F | -CHMe- | -CH₂- | |
| 65 | NHC(=O)CH=C H₂ | H | H | -C=C-C=C- | 3-F | -CHMe- | -CH₂- | |
| 66 | NHC(=O)Ph | H | H | -C=C-C=C- | 3-F | -CHMe- | -CH₂- | |
| 67 | NHC(=O)Me | H | H | -C=C-C=C- | 3-F | -CH₂- | -CH₂- | |
| 68 | NMeC(=O)Me | H | H | -C=C-C=C- | 3-F | -CH₂- | -CH₂- | |
| 69 | NHC(=O)CH₂-OMe | H | H | -C=C-C=C- | 3-F | -CH₂- | -CH₂- | |
| 70 | NHC(=O)CF₃ | H | H | -C=C-C=C- | 3-F | -CH₂- | -CH₂- | |
| 71 | NHC(=O)CH₂-CH₂-Cl | H | H | -C=C-C=C- | 3-F | -CH₂- | -CH₂- | |
| 72 | NHC(=O)CH=C H₂ | H | H | -C=C-C=C- | 3-F | -CH₂- | -CH₂- | |
| 73 | NHC(=O)Ph | H | H | -C=C-C=C- | 3-F | -CH₂- | -CH₂- | |
| 74 | NMe₂ | H | H | -C=C-C=C- | 3-F | -CH₂- | -CH₂- | |
| 75 | Morpholino | H | H | -C=C-C=C- | 3-F | -CH₂- | -CH₂- | |
| 76 | NHAc | H | H | -C=C-C=C- | 1-Me, 3-Me | -CH₂-CH₂- | -S- | |
| 77 | NHNH₂ | H | H | -C=C-C=C- | 1-Me, 3-Me | -CH₂-CH₂- | -S- | |
| 78 | NHPiperidino | H | H | -C=C-C=C- | 1-Me, 3-Me | -CH₂-CH₂- | -S- | |
| 79 | NH₂ | H | H | -C=C-C=C- | 2-Me, 4-Me | -CH₂-CH₂- | -S- | 114-116 |
| 80 | NH₂ | H | H | -C=C-C=C- | 1-Me, 2-Me, 3-Me | -CH₂-CH₂- | -S- | |
| 81 | NH₂ | H | H | -C=C-C=C- | 1Me, 3-Me | -CH₂-CHMe- | -S- | |
| 82 | NH₂ | H | H | -C=C-C=C- | 2-Me, 4-Me | -CH₂- | -S- | |
| 83 | NH₂ | H | H | -C=C-C=C- | 1-Me, 3-Me | -CMeH-CH₂- | -S- | |
| 84 | NH₂ | H | H | -C=C-C=C- | 4-Me | -CH₂-CHPh- | -S- | |
| 85 | NH₂ | H | H | -C=C-C=C- | 1-Me, 4-Me | -CH₂-CMe₂- | -S- | |
| 86 | NH₂ | H | H | -C=C-C=C- | 1-F, 3-F | -CH₂- | -S- | |
| 87 | NH₂ | H | H | -C=C-C=C- | 3-Me | -CH₂- | -S- | |
| 88 | NH₂ | H | H | -C=C-C=C- | 1-Me, 4-Me | -CH₂- | -S- | |
| 89 | NH₂ | H | H | -C=C-C=C- | 1-Me, 3-Cl | -CH₂- | -S- | |
| 90 | NMe₂ | H | H | -C=C-C=C- | 1-Me, 3-Me | -CH₂- | -S- | |
| 91 | Morpholino | H | H | -C=C-C=C- | 1-Me, 3-Me | -CH₂-CH₂- | -SO₂- | |
| 92 | NHAc | H | H | -C=C-C=C- | 1-Me, 3-Me | -CH₂- | -S- | |
| 93 | NHNH₂ | H | H | -C=C-C=C- | 1-Me, 3-Me | -CH₂- | -S- | |
| 94 | NHPiperidino | H | H | -C=C-C=C- | 1-Me, 3-Me | -CH₂- | -S- | |
| 95 | NH₂ | H | H | -C=C-C=C- | 2-Me, 4-Me | -CH₂- | -S- | |
| 96 | NH₂ | H | H | -C=C-C=C- | 1-Me, 3-F | -CH₂- | -S- | |
| 97 | NH₂ | H | H | -C=C-C=C- | 1 Me, 3-OMe | -CH₂- | -S- | |
| 98 | NMe₂ | H | H | -C=C-C=C- | 2-Me, 4-Me | -CH₂- | -0- | |
| 99 | NH₂ | H | H | -C=C-C=C- | 1-Me, 3-Me | -CMeH-CH₂- | -O- | |
| 100 | NH₂ | H | H | -C=C-C=C- | 4-Me | -CH₂-CHPh- | -O- | |
| 101 | NH₂ | H | H | -C=C-C=C- | 1-Me, 4-Me | -CH₂-CMe₂- | -O- | |
| 102 | NH₂ | H | H | -C=C-C=C- | 1-F, 3-F | -CH₂- | -O- | |
| 103 | NH₂ | H | H | -C=C-C=C- | 1-Me, 3-Me | -CH₂- | -O- | |
| 104 | NH₂ | H | H | -C=C-C=C- | 1-Me, 4-Me | -CH₂- | -CO- | |
| 105 | NHMe | H | H | -C=C-C=C- | 1-Me, 3-Me | -CH₂- | -O- | |
| 106 | NMe₂ | H | H | -C=C-C=C- | 1-Me, 3-Me | -CH₂- | -O- | |
| 107 | Morpholino | H | H | -C=C-C=C- | 1-Me, 3-Me | -CH₂-CH₂- | -O- | |
| 108 | NHAc | H | H | -C=C-C=C- | 1-Me, 3-Me | -CH₂- | -O- | |
| 109 | NHNH₂ | H | H | -C=C-C=C- | 1-Me, 3-Me | -CH₂- | -O- | |
| 110 | NHPiperidino | H | H | -C=C-C=C- | 1-Me, 3-Me | -CH₂- | -O- | |
| 111 | NH₂ | H | H | -C=C-C=C- | 2-Me, 4-Me | -CMeH- | -O- | |
| 112 | NH₂ | Me | H | -C=C-C=C- | 1-Me, 3-Me | -CH₂- | -O- | |
| 113 | NH₂ | H | Me | -C=C-C=C- | 1 Me, 3-Me | -CH₂- | -O- | |
| 114 | NH₂ | H | Me | -C=C-C=C- | 2-Me, 4-Me | -CH₂-CH₂- | -O- | |
| 115 | NH₂ | H | H | -C=C-C=C- | 1-Me, 2-OMe, 3-Me | -CH₂-CH₂- | -O- | |
| 116 | NHMe | H | H | -C=C-C=C- | 4-Me | -CH₂-CH₂- | -O- | |
| 117 | NH₂ | H | H | -C=C-C=C- | 1-Me, 2-OEt,4-Me | -CH₂-CH₂- | -O- | |
| 118 | NH₂ | H | H | -C=C-C=C- | 1-F, 3-F | -CH₂-CH₂- | -O- | |
| 119 | NH₂ | H | -CH₂-OMe | -C=C-C=C- | 1-Me, 3-Me | -CH₂-CH₂- | -O- | |
| 120 | NH₂ | H | H | -C=C-C=C- | 2-OEt, 4-Me | -CH₂-CH₂- | -O- | |
| 121 | NH₂ | H | H | -C=C-C=C- | 1-Me, 3-Me | -CH₂-CH₂- | -O- | |
| 122 | NMe₂ | H | H | -C=C-C=C- | 1-Me, 3-Me | -CH₂-CH₂ | -O- | |
| 123 | Morpholino | Me | H | -C=C-C=C- | 1-Me, 3-Me | -CH₂-CH₂- | -O- | |
| 124 | NHAc | H | H | -C=C-C=C- | 1-Me, 3-Me | -CH₂-CH₂- | -O- | |
| 125 | NHNH₂ | H | H | -C=C-C=C- | 1-Me, 3-Me | -CH₂-CH₂- | -O- | |
| 126 | NHPiperidino | H | H | -C=C-C=C- | 1-Me, 3-Me | -CH₂-CH₂- | -O- | |
| 127 | NH₂ | H | H | -C=C-C=C- | 2-Me, 3-Me, 4-Me | -CH₂-CH₂- | -O- | |
| 128 | NH₂ | H | H | -C=C-C=C- | 2-Me, 3-Me | -CH₂-CH₂- | -O- | |
| 129 | NH₂ | H | H | -C=C-C=C- | 1Me, 3-Me | -CH₂-CHMe- | -O- | |
| 130 | NH₂ | H | H | -C=C-C=C- | 2-Me, 4-Me | -CH₂-CH₂- | -NH- | |
| 131 | NH₂ | H | H | -C=C-C=C- | 1-Me, 3-Me | -CH₂-CH₂- | -NMe- | |
| 132 | NH₂ | H | H | -C=C-C=C- | 4-Me | -CH₂-CH₂- | -NH- | |
| 133 | NH₂ | H | H | -C=C-C=C- | 1-Me, 4-Me | -CH₂-CH₂- | -NAc- | |
| 134 | NH₂ | H | H | -C=C-C=C- | 1-F, 3-F | -CH₂-CH₂- | -NBz- | |
| 135 | NH₂ | H | H | -C=C-C=C- | 1-Me, 3-Me | -CH₂-CH₂- | -NH- | |
| 136 | NH₂ | H | H | -C=C-C=C- | 1-Me, 4-Me | -CH₂-CH₂- | -N(=O)- | |
| 137 | NH₂ | H | H | -C=N-C=N- | 1-Me, 3-Me | -CH₂-CH₂- | -NH- | |
| 138 | NMe₂ | H | H | -C=C-C=C- | 1-Me, 3-Me | -CH₂-CH₂- | -NPh- | |
| 139 | Morpholino | H | H | -C=C-C=C- | 1-Me, 3-Me | -CH₂-CH₂- | -NEt- | |
| 140 | NHAc | H | H | -C=C-C=C- | 1-Me, 3-Me | -CH₂-CH₂- | -N-i-Pr- | |
| 141 | NHNH₂ | H | H | -C=C-C=C- | 1-Me, 3-Me | -CH₂-CH₂- | -N- | |
| | | | | | | | n-Pr- | |
| 142 | NHPiperidio | H | H | -C=C-C=C- | 1-Me, 3-Me | -CH₂-CH₂- | -NH- | |
| 143 | NH₂ | H | H | -S-C=C- | 4-Me | -CH₂-CH₂- | -NH- | |
| 144 | NH₂ | H | H | -C=C-S- | 1-Me, | -CH₂-CH₂- | -NH- | |
| 145 | NH₂ | H | H | -C=C-C=C- | 1Me, 3-Me | -CH₂-CHMe- | -NPh- | |
| 146 | NH₂ | H | H | -C=C-C=C- | 2-Me | -CH₂-CH₂- | - | fester Schaum |
| 146-R | NH₂ | H | H | -C=C-C=C- | 2-Me | -CH₂-CH₂- | - | 141-143; opt. Rot.: 93.1°, c=1, CHCl₃, 22°C; EE: 95,7 % |
| 146-S | NH₂ | H | H | -C=C-C=C- | 2-Me | -CH₂-CH₂- | - | 141-143; opt. Rot.: - 94.4°, c=1, CHCl₃, 22°C; EE: 90,2% |
| 147 | NH₂ | H | H | -C=C-C=C- | 2-Et | -CH₂-CH₂- | - | |
| 148 | NH₂ | H | H | -C=C-C=C- | 2-i-Pr | -CH₂-CH₂- | - | |
| 149 | NH₂ | H | H | -C=C-C=C- | 2-OMe, 4-Me | -CH₂CH₂- | - | |
| 150 | NH-Piperidino | H | H | -C=C-C=C- | 2-Me, 4-Me | -CH₂-CH₂- | - | |
| 151 | NH₂ | H | H | -C=C-C=C- | 2-Me, 4-Ph | -CH₂- | -O- | |
| 152 | NH₂ | H | H | -C=C-C=C- | 2-F, 4-Me | -CH₂-CH₂- | - | |
| 153 | NH₂ | H | H | -C=C-C=C- | 2-Me, 4-Ph | -CH₂-CH₂- | - | |
| 154 | NH₂ | H | H | -C=C-C=C- | 2-Me, 3-Cl, 4-Cl | -CH₂-CH₂- | - | |
| 155 | NH₂ | H | H | -C=C-C=C- | 2-Me, 4-OMe | -CH₂-CH₂- | - | |
| 156 | NH₂ | H | H | -C=C-C=C- | 2-Me, 4-F | -CH₂-CH₂- | - | |
| 157 | NH₂ | Me | Me | -C=C-C=C- | 2-Me, 4-Me | -CH₂-CH₂- | - | |
| 158 | NH₂ | H | H | -C=C-C=C- | 2-Et, 4-Me | -CH₂-CH₂- | - | |
| 159 | NH₂ | H | H | -C=C-C=C- | 2-i-Pr, 4-Me | -CH₂-CH₂- | - | |
| 160 | NH₂ | H | H | -C=C-C=C- | 2-Me, 4-Me | -CMeH-CH₂- | - | 127-129 |
| 161 | NH₂ | H | H | -C=C-C=C- | 1-Me, 4-Cl | -CH₂-CH₂- | - | |
| 162 | NHC(=O)-CH₂-CH₂-Cl | H | H | -C=C-C=C- | 1-Me, 4-Me | -CH₂-CH₂- | - | |
| 163 | NH₂ | Ac | H | -C=C-C=C- | 1-Me, 4-Me | -CH₂-CH₂- | - | |
| 164 | NH₂ | H | H | -C=C-C=C- | 2-Me, 3-F | -CH₂-CH₂- | - | 108-115 |
| 165 | NH₂ | H | H | -C=C-C=C- | 2-Cl, 3-Me | -CH₂-CH₂- | - | |
| 166 | NH₂ | H | H | -C=C-C=C- | 2-CH₂-CH₂- CH₂-3 | -CH₂-CH₂- | - | |
| 167 | NH₂ | H | H | -C=C-C=C- | 3-Et | -CH₂-CH₂- | - | |
| 168 | NH₂ | H | H | -O-C=C- | 3-Me | -CH₂-CH₂- | - | |
| 169 | NH₂ | H | H | -C=C-C=C- | 1-Me, 3-Et | -CH₂-CH₂- | - | |
| 170 | NH₂ | H | H | -C=C-C=C- | 1-Me, 3-Me | -CH₂-CH₂- | - | |
| 171 | NH₂ | H | H | -C=C-C=C- | 2-Me, 4-Me | -CH₂-CH₂- | - | |
| 172 | NH₂ | H | H | -C=C-C=C- | 1-Me, 3-Me, 4-Me | -CH₂-CH₂- | - | |
| 173 | NH₂ | H | H | -N=C-C=C- | 2-Me, 4-Me | -CH₂-CH₂- | - | |
| 174 | NH₂ | H | H | -N=C-C=C- | 3-Me | -CH₂-CH₂- | - | |
| 175 | NH₂ | H | H | -C=C-C=N- | 1-Me, 3-Me | -CH₂-CH₂- | - | |
| 176 | NH₂ | Me | H | -C=C-C=C- | 2-Me | -CH₂-CH₂- | - | |
| 177 | NH₂ | H | H | -C=C-C=C- | 2-Et, 3-Me | -CH₂-CH₂- | - | |
| 178 | NH₂ | H | H | -C=C-C=C- | 3-COOMe | -CH₂-CH₂- | - | |
| 179 | NH₂ | H | H | -C=C-C=C- | 2-COOEt | -CH₂-CH₂- | - | |
| 180 | NH₂ | H | H | -C=C-C=C- | 2-CF₃ | -CH₂-CH₂- | - | |
| 181 | NH₂ | H | H | -C=C-C=C- | 2-c-Propyl | -CH₂-CH₂- | - | |
| 182 | NH₂ | H | H | -C=C-C=C- | 2-CClF₃ | -CH₂-CH₂- | - | |
| 183 | NH₂ | H | H | -C=C-C=C- | 1-Me, 2-Me, 3-Me | -CH₂-CH₂- | - | |
| 184 | NH₂ | H | H | -C=C-C=C- | 2-CH₂-OMe | -CH₂-CH₂- | - | |
| 185 | NH₂ | H | H | -C=C-C=C- | H | -CH₂-CH₂- | - | 183-185 |
| 185-R | NH₂ | H | H | -C=C-C=C- | H | -CH₂-CH₂- | - | 149-151; opt. Rot.: 93.3°, c=1, CHCl₃, 21°C; |
| 185-S | NH₂ | H | H | -C=C-C=C- | H | -CH₂-CH₂- | - | 149-151; opt. Rot.: -84,6°, c=1, CHCl₃, 21°C; |
| 186 | NH₂ | H | H | -C=C-C=C- | 2-Ac | -CH₂=CH₂ | - | |
| 187 | NH₂ | H | H | -C=C-C=C- | 2-Ac, 4-Me | -CH₂-CH₂- | - | |
| 188 | NH₂ | H | H | -C=C-C=C- | 2-CN | -CH₂-CH₂- | - | |
| 189 | NH₂ | H | H | -C=C-C=C | 2-Ph | -CH₂-CH₂- | - | |
| 190 | NH₂ | H | H | -C=C-C=C- | 2-(2-Thienyl) | -CH₂-CH₂- | - | |
| 191 | NH₂ | H | H | -C=C-C=C- | 4-Ph | -CH₂-CH₂- | - | |
| 192 | NHPh | H | H | -C=C-C=C- | 2-Me | -CH₂-CH₂- | - | |
| 193 | NH₂ | Ph | H | -C=C-C=C- | 2-Me | -CH₂-CH₂- | - | |
| 194 | NH₂ | C(=O)-CH₂-OMe | H | -C=C-C=C- | 1-Me | -CH₂-CH₂- | - | |
| 195 | NHBz | H | H | -C=C-C=C- | 2-Me, 3-Me | -CH₂-CH₂- | - | |
| 196 | NH₂ | H | H | -C=C-C=C- | 1-Me, 2-F, 4-Me | -CH₂-CH₂- | - | |
| 197 | NH₂ | H | H | -C=C-C=C- | 3-F, 4-Me | -CH₂-CH₂- | - | 122-123 |
| 198 | NH₂ | H | H | -C=C-C=C- | H | -CHMe-CH₂ | - | 103-105 |
| 199 | NH₂ | CH₂-OMe | H | -C=C-C=C- | H | -CH₂-CH₂- | - | |
| 200 | NH₂ | CH₂- CH₂-OMe | H | -C=C-C=C- | H | -CH₂-CH₂- | - | |
| 201 | NH₂ | Ac | H | -C=C-C=C- | H | -CH₂-CH₂- | - | |
| 202 | NH₂ | H | H | -S-C=C- | H | -CH₂-CH₂- | - | |
| 203 | NH₂ | H | H | -C=C-C=C- | H | -CH₂ | -O- | 188-192 |
| 204 | NH₂ | H | H | -C=C-C=C- | 2-OH | -CH₂-CH₂- | - | |
| 205 | NH₂ | H | H | -C=C-C=C- | 3-CH=CH₂ | -CH₂-CH₂- | - | |
| 206 | NH₂ | Bz | H | -C=C-C=C- | H | -CH₂-CH₂- | - | |
| 207 | NH₂ | H | Me | -C=C-C=C- | H | -CH₂-CH₂- | - | |
| 208 | NH₂ | H | H | -C=C-C=C- | 3-C≡CH | -CH₂-CH₂- | - | |
| 209 | NH₂ | Ph | H | -C=C-C=C- | H | -CH₂-CH₂- | - | |
| 210 | NH₂ | H | H | -C=C-C=C- | 2-CH≡CH | -CH₂-CH₂- | - | |
| 211 | NH₂ | H | H | -C=C-C=C- | 2-CH=CH₂ | -CH₂-CH₂- | - | |
| 212 | NH₂ | H | H | -C=C-C=C- | 2-CH=CH₂ | -CMeH-CH₂- | - | |
| 213 | NH₂ | H | H | -C=C-C=C- | 2-1 | -CH₂-CH₂- | - | |
| 214 | NH₂ | H | H | -C=C-C=C- | 3-1 | -CH₂-CH₂- | - | |
| 215 | NH₂ | CHO | H | -C=C-C=C- | H | -CH₂-CH₂- | - | |
| 216 | NH₂ | H | H | -C=C-C=C- | 2-Br | -CH₂-CH₂- | - | |
| 217 | NH₂ | H | H | -C=C-C=C- | 3-Br | -CH₂-CH₂- | - | |
| 218 | NH₂ | H | H | -C=C-C=C- | 2-NHCHO | -CH₂-CH₂- | - | |
| 219 | NH₂ | H | H | -C=C-C=C- | 2-NHAc | -CH₂-CH₂- | - | |
| 220 | NH₂ | H | H | -C=C-C=C- | 2-NHCO-Ph | -CH₂-CH₂- | - | |
| 221 | NH₂ | H | H | -C=C-C=C- | 2-NHPh | -CH₂-CH₂- | - | |
| 222 | NH₂ | H | H | -C=C-C=C- | 2-Ph, 4-Me | -CH₂-CH₂- | - | |
| 223 | NH₂ | H | H | -C=C-C=C- | 2-O-Ph | -CH₂-CH₂- | - | |
| 224 | NH₂ | H | H | -C=C-C=C- | 2-OMe | -CH₂-CH₂- | - | |
| 225 | NH₂ | H | H | -C=C-C=C- | 2-OEt | -CH₂-CH₂- | - | |
| 226 | NH₂ | H | H | -C=C-C=C- | 2-O-i-Pr | -CH₂-CH₂- | - | |
| 227 | NH₂ | H | H | -C=C-C=C- | 4-OMe | -CH₂-CH₂- | - | |
| 228 | NH₂ | H | H | -C=C-C=C- | 4-OEt | -CH₂-CH₂- | - | |
| 229 | NH₂ | H | H | -C=C-C=C- | 4-O-i-Pr | -CH₂-CH₂- | - | |
| 230 | NH₂ | H | H | -C=N-C=N | H . | -CH₂-CH₂- | - | |
| 231 | NH₂ | H | H | -N=C-N=C- | H | -CH₂-CH₂- | - | |
| 232 | NH₂ | H | H | -C=N-N=C- | H | -CH₂-CH₂- | - | |
| 233 | NH₂ | H | H | -N=C-C=N- | H | -CH₂-CH₂- | - | |
| 234 | NH₂ | H | H | N=C-N=C | 2-Me | -CH₂-CH₂- | - | |
| 235 | NH₂ | H | H | -N=C-N=C- | 2-CF₃ | -CH₂-CH₂- | - | |
| 236 | NH₂ | H | H | -N=C-N=C- | 2-(1-Fluoro)-Et | -CH₂-CH₂- | - | |
| 237 | NH₂ | H | H | -N=C-N=C- | 2-(1-Fluoro)-i-Pr | -CH₂-CH₂- | - | |
| 238 | NH₂ | H | H | -N=C-N=C- | 4-OMe | -CH₂-CH₂- | - | |
| 239 | NH₂ | H | H | -N=C-N=C- | 2-OMe | -CH₂-CH₂- | - | |
| 240 | NH₂ | H | H | -N=C-N=C- | 2Me, 4-OMe | -CH₂-CH₂- | - | |
| 241 | NH₂ | H | H | -N=C-N=C- | 2-OMe, 4-OMe | -CH₂-cH₂- | - | |
| 242 | NHAc | H | H | -N=C-N=C- | 2-OMe, 4-OMe | -CH₂-CH₂- | - | |
| 243 | NHAc | H | H | -N=C-C=C- | 2-OMe | -CH₂-CH₂- | - | |
| 244 | NH₂ | H | H | -N=C-C=C- | 2-OMe | -CH₂-CH₂- | - | |
| 245 | NH₂ | H | H | -N=C-C=C- | 4-OMe | -CH₂-CH₂- | - | |
| 246 | NH₂ | H | H | -C=C-C=N- | 2-OMe | -CH₂-CH₂- | - | |
| 247 | NH₂ | H | H | -C=C-C=N- | H | -CH₂-CH₂- | - | |
| 248 | NH₂ | CHO | H | -C=C-C=N- | H | -CH₂-CH₂- | - | |
| 249 | NH₂ | H | -CH₂-OMe | -C=C-C=C- | H | -CH₂-CH₂- | - | |
| 250 | NH₂ | H | Me | -C=C-C=C- | H | CH₂ | O | |
| 251 | NH₂ | H | H | -C=C-C=C- | H | CH₂ | NMe | |
| 252 | NH₂ | H | H | -C=C-C=C- | 1-Me, 4-Me | CH₂ | O | |
| 253 | NH₂ | H | H | -C=C-C=C- | 1-Me, 4-F | CHMeCH₂ | | |
| 254 | NH₂ | Me | COO-Me | -C=C-C=C- | 2-Me, 4-Cl | CH₂CPhH | O | |
| 255 | NH₂ | Me | CN | -C=C-C=C- | 2-Me, 4-Me | CH₂ | CH₂ | |
| 256 | NH₂ | Ac | Ac | -C=C-C=C- | 2-Me, 4-Me | CH₂ | CH₂ | |
| 257 | NH₂ | CHO | Me | -C=C-C=C- | 2-Me, 4-Me | CH₂ | CH₂ | |
| 258 | NMe₂ | H | H | -C=C-C=C- | 2-Me, 4-Me | CH₂ | CH₂ | |
| 259 | NHCHO | CH₂O-Me | H | -C=C-C=C- | 2-Me, 4-CF₃ | CH₂ | - | |
| 260 | Morpholino | Bz | Me | -C=C-C=C- | 1-Me, 4-OMe | CH₂-CHMe | NMe | |
| 261 | Piperidino | H | H | -C=C-C=C- | 3-OMe | CH₂-CH₂ | CHMe | |
| 262 | NHAc | H | H | -C=C-C=C- | 2-Me, 4-Me | CH₂-CHPh | - | |
| 263 | NHNH₂ | Me | H | -C=C-C=C- | 3-Me, 4-Me | CH₂-CHMe | S | |
| 264 | NEt₂ | H | H | -C=C-C=C- | 3-Me, 4-Et | CHMe-CH₂ | S | |
| 265 | NHPh | H | H | -C=C-C=C- | 2-Ac, 4-Me | CH₂-CH₂ | S | |
| 266 | NHBz | H | H | -C=C-C=C- | 3-OPh | CH₂-CH₂ | S | |
| 267 | NHCOPh | H | H | -C=C-C=C- | 3-OCOPh | CH₂CH₂ | O | |
| 268 | NH₂ | 4-Cl-Bz | H | -C=C-C=C- | 2-Me, 4-Me | CH₂-CH₂ | O | |
| 269 | NH₂ | H | Me | -C=C-C=C- | 2-Me, 4-Me | CH₂-CH₂ | S | |
| 270 | NH₂ | H | Et | -C=C-C=C- | 2-Me, 4-OEt | CH₂-CH₂ | S | |
| 271 | NH₂ | H | H | -C=C-C=N- | 2-Me | CH₂-CH₂ | S | |
| 272 | NH₂ | H | H | -C=C-C=N- | H | -CH₂-CH₂- | - | |
| 273 | NHMe | H | H | -N=C-N- | 3-Me | -CH₂-CH₂- | CH₂ | |
| 274 | NHMe | H | H | -N=C-N- | 2-Me, 3-Me | -CH₂-CH₂- | CH₂ | |
| 275 | NH₂ | H | H | -N=C-N- | 3-Me | -CH₂-CH₂- | CH₂ | |
| 276 | NH₂ | H | H | -N=C-N- | 2-Me, 3-Me | -CH₂-CH₂- | CH₂ | |
| 277 | NHMe | H | H | -N=C-N- | 3-Me | -CH₂-CH₂- | **-** | |
| 278 | NHMe | H | H | -N=C-N- | 2-Me, 3-Me | -CH₂-CH₂- | - | |
| 279 | NH₂ | H | H | -N=C-N- | 3-Me | -CH₂-CH₂- | - | |
| 280 | NH₂ | H | H | -N=C-N- | 2-Me, 3-Me | -CH₂-CH₂- | - | |
| 281 | NH₂ | H | H | -C=C-C=C- | 2-Me, | -CHMe-CH₂- | - | 198-200 |
| 282 | NH₂ | H | H | -C=C-C=C- | 2-F, | -CHMe-CH₂- | - | 95-97 |
| 283 | NHAc | H | H | -C=C-C=C- | 2-F, | -CHMe-CH₂- | - | 175-177 |
| 284 | NHC(=O)CCl₃ | H | H | -C=C-C=C- | 2-F, | -CHMe-CH₂- | - | |
| 285 | NHC(=O)CF₃ | H | H | -C=C-C=C- | 2-F, | -CHMe-CH₂- | - | wachsartig |
| 286 | NHC(=O)CH₂Cl | H | H | -C=C-C=C- | 2-F, | -CHMe-CH₂- | - | wachsartig |
| 287 | NHC(=O)C₂H₅ | H | H | -C=C-C=C- | 2-F, | -CHMe-CH₂- | - | |
| 288 | NH₂ | H | H | -C=C-C=C- | 2-F, 4-Me | -CH₂-CH₂- | O | 96-99 |
| 289 | NH₂ | H | H | -C=C-C=C- | 2-F, | -CH₂-CH₂- | O | 92-94 |
| 290 | NH₂ | H | H | -C=C-C=C- | 1-Me | -CHMe-CH₂- | - | fester Schaum |
| 291 | NH₂ | H | H | -C=C-C=C- | 2-Me | -CHEt-CH₂- | - | fester Schaum |
| 292 | NH₂ | H | H | -C=C-C=C- | 2-Me | -CMe₂-CH₂- | - | 186-188 |
| 293 | NH₂ | H | H | -C=C-C=C- | 2-Me, 3-Me | -CHMe-CH₂- | - | 155-160 |
| 294 | NH₂ | H | H | -C=C-C=C- | 4-Me | -CHMe-CH₂- | - | |
| 295 | NH₂ | H | H | -C=C-C=C- | H | CH₂CH₂CH₂ | O | |
| 296 | NH₂ | H | H | -C=C-C=C- | 1,2-Me, 4-F | -CH₂-CH₂- | - | 211- 212 |
| 297 | NH₂ | H | H | -C=C-C=C- | 1,4-Cl | -CH₂-CH₂- | - | 210 |
| 297 | NH₂ | H | H | -C=C-C=C- | 1-Me | -CH₂-CH₂- | - | fester Schaum |
| 297 | NH₂ | H | H | -C=C-C=C- | 1,4-F | -CH₂-CH₂- | - | 158-161 |
| 197 | NH₂ | H | H | -C=C-C=C- | 1-F, 3-Me | -CH₂-CH₂- | - | 194-204 |
| 297 | NH₂ | H | H | -C=C-C=C- | 2,3-F | -CH₂-CH₂- | - | Öl |
| 297 | NH₂ | H | H | -C=C-C=C- | 2,3,4-Me | -CHCH₃- | -CH₂- | fester Schaum |
| 298 | NH₂ | H | H | -C=C-C=C- | 4-OMe | -CH₂- | -O- | fester Schaum |
| 299 | NH₂ | H | H | -C=C-C=C- | 1.2-Me | -CH₂-CH₂- | - | Öl |
| 300 | NH₂ | H | H | -C=C-C=C- | 3-Me, 4-Cl | -CH₂-CH₂- | - | 217-218 |
| 301 | NHC(=O)CH₃ | H | H | -C=C-C=C- | 2-Me | -CH₂-CH₂- | -O- | 186-188 |
| 302 | NH₂ | H | H | -C=C-C=C- | 2-Me | -CH₂-CH₂- | -O- | 102-104 |
| 303 | NH₂ | H | H | -C=C-C=C- | 4-Me | -CHCH₃- | -CH₂- | fester Schaum |

**Tabelle 2: Verbindungen der Formel (I-B)**

| 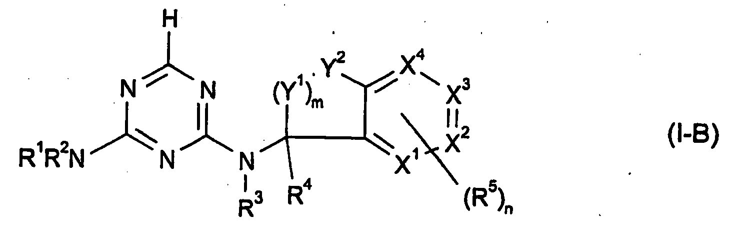 | | | | | | | | |
|---|---|---|---|---|---|---|---|---|
| Nr. | NR¹R² | R³ | R⁴ | =X¹-X²=X³X4⁴= | (R⁵)ₙ | (Y¹)ₘ. | Y² | Zustand / Fp. (°C) |
| B-1 | NH₂ | H | H | =N-S-C= | H | -CH₂-CH₂- | - | |
| B-2 | NH₂ | H | H | =N-S-C= | 4-Me | -CH₂-CH₂- | - | |
| B-3 | NH₂ | H | H | =N-S-C= | 4-OMe | -CH₂-CH₂- | - | |
| B-4 | NH₂ | H | H | =N-O-C= | H | -CH₂-CH₂- | - | |
| B-5 | NH₂ | H | H | =N-O-C= | 4-Me | -CH₂-CH₂- | - | |
| B-6 | NH₂ | H | H | -=N-O-C= | 4-OMe | -CH₂-CH₂- | - | |
| B-7 | NH₂ | H | H | =C-O-N= | H | -CH₂-CH₂- | - | |
| B-8 | NH₂ | H | H | =C-O-N= | 2-Me | -CH₂-CH₂- | - | |
| B-9 | NH₂ | H | H | =C-O-N= | 2-OMe | -CH₂-CH₂- | - | |
| B-10 | NH₂ | H | H | =N-O-N= | H | -CH₂-CH₂- | - | |
| B-11 | NH₂ | H | H | =N-S-N= | H | -CH₂-CH₂- | - | |
| B-12 | NHMe | H | H | =N-S-C= | H | -CH₂-CH₂- | - | |
| B3-13 | NH₂ | H | H | =N-S-C= | H | -CH₂-CH₂- | CH₂ | |
| B-14 | NH₂ | H | H | =N-S-C= | 4-Me | -CH₂-CH₂- | CH₂ | |
| B-15 | NH₂ | H | H | =N-S-C= | 4-OMe | -CH₂-CH₂- | CH₂ | |
| B-16 | NH₂ | H | H | =N-O-C= | H | -CH₂-CH₂- | CH₂ | |
| B-17 | NH₂ | H | H | =N-O-C=. | 4-Me | -CH₂CH₂- | CH₂ | |
| B-18 | NH₂ | H | H | =N-O-C= | 4-OMe | -CH₂-CH₂- | CH₂ | |
| B-19 | NH₂ | H | H | =C-O-N= | H | -CH₂-CH₂- | CH₂ | |
| B-20 | NH₂ | H | H | =C-O-N= | 2-Me | -CH₂-CH₂- | CH₂ | |
| B-21 | NH₂ | H | H | =C-O-N= | 2-OMe | -CH₂CH₂- | CH₂ | |
| B-22 | NH₂ | H | H | =N-O-N= | H | -CH₂-CH₂- | CH₂ | |
| B-23 | NH₂ | H | H | =N-S-N= | H | -CH₂-CH₂- | CH₂ | |
| B-24 | NHMe | H | H | =N-S-C= | H | -CH₂-CH₂- | CH₂ | |

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz-und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (^{®}Triton X 207), 3 Gew.-Teilen Isotridecanolpolyglykolether (8 EO) und 71 Gew.-Teilen paraffinischem Mineralöl (Siedebereich z.B. ca. 255 bis über 277 °C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gewichtsteile einer Verbindung der Formel (I),
   10 " ligninsulfonsaures Calcium,
   5 " Natriumlaurylsulfat,
   3 " Polyvinylalkohol und
   7 " Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man
   25 Gewichtsteile einer Verbindung der Formel (I),
   5 " 2,2'-dinaphthylmethan-6,6'-disulfonsaures Natrium
   2 " oleoylmethyltaurinsaures Natrium,
   1 Gewichtsteil Polyvinylalkohol,
   17 Gewichtsteile Calciumcarbonat und
   50 " Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Unkrautwirkung im Vorauflauf

Samen bzw. Rhizomstücke von mono- und dikotylen Unkrautpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 1/ha in unterschiedlichen Dosierungen auf die Oberfläche der Abdeckerde appliziert.
Nach der Behandlung werden die Töpfe im Gewächshaus aufgestellt und unter guten Wachstumsbedingungen für die Unkräuter gehalten. Die optische Bonitur der Pflanzen- bzw. Auflaufschäden erfolgt nach dem Auflaufen der unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise zeigen die Beispiele Nr. 1, 4, 5, 19, 55, 58, 79, 146, 185, 198, 281, 282, 283, 285,286, 288, 289, 290, 291, 292 und 293 (s. Tabellen 1 und 2) im Test sehr gute herbizide Wirkung gegen Schadpflanzen wie Sinapis alba, Chrysanthemum segetum, Avena sativa, Stellaria media, Echinochloa crus-galli, Lolium multiflorum, Setaria spp., Abutilon theophrasti, Amaranthus retrofluxus und Panicum miliaceum im Vorauflaufverfahren bei einer Aufwandmenge von 0,5 kg und weniger Aktivsubstanz pro Hektar.

### 2. Unkrautwirkung im Nachauflauf

Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt. Die als Spritzpulver bzw. als Emulsionskonzentrate formulierten erfindungsgemäßen Verbindungen werden in verschiedenen Dosierungen mit einer Wasseraufwandmenge von umgerechnet 600 bis 800 I/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise zeigen die Beispiele 1, 4, 5, 19, 55, 58, 79, 146,185, 198, 281, 282, 283, 285,286, 288, 289, 290, 291, 292 und 293 (s. Tabellen 1 und 2) im Test sehr gute herbizide Wirkung gegen Schadpflanzen wie Sinapis alba, Echinochloa crus-galli, Lolium multiflorum, Chrysanthemum segetum, Setaria spp., Abutilon theophrasti, Amaranthus retroflexus und Panicum miliaceum, Avena sativa im Nachauflaufverfahren bei einer Aufwandmenge von 0,5 kg und weniger Aktivsubstanz pro Hektar.

### 3. Wirkung auf Schadpflanzen in Reis

Verpflanzter und gesäter Reis sowie typische Reisunkräuter werden im Gewächshaus bis zum Dreiblattstadium (Echinochloa 1,5-Blatt) unter Paddyreis-Bedingungen (Anstauhöhe des Wassers: 2-3 cm) in geschlossenen Plastiktöpfen angezogen. Danach erfolgt die Behandlung mit den erfindungsgemäßen Verbindungen. Hierzu werden die formulierten Wirkstoffe in Wasser suspendiert, gelöst bzw. emulgiert und mittels Gießapplikation in das Anstauwasser der Test pflanzen in unterschiedlichen Dosierungen ausgebracht.
Nach der so durchgeführten Behandlung werden die Versuchspflanzen im Gewächshaus unter optimalen Wachstumsbedingungen aufgestellt und während der gesamten Versuchszeit so gehalten.
Etwa drei Wochen nach der Applikation erfolgt die Auswertung mittels optischer Bonitur der Pflanzenschäden im Vergleich zu unbehandelten Kontrollen, wobei beispielsweise die Verbindungen Nr. 1, 4, 5, 19, 55, 58, 79, 146, 185, 198, 281, 282, 283, 285,286, 288, 289, 290, 291, 292 und 293 (s. Tabellen 1 und 2) sehr gute herbizide Wirkung gegen Schadpflanzen zeigen, die typisch für Reiskulturen sind, wie z.B. Cyperus monti, Echinochloa crus-galli, Eleocharis acicularis und Sagittaria pygmaea.

### 4. Kulturpflanzenverträglichkeit

In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, dass die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt lassen. Einige Substanzen schonen darüber hinaus aus Gramineen-Kulturen wie z.B. Gerste, Weizen, Roggen, Sorghum-Hirsen, Mais oder Reis. Unter anderen zeigen die Verbindungen Nr. 1,4,5,19,55, 58, 79, 146, 185, 198, 281, 282, 283, 285,286, 288, 289, 290, 291, 292 und 293 (s. Tabellen 1 und 2) entsprechend der Formel (I) teilweise eine hohe Selektivität und eignen sich deshalb zur Bekämpfung von unerwünschten Pflanzenwuchs in landwirtschaftlichen Kulturen.

## Patentansprüche

1. Verbindungen der Formel (I) oder deren Salze, 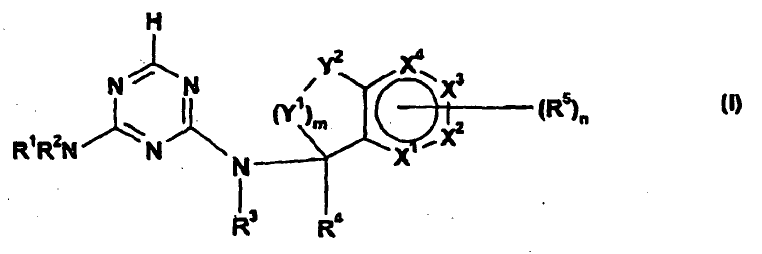 worin
R¹ und R² jeweils unabhängig voneinander Wasserstoff, Amino, Alkylamino oder Dialkylamino mit jeweils 1 bis 4 C-Atomen im Alkylrest oder (C₃-C₈)Cycloalkylamino, (C₅-C₈)Cycloalkenyl-amino, einen (acyclischen oder cyclischen) Kohtenwasserstoffrest oder Kohlenwasserstoffoxyrest mit jeweils 1 bis 10 C-Atomen, vorzugsweise mit 1 bis 6 Atomen, oder einen Heterocydylrest, Heterocyclyloxyrest, Heterocyclylthiorest oder Heterocyclylaminorest mit jeweils 3 bis 9 Ringatomen, vorzugsweise 3 bis 6 Ringatomen, und 1 bis 3 Heteroringatomen aus der Gruppe N, O und S, wobei jeder der sechs letztgenannten Reste unsubstituiert oder durch einen oder mehrere Reste aus der Gruppe Halogen, Hydroxy, Amino, Nitro, Formyl, Aminocarbonyl, Carboxy, Sulfonyl, Cyano, Thiocyanato, (C₁-C₆)Alkoxy, (C₁-C₆)Alkenyloxy, (C₂-C₆)Alkinyloxy, (C₁-C₆)Alkoxy-poly(alkylenoxy), Hydroxy-poly(alkylenoxy), (C₁-C₆)Alkylthio, Mono- und Di[(C₁-C₆)alkyl]amino, [(C₁-C₈)Alkyl]carbonyl, [(C₁-C₆)Alkenyl]carbonyl, [(C₂-C₆)Alkinyl]carbonyl, [(C₁C₆)Alkoxy]carbonyl, [(C₂-C₆)Alkenyloxy]carbonyl, [(C₂-C₆)Alkinyloxy]carbonyl, Mono- und Di-[(C₁-C₆)alkyl]amino-carbonyl, Phenyl, Phenoxy, (C₃-C₆)Cycloalkyl, Phenylcarbonyl. Phenoxycarbonyl, Heterocydyl, (C₁-C₄)Alkylsulfonyl und im Falle cyclischer Reste auch (C₁-C₄)Alkyl substituiert ist.
wobei jeder der tetztgenannten 24 Substituenten unsubstituiert oder durch einen oder mehrere Reste aus der Gruppe Halogen, CN, SCN, NO₂, (C₁-C₄)Alkoxy, (C₁-C₄)Alkylthio, (C₁-C₄)Haloalkoxy, Formyl, (C₁-C₄)Alkylcarbonyl, [(C₁-C₄)Haloalkyl]carbonyl;
[(C₁-C₄)Alkoxy]carbonyl. [(C₁-C₄)Haloalkoxylcarbonyl und im Falle cydischer Reste auch (C₁₋C₄)Alkyl und (C₁-C₄)Haloalkyl substituiert ist.
oder einen Acylrest einer organischen Säure aus der Gruppe Formyl, Aminocarbonyl, Mono- oder Di-[(C₁-C₄)alkyl]amino-carbonyl, [(C₁-C₆)Alkyl]carbonyl, [(C₂C₈)Alkenyl]carbonyl, [(C₂-C₆)Alkinyl]carbonyl. [(C₁-C₆)Alkoxy]carbonyl, [(C₂-C₆)Alkenyloxy]carbonyl, Phenylcarbonyl, (C₁C₆)Alkylsulfonyl, wobei jeder der letztgenannten 9 Reste im aliphatischen Teil oder im Phenylring unsubstituiert oder durch einen oder mehrere Reste aus der Gruppe Halogen, CN, SCN, NO₂, (C₁-C₄)Alkoxy, (C₁-C₄)Haloalkoxy, gegebenenfalls substituiertes und vorzugsweise unsubstituiertes Phenyl und im Falle cyclischer Reste auch (C₁-C₆)Alkyl und (C₁-C₆)Haloalkyl substituiert ist,
wobei Heterocyclyl, wenn nicht näher definiert, auch in zusammengesetzten Resten jeweils 3 bis 9 Ringatome, vorzugsweise 3 bis 6 Ringatome, und 1 bis 3 Heteroringatome aus der Gruppe N. O und S aufweist, der
R¹ und R² gemeinsam mit dem Stickstoffatom der Gruppe NR¹R² einen gesättigten oder ungesättigten, nicht aromatischen heterocyclischen Rest mit 3 bis 6 Ringatomen und 1 bis 4 Heteroringatomen, wobei neben dem N-Atom die gegebenenfalls weiteren Heteroringatome aus der Gruppe N, O und S ausgewählt sind und der Rest unsubstituiert oder durch einen oder mehrere Reste aus der Gruppe Halogen, CN, (C₁-C₄)Alkyl, (C₁-C₄)Haloalkyl. (C₁-C₄)Alkoxy, (C₁-C₄)Haloalkoxy,(C₁-C₄)Alkoxy-carbonyl und Oxo substituiert ist,
wobei jeder der kohlenstoffhaltigen Reste R¹ und R² inklusive Substituenten 1 bis 20 C-Atome aufweist.
R³ Wasserstoff, Amino, (C₁-C₆)Alkyl, (C₁-C₆)Alkoxy, (C₂-C₆)Alkenyl, (C₂-C₆)Alkinyl, (C₃-C₆)Cycloalkyl, (C₅-C₆)Cycloalkenyl, Phenyl oder Heterocyclyl, wobei jeder der acht letztgenannten Reste unsubstituiert oder durch einen oder mehrere Reste aus der Gruppe Halogen. Hydroxy, Amino. Nitro, Formyl, Carboxy, Cyano, Thiocyanato. (C₁-C₄)Alkoxy, (C₁-C₄)Alkoxy-(C₁-C₄)alkoxy, Hydroxy-(C₁-C₄)alkoxy, (C₁-C₄)Alkoxy-poly(alkylenoxy), Hydroxy-poly(alkylenoxy), (C₁-C₄)Haloalkoxy, (C₁-C₄)Alkylthio, (C₁-C₄)Haloalkylthio, Mono(C₁-C₄)alkylamino, Di(C₁-C₄)alkylamino, [(C₁-C₄)Alkylcarbonyl, [(C₁-C₄)Alkoxy]carbonyl, Aminocarbonyl, Mono(C₁-C₄)alkylamino-carbonyl, Di(C₁-C₄)alkylamino-carbonyl, Phenyl, Phenoxy, (C₃-C₆)Cycloalkyl, Phenylcarbonyl, Phenoxycarbonyl, Heterocyclyl, wobei jeder der letztgenannten 8 Reste unsubstituiert oder durch einen oder mehrere Reste aus der Gruppe Halogen, CN, SCN, NO₂, (C₁-C₄)Alkyl, (C₁-C₄)Haloalkyl, (C₁-C₄)Alkoxy und (C₁-C₄)Haloalkoxy substituiert ist und (C₁-C₄)Alkylsulfonyl und (C₁-C₄)Haloalkylsulfonyl und im Falle cyclischer Reste auch (C₁-C₄)Alkyl und (C₁-C₄)Haloalkyl substituiert ist, oder einen Acylrest der Formel -B*-A*, worin
A* Wasserstoff oder (C₁-C₆)Alkyl, (C₂-C₆)Alkenyl, (C₂-C₆)Alkinyl, (C₃-C₆)Cycloalkyl, (C₅-C₆)Cycloalkenyl oder Phenyl, wobei jeder der sechs letztgenannten Reste unsubstituiert oder durch einen oder mehrere Reste aus der Gruppe Halogen, Hydroxy, Amino, Nitro, Formyl, Carboxy, Cyano, Thiocyanato, (C₁-C₄)Alkoxy, (C₁-C₄)Haloalkoxy, (C₁-C₄)Alkylthio, Mono(C₁-C₄)alkylamino, Di(C₁-C₄)alkylamino. Phenyl und (C₃-C₈)Cycloalkyl,wobei jeder der letztgenannten 2 Reste unsubstituiert oder durch einen oder mehrere Reste aus der Gruppe Halogen,CN, SCN, NO₂, (C₁₋C₄)Alkyl, (C₁-C₄)Haloalkyl, (C₁-C₄)Alkoxy und (C₁-₄)Haloalkoxy substituiert ist, und im Falle cyclischer Reste auch (C₁-C₄)Alkyl und (C₁-C₄)Haloalkyl substituiert ist, und
B* eine divalente Gruppe der Formel -CO-, -CO-O-, -CO-NR'-, -S(O)ₚ- oder -S(O)ₚ-O-, wobei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 4 C-Atomen ist,
bedeuten,
wobei -B*-A* inklusive Substituenten 1 bis 12 C-Atome aufweist,
R⁴ einen Rest der Formel -Z¹-R⁶, wobei Z¹ und R⁶ wie unten angegeben definiert sind.
R⁵ jeweils unabhängig voneinander Halogen, Cyano, Isocyanato, Nitro, einen Rest der Formel -Z²-R⁷, wobei Z² und R⁷ wie unten angegeben definiert sind, oder zwei benachbarte Reste R⁵ gemeinsam einen ankondensierten Cyclus mit 4 bis 6 Ringatomen, der carbocyclisch ist oder 1 bis 3 Heteroringatome aus der Gruppe O, S und N enthält und der unsubstituiert oder durch einen oder mehrere Reste aus der Gruppe Halogen, (C₁-C₄)Alkyl und Oxo substituiert ist,
R⁶ und R⁷, R⁸, R⁹ jeweils unabhängig voneinander Wasserstoff, ausgenommen R⁷ = Wasserstoff. wenn Z² = direkte Bindung, oder einen acyclischen Kohlenwasserstoffrest mit 1 bis 10 C-Atomen oder einen cyclischen Kohlenwasserstoffrest mit 3 bis 6 C-Atomen oder einen heterocyclischen Rest, wobei jeder der letztgenannten kohlenstoffhaltigen Reste unsubstituiert oder durch einen oder mehrere Reste aus der Gruppe Halogen, Hydroxy, Amino. Nitro, Formyl, Aminocarbonyl, Carboxy, Sulfonyl, Cyano, Thiocyanato, (C₁-C₆)Alkoxy, (C₂-C₆)Alkenyloxy, (C₂-C₈)Alkinyloxy, (C₁-C₆)Alkoxy-poly(alkylenoxy), Hydroxy-poly(alkylenoxy), (C₁-C₈)Alkylthio, Mono- und Di[(C₁-C₈)alkyl]amino, [(C₁-C₆)Alkyl]carbonyl, [(C₂-C₆)Alkenyl]carbonyl, [(C₂-C₆)Alkinyl]carbonyl, [(C₁-C₆)Alkoxylcarbonyl, [(C₂-C₆)Alkenyloxy]carbonyl, [(C₂-C₆)Alkinyloxy]carbonyl, Mono- und Di-[(C₁-C₆)alkyl]amino-carbonyl, Phenyl, Phenoxy, (C₃-C₆)Cycloalkyl, Phenylcarbonyl, Phenoxycarbonyl, Heterocyclyl, (C₁-C₄)Alkylsulfonyl und im Falle cyclischer Reste auch (C₁-C₄)Alkyl substituiert ist,
wobei jeder der letztgenannten 24 Substituenten unsubstituiert oder durch einen oder mehrere Reste aus der Gruppe Halogen, CN, SCN, NO₂, (C₁-C₄)Alkoxy, (C₁-C₄)Alkylthio, (C₁-C₄)Haloalkoxy, Formyl, (C₁-C₄)Alkylcarbonyl, [(C₁-C₄)Haloalkyl]carbonyl, [(C₁-C₄)Alkoxyl]carbonyl, [(C₁-C₄)Haloalkoxy]carbonyl und im Falle cyclischer Reste auch (C₁-C₄)Alkyl und (C₁-C₄)Haloalkyl substituiert ist,
oder R⁸ und R⁹ zusammen mit dem C-Atom einer Gruppe CR⁸R⁹ (for Y¹ oder Y²) oder zwei Reste R⁸ oder R⁹ aus zwei Gruppen Y¹ und/oder Y² gemeinsam mit den verknüpften Atomen der Gruppen Y¹ und/oder Y² jeweils einen carbocyclischen Rest mit 3 bis 8 C-Atomen oder einen heterocyclischen Rest, wobei jeder der zwei letztgenannten Reste unsubstituiert oder durch einen oder mehrere Reste aus der Gruppe Halogen, (C₁-C₄)Alkyl, (C₁-C₄)Haloalkyl, (C,-C₄)Alkoxy, (C₁-C₄)Haloalkoxy, Nitro und Cyano substituiert ist,
X¹, X², X³, X⁴ jeweils unabhängig voneinander ein Kohlenstoffatom, das mit einem Wasserstoffatom oder einer der definierten Substituenten R⁵ substituiert ist. oder ein Stickstoffatom oder zwei benachbarte Symbole X¹, X², X³ und X⁴ jeweils gemeinsam eine divalente Gruppe der Formel -O-, -S-, -NH- oder -NR-, worin R wie für R³ definiert ist, vorausgesetzt die Gruppen X¹, X², X³, X⁴ bilden zusammen mit der verknüpften C₂-Einheit des ankondensierten Rings einen carbocyclischen oder heterocyclischen aromatischen fünfgliedrigen oder sechsgliedrigen Ring.
(Y¹)ₘ m divalente Gruppen Y¹, wobei jede Gruppe Y¹ unabhängig von anderen Resten Y¹ eine Gruppe der Formel -O-, -CO-, -C(=NR*)-, -S(O)_{q}-, -NR*- oder -N(O)-, wobei q = 0, 1 oder 2 ist und R* wie für R³ definiert ist, oder eine Gruppe der Formel CR⁸R⁹, wobei R⁸ und R⁹ wie oben definiert ist bedeutet, und
Y² eine Gruppe wie für Y¹ definiert oder eine direkte Bindung,
wobei zwei benachbarte Gruppen der Symbolpaare Y¹ und Y¹ oder der Symbolpaare Y¹ und Y² nicht heteroatomige Gruppen derselben Bedeutung darstellen und wobei die Gruppen (Y¹)ₘ und Y² zusammen mit der verknüpften C₂-Einheit des aromatischen Rings und dem mit R⁴ verknüpften C-Atom einen ankondensierten carbocyclischen oder heterocyclischen nicht aromatischen viergliedrigen bis achtgliedrigen Ring bilden,
Z¹ und die Gruppen Z² jeweils unabhängig voneinander eine direkte Bindung oder eine divalente Gruppe der Formel -O-, -S(O)p-, -S(O)ₚ-O-, -O-S(O)p-, -CO-, -O-CO-, -CO-O-, -NR'-, -O-NR'-, -NR'-O-, -NR'-CO-, -CO-NR'- bedeutet und dabei p = 0, 1 oder 2 ist und R' Wasserstoff, Alkyl mit 1 bis 8 C-Atomen, Phenyl, Benzyl, (C₃-C₈)Cycloalkyl oder Alkanoyl mit 1 bis 6 C-Atomen ist,
m 0, 1, 2, 3 oder 4 und
n 0, 1, 2, 3 oder 4
bedeuten.

2. Verbindungen oder deren Salze gemäß Anspruch 1, **dadurch gekennzeichnet, dass**
R¹ und R² jeweils unabhängig voneinander Wasserstoff, Amino, Alkylamino oder Dialkylamino mit jeweils 1 bis 4 C-Atomen im Alkylrest oder (C₃-C₈)Cycloalkylamino, (C₅-C₆)Cycloalkenyl-amino, einen Kohlenwasserstoffrest oder Kohlenwasserstoffoxyrest mit jeweils 1 bis 10 C-Atomen oder einen Heterocyclylrest, Heterocyclyloxyrest, Heterocyclylthiorest oder Heterocyclylaminorest mit jeweils 3 bis 9 Ringatomen und 1 bis 3 Heteroringatomen aus der Gruppe N, O und S, wobei jeder der sechs letztgenannten Reste unsubstituiert oder durch einen oder mehrere Reste aus der Gruppe Halogen, Hydroxy, Amino, Nitro, Formyl, Aminocarbonyl, Carboxy, Sulfonyl, Cyano, Thiocyanato, (C₁-C₆)Alkoxy, (C₂-C₈)Alkenyloxy, (C₂-C₆)Alkinyloxy, (C₁-C₈)Alkoxy-poly(alkylenoxy), Hydroxy-poly(alkylenoxy), (C₁-C₈)Alkylthio, Mono- und Di[(C₁-C₆)alkyl]amino, [(C₁-C₆)Alkyl]carbonyl, [(C₂-C₆)Alkenyl]carbonyl, [(C₂-C₆)Alkinyl]carbonyl, [(C₁-C₆)Alkoxy]carbonyl, [(C₂-C₆)Alkenyloxy]carbonyl, [(C₂-C₆)Alkinyloxy]carbonyl, Mono- und Di-[(C₁-C₆)alkyl]amino-carbonyl, Phenyl, Phenoxy, (C₃-C₆)Cycloalkyl, Phenylcarbonyl, Phenoxycarbonyl, Heterocyclyl, (C₁-C₄)Alkylsulfonyl und im Falle cyclischer Reste auch (C₁-C₄)Alkyl substituiert ist,
wobei jeder der letztgenannten 24 Substituenten unsubstituiert oder durch einen oder mehrere Reste aus der Gruppe Halogen, CN, SCN, NO₂, (C₁-C₄)Alkoxy, (C₁-C₄)Alkylthio, (C₁-C₄)Haloalkoxy, Formyl, (C₁-C₄)Alkylcarbonyl, [(C₁-C₄)Haloalkyl]carbonyl, [(C₁-C₄)Alkoxylcarbonyl, [(C₁-C₄)Haloalkoxy]carbonyl und im Falle cyclischer Reste auch (C₁-C₄)Alkyl und (C₁-C₄)Haloalkyl substituiert ist,
oder einen-Acylrest einer organischen Säure aus der Gruppe Formyl, Aminocarbonyl, Mono- oder Di(C₁-C₄)alkyl]amino-carbonyl, [(C₁-C₆)Alkyl]carbonyl, [(C₂-C₈)Alkenyl]carbonyl, [(C₂-C₆)Alkinyl]carbonyl, [(C₁-C₆)Alkoxy]carbonyl, [(C₂-C₈)Alkenyloxy]carbonyl, Phenylcarbonyl, (C₁-C₆)Alkylsulfonyl, wobei jeder der letztgenannten 9 Reste im aliphatischen Teil oder im Phenylring unsubstituiert oder durch einen oder mehrere Reste aus der Gruppe Halogen, CN, SCN, NO₂, (C₁-C₄)Alkoxy, (C₁-C₄)Haloalkoxy, gegebenenfalls substituiertes und unsubstituiertes Phenyl und im Falle cyclischer Reste auch (C₁-C₆)Alkyl und (C₁-C₆)Haloalkyl substituiert ist, oder R¹ und R² gemeinsam mit dem Stickstoffatom der Gruppe NR¹R² einen gesättigten oder ungesättigten, nicht aromatischen heterocyclischen Rest mit 3 bis 6 Ringatomen und 1 bis 4 Heteroringatomen, wobei neben dem N-Atom die gegebenenfalls weiteren Heteroringatome aus der Gruppe N, O und S ausgewählt sind und der Rest unsubstituiert oder durch einen oder mehrere Reste aus der Gruppe Halogen, CN, (C₁-C₄)Alkyl, (C₁-C₄)Haloalkyl, (C₁-C₄)Alkoxy, (C₁-C₄)Haloalkoxy, (C₁-C₄)Alkoxy-carbonyl und Oxo substituiert ist.
R³ Wasserstoff, Amino, (C₁-C₆)Alkyl, (C₁-C₈)Alkoxy, (C₂-C₆)Alkenyl, (C₂-C₆)Alkinyl, (C₃-C₆)Cycloalkyl, (C₅-C₆)Cycloalkenyl, Phenyl oder Heterocyclyl, wobei jeder der acht letztgenannten Reste unsubstituiert oder durch einen oder mehrere Reste aus der Gruppe Halogen, Hydroxy, Amino, Nitro, Formyl, Carboxy, Cyano, Thiocyanato, (C₁-C₄)Alkoxy, (C₁-C₄)Alkoxy-(C₁-C₄)alkoxy, Hydroxy-(C₁-C₄)alkoxy, (C₁-C₄)Alkoxy-poly(alkylenoxy), Hydroxy-poly(alkylenoxy), (C₁-C₄)Haloalkoxy, (C₁-C₄)Alkylthio, (C₁-C₄)Haloalkylthio, Mono(C₁-C₄)alkylamino, Di(C₁-C₄)alkylamino, [(C₁-C₄)Alkyl]carbonyl, [(C₁-C₄)Alkoxy]carbonyl, Aminocarbonyl, Mono(C₁-C₄)alkylamino-carbonyl, Di(C₁-C₄)alkylamino-carbonyl, Phenyl, Phenoxy. (C₃-C₆)Cycloalkyl, Phenylcarbonyl, Phenoxycarbonyl, Heterocyclyl, wobei jeder der letztgenannten 6 Reste unsubstituiert oder durch einen oder mehrere Reste aus der Gruppe Halogen, CN, SCN, NO₂, (C₁-C₄)Alkyl, (C₁-C₄)Haloalkyl, (C₁-C₄)Alkoxy und (C₁-C₄)Haloalkoxy substituiert ist, und (C₁-C₄)Alkylsulfonyl und (C₁-C₄)Haloalkylsulfonyl und im Falle cyclischer Reste auch (C₁₋C₄)Alkyl und (C₁-C₄)Haloalkyl substituiert ist,
oder einen Acylrest der Formel -B*-A*, worin
A* Wasserstoff oder (C₁-C₆)Alkyl, (C₂-C₆)Alkenyl, (C₂-C₆)Alkinyl, (C₃-C₆)Cycloalkyl, (C₅-C₆)Cycloalkenyl oder Phenyl, wobei jeder der sechs letztgenannten Reste unsubstituiert oder durch einen oder mehrere Reste aus der Gruppe Halogen, Hydroxy. Amino, Nitro, Formyl, Carboxy, Cyano, Thiocyanato, (C₁-C₄)Alkoxy, (C₁-C₄)Haloalkoxy, (C₁-C₄)Alkylthio, Mono(C₁-C₄)alkylamino, Di(C₁-C₄)alkylamino, Phenyl und (C₃-C₆)Cycloalkyl, wobei jeder der letztgenannten 2 Reste unsubstituiert oder durch einen oder mehrere Reste aus der Gruppe Halogen, CN, SCN, NO₂, (C₁-C₄)Alkyl, (C₁-C₄)Haloalkyl, (C₁-C₄)Alkoxy und (C₁-C₄)Haloalkoxy substituiert ist, und im Falle cyclischer Reste auch (C₁-C₄)Alkyl und (C₁-C₄)Haloalkyl substituiert ist, und
B* eine divalente Gruppe der Formel -CO-, -CO-O-, -CO-NR'-, -S(O)p- oder -S(O)p-O-, wobei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 4 C-Atomen ist,
wobei -B*-A* inklusive Substituenten 1 bis 12 C-Atome aufweist.
R⁴ einen Rest der Formel -Z¹-R⁶, wobei Z¹ und R⁶ wie unten angegeben definiert sind,
R⁵ jeweils unabhängig voneinander Halogen, CN, SCN, NO₂, einen Rest der Formel -Z²-R⁷, wobei Z² und R⁷ wie unten angegeben definiert sind, oder zwei benachbarte Reste R⁵ gemeinsam einen ankondensierten Cyclus mit 4 bis 6 Ringatomen, der carbocyclisch ist oder 1 bis 3 Heteroringatome aus der Gruppe O. S und N enthält und der unsubstituiert oder durch einen oder mehrere Reste aus der Gruppe Halogen, (C₁-C₄)Alkyl und Oxo substituiert ist, und
R⁶ und R⁷, R⁸, R⁹ jeweils unabhängig voneinander Wasserstoff, ausgenommen R⁷ = Wasserstoff, wenn Z² = direkte Bindung, oder einen acyclischen Kohlenwasserstoffrest mit 1 bis 10 C-Atomen oder einen cyclischen Kohlenwasserstoffrest mit 3 bis 6 C-Atomen oder einen heterocyclischen Rest, wobei jeder der letztgenannten 3 kohlenstoffhaltigen Reste unsubstituiert oder durch einen oder mehrere Reste aus der Gruppe Halogen, Hydroxy. Amino. Nitro, Formyl, Aminocarbonyl, Carboxy, Sulfonyl, Cyano, Thiocyanato, (C₁-C₆)Alkoxy, (C₂-C₆)Alkenyloxy, (C₂-C₆)Alkinyloxy, (C₁-C₆)Alkoxy-poly(alkylenoxy), Hydroxy-poly(alkylenoxy), (C₁-C₆)Alkylthio, Mono- und Di[(C₁-C₆)alkyl]amino, [(C₁-C₆)Alkyl]carbonyl, [(C₂-C₅)Alkenyl]carbonyl, [(C₂-C₆)Alkinyl]carbonyl, [(C₁-C₆)Alkoxyl]carbonyl, [(C₂-C₆)Alkenyloxy]carbonyl, [(C₂-C₆)Alkinyloxy]carbonyl, Mono- und Di-[(C₁-C₆)alkyl]amino-carbonyl, Phenyl, Phenoxy, (C₃-C₆)Cycloalkyl, Phenylcarbonyl, Phenoxycarbonyl, Heterocyclyl, (C₁-C₄)Alkylsulfonyl und im Falle cyclischer Reste auch (C₁-C₄)Alkyl substituiert ist,
wobei jeder der letztgenannten 24 Substituenten unsubstituiert oder durch einen oder mehrere Reste aus der Gruppe Halogen, CN, SCN, NO₂, (C₁-C₄)Alkoxy, (C₁-C₄)Alkylthio, (C₁-C₄)Haloalkoxy, Formyl. (C₁-C₄)Alkylcarbonyl, [(C₁-C₄)Haloalkyl]carbonyl, [(C₁-C₄)Alkoxy]carbonyl, [(C₁-C₄)Haloalkoxy]carbonyl und im Falle cyclischer Reste auch (C₁-C₄)Alkyl und (C₁-C₄)Haloalkyl substituiert ist,
X¹, X², X³, X⁴ jeweils unabhängig voneinander ein Kohlenstoffatom, das mit einem Wasserstoffatom oder einer der definierten Substituenten R⁵ substituiert ist, oder ein Stickstoffatom oder zwei benachbarte Symbole X¹, X², X³ und X⁴ jeweils gemeinsam eine divalente Gruppe der Formel -O-, -S-, -NH- oder -NR-, worin R wie für R³ definiert ist, vorausgesetzt die Gruppen X¹, X², X³, X⁴ bilden zusammen mit der verknüpften C₂-Einheit des ankondensierten Rings einen carbocyclischen oder heterocyclischen aromatischen fünfgliedrigen oder sechsgliedrigen Ring,
(Y¹)ₘ m divalente Gruppen Y¹. wobei jede Gruppe Y¹ unabhängig von anderen Resten Y¹ eine Gruppe der Formel -O-, -CO-, -C(=NR*)-, -S(O)q-, -NR*- oder -N(O)-, wobei q = 0, 1 oder 2 ist und R* wie für R³ definiert ist, oder eine Gruppe der Formel CR⁸R⁹ bedeutet,
Y² eine Gruppe wie für Y¹ definiert oder eine direkte Bindung,
wobei zwei benachbarte Gruppen der Symbolpaare Y¹ und Y¹ oder der Symbolpaare Y¹ und Y² nicht heteroatomige Gruppen derselben Bedeutung darstellen und wobei die Gruppen (Y¹)ₘ und Y² zusammen mit der verknüpften C₂-Einheit des aromatischen Rings und dem mit R⁴ verknüpften C-Atom einen ankondensierten carbocyclischen oder heterocyclischen nicht aromatischen viergliedrigen bis achtgliedrigen Ring bilden, und
Z¹ und die Gruppen Z² jeweils unabhängig voneinander eine direkte Bindung oder eine divalente Gruppe der Formel -O-, -S(O)p-, -S(O)p-O-, -O-S(O)p-, -CO-, -O-CO-, -CO-O-, -NR'-, -O-NR'-, -NR'-O-, -NR'-CO-, -CO-NR'- bedeuten und dabei p = 0, 1 oder 2 ist und R' Wasserstoff, Alkyl mit 1 bis 6 C-Atomen, Phenyl, Benzyl, Cycloalkyl mit 3 bis 6 C-Atomen oder Alkanoyl mit 1 bis 6 C-Atomen ist,
bedeuten,
wobei Heterocyclyl jeweils, wenn nicht naher definiert, auch in zusammengesetzten Resten jeweils 3 bis 9 Ringatome und 1 bis 3 Heteroringatome aus der Gruppe N, O und S aufweist.

3. Verbindungen oder deren Salze gemäß Anspruch 1 oder 2, **dadurch gekennzeichnet, dass**
R¹ und R² jeweils unabhängig voneinander Wasserstoff, Amino, Mono- oder Di[(C₁-C₄)alkyl]amino oder (C₃-C₆)Cycloalkyl-amino, (C₅-C₈)Cycloalkenyl-amino, einen Kohlenwasserstoffrest oder Kohlenwasserstoffoxyrest mit jeweils 1 bis 10 C-Atomen oder einen Heterocyclylrest, Heterocyclyloxyrest, Heterocyclylthiorest oder Heterocyclylaminorest mit jeweils 3 bis 9 Ringatomen und 1 bis 3 Heteroringatomen aus der Gruppe N, O und S, wobei jeder der sechs letztgenannten Reste unsubstituiert oder durch einen oder mehrere Reste aus der Gruppe Halogen, Hydroxy, Amino, Nitro, Formyl, Aminocarbonyl, Carboxy, Sulfonyl, Cyano, Thiocyanato, (C₁-C₄)Alkoxy, (C₂-C₄)Alkenyloxy, (C₂-C₄)Alkinyloxy, (C₁-C₄)Alkoxy-poly(alkylenoxy), Hydroxy-poly(alkylenoxy), (C₁-C₄)Alkylthio, Mono- und Di[(C₁-C₄)alkyl]amino, (C₁-C₄)Alkyl]carbonyl, [(C₂-C₄)Alkenyl]carbonyl, [(C₂-C₄)Alkinyl]carbonyl, [(C₁-C₄)Alkoxyl]carbonyl, [(C₂-C₄)Alkenyloxy]carbonyl, [(C₂-C₄)Alkinyloxy]carbonyl, Mono- und Di-[(C₁-C₄)alkyl]amino-carbonyl, Phenyl, Phenoxy, (C₃-C₆)Cycloalkyl, Phenylcarbonyl, Phenoxycarbonyl, Heterocyclyl, (C₁-C₄)Alkylsulfonyl und im Falle cyclischer Reste auch (C₁-C₄)Alkyl substituiert ist,
wobei jeder der letztgenannten 24 Substituenten unsubstituiert oder durch einen oder mehrere Reste aus der Gruppe Halogen, CN. SCN, NO₂, (C₁-C₄)Alkoxy, (C₁**-**C₄)Haloalkoxy, Formyl, (C₁-C₄)Alkylcarbonyl, [(C₁-C₄)Haloalky]carbonyl, [(C₁-C₄)Alkoxy]carbonyl, [(C₁-C₄)Haloalkoxy]carbonyl und im Falle cyclischer Reste auch (C₁-C₄)Alkyl und (C₁-C₄)Haloalkyl substituiert ist,
oder einen Acylrest einer organischen Säure aus der Gruppe Formyl, Aminocarbonyl, Mono- oder Di-[(C₁-C₄)alkyl]amino-carbonyl, [(C₁-C₄)Alkyl]carbonyl, [(C₂-C₄)Alkenyl]carbonyl, [(C₂-C₄)Alkinyl]carbonyl, [(C₁-C₄)Alkoxy]carbonyl, [(C₂-C₄)Alkenyloxy]carbonyl, Phenylcarbonyl, (C₁-C₄)Alkylsulfonyl, wobei jeder der letztgenannten 9 Reste im aliphatischen Teil oder im Phenylring unsubstituiert oder durch einen oder mehrere Reste aus der Gruppe Halogen, CN, SCN, NO₂, (C₁-C₄)Alkoxy, (C₁-C₄)Haloalkoxy, gegebenenfalls substituiertes und unsubstituiertes Phenyl und im Falle cyclischer Reste auch (C₁-C₄)Alkyl und (C₁-C₄)Haloalkyl substituiert ist,
oder
R¹ und R² gemeinsam mit dem Stickstoffatom der Gruppe NR¹R² einen gesättigten oder ungesättigten, nicht aromatischen heterocyclischen Rest mit 3 bis 6 Ringatomen und 1 bis 4 Heteroringatomen, wobei neben dem N-Atom die gegebenenfalls weiteren Heteroringatome aus der Gruppe N, O und S ausgewählt sind und der Rest unsubstituiert oder durch einen oder mehrere Reste aus der Gruppe Halogen, CN, (C₁-C₄)Alkyl, (C₁-C₄)Haloalkyl, (C₁-C₄)Alkoxy, (C₁-C₄)Haloalkoxy, (C₁-C₄)Alkoxy-carbonyl und Oxo substituiert ist,
R³ Wasserstoff, Amino, Formyl, (C₁-C₄)Alkyl, Cyano-(C₁-C₄)alkyl, (C₁-C₄)Haloalkyl, Mono-, Di- oder Poly-hydroxy-(C₁-C₄)alkyl, Hydroxy-poly-[(C₂-C₄)alkylenoxy]-(C₁-C₄)alkyl, Mono-, Di- oder Poly-(C₁-C₄)alkoxy-(C₁-C₄)alkyl, (C₁-C₄)Alkoxy-poly-[(C₂-C₄)alkylenoxy]-(C₁-C₄)alkyl. (C₁-C₄)Haloalkoxy-(C₁-C₄)alkyl, (C₂-C₆)Alkenyl, (C₂-C₆)Haloalkenyl, (C₂-C₆)Alkinyl, (C₂-C₆)Haloalkinyl oder Aminocarbonyl oder
(C₁-C₄)Alkylamino-(C₁-C₄)alkyl, Di-[(C₁-C₄)alkyl]-amino-(C₁-C₄)alkyl, (C₁-C₄)Alkylamino-carbonyl, Di(C₁-C₄)alkylamino-carbonyl, (C₁-C₅)Alkanoyl, [(C₂-C₄)Alkenyl]carbonyl, [(C₁-C₄)Alkoxy]carbonyl, (C₂-C₄)Alkenyloxy-carbonyl, Aminocarbonyl-(C₁-C₄)alkyl, (C₁-C₄)Alkylamino-carbonyl-(C₁-C₄)alkyl, Di[(C₁-C₄)alkyl]amino-carbonyl-(C₁-C₄)alkyl, (C₁-C₄)Alkoxy-carbonyl-(C₁-C₄)alkyl, (C₁-C₅)Alkanoyl-(C₁-C₄)alkyl, [(C₂-C₄)Alkenyl]carbonyl-(C₁-C₄)alkyl, [(C₁-C₄)Alkoxy]carbonyl-(C₁-C₄)alkyl oder (C₂-C₄)Alkenyloxy-carbonyl-(C₁-C₄)alkyl, wobei jeder der letztgenannten 16 Reste unsubstituiert oder durch einen oder mehrere Reste aus der Gruppe Halogen, CN, (C₁-C₄)Alkoxy und (C₁-C₄)Haloalkoxy substituiert ist, oder
(C₃-C₈)Cycloalkyl, (C₃-C₆)Cycloalkylamino-(C₁-C₄)alkyl, Phenyl, Phenylcarbonyl, Phenoxycarbonyl, Phenylcarbonyl-(C₁-C₄)alkyl, Phenoxy-(C₁-C₄)alkyl, Phenyl-(C₁-C₄)alkyl, Heterocyclyl, Heterocyclyl-(C₁-C₄)alkyl oder einen der letztgenannten 10 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio, (C₁-C₄)Haloalkyl, (C₁-C₄)Haloalkoxy, Formyl, (C₁-C₄)Alkyl-carbonyl, (C₁-C₄)Alkoxy-carbonyl und (C₁-C₄)Alkoxy substituiert ist,
R⁴ Wasserstoff, Formyl, Carboxy, Cyano, Thiocyanato, Aminocarbonyl, (C₁-C₄) Alkyl, Cyano-(C₁-C₄)alkyl, (C₁-C₄)Alkoxy, (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkenyl, Halo-(C₂-C₆)Alkenyl, (C₂-C₈)Alkinyl, Halo-(C₂-C₆)alkinyl, (C₁-C₄)Alkylamino-(C₁-C₄)alkyl, Di-[(C₁-C₄)alkyl]-amino-(C₁-C₄)alkyl, (C₃-C₉)Cycloalkylamino-(C₁-C₄)alkyl. (C₃-C₈)Cycloalkyl, Heterocyclyl-(C₁-C₄)alkyl mit 3 bis 6 Ringgliedern, wobei die cyclischen Gruppen in den letztgenannten 3 Resten unsubstituiert oder durch einen oder mehrere Reste aus der Gruppe (C₁-C₄)Alkyl. Halogen und Cyano substituiert sind, oder Phenyl, Phenoxy, Phenylcarbonyl. Phenylcarbonyl-(C₁-C₄)alkyl, Phenoxycarbonyl, (C₁-C₄)Alkyl-carbonyl, (C₁-C₄)Alkoxy-carbonyl, (C₁-C₄)Alkylamino-carbonyl, (C₁-C₄)Alkoxy-carbonyl-(C₁-C₄)alkyl, (C₁-C₄)Alkylamino-carbonyl-(C₁-C₄)alkyl, Phenoxy-(C₁-C₄)alkyl. Phenyl-(C₁-C₄)alkyl, Heterocyclyl, Hoterocyclylamino, Heterocyclyloxy, Heterocyclylthio, (C₁-C₅)Alkanoylamino. N-[(C₁-C₅)Alkanoyl]-N-[(C₁-C₄)alkyl]-amino, [(C₂-C₄)Alkenyl]carbonylamino, [(C₂-C₄)Alkinyl]carbonylamino, [(C₁-C₄)Alkoxylcarbonylamino, [(C₂-C₄)Alkenyloxy]carbonylamino, [(C₂-C₄)Alkinyloxy]carbonylamino, Phenylcarbonylamino, Phenoxycarbonylamino, (C₁-C₄)Alkylsulfonyl, (C₂-C₄)Alkenylsulfonyl oder einen der letztgenannten 27 Reste, der im acyclischen Teil oder im cyclischen Teil durch einen oder mehrere Reste aus der Gruppe Halogen, Nitro, Cyano, (C₁-C₄)Alkoxy, (C₁-C₄)Alkylthio, (C₁-C₄)Haloalkoxy. Formyl, (C₁-C₄)Alkyl-carbonyl, (C₁-C₄)Alkoxy-carbonyl, (C₁-C₄)Alkoxy und im Falle cyclischer Teile auch (C₁-C₄)Alkyl und (C₁-C₄)Haloalkyl substituiert ist,
R⁵ wenn n = 1 ist, und die Reste R⁵, jeweils unabhängig voneinander, wenn n größer als 1 ist, Halogen, Hydroxy, Amino, Nitro, Formyl, Carboxy, Cyano. Thiocyanato, Aminocarbonyl, (C₁-C₄)Alkyl, Cyano-(C₁-C₄)alkyl, (C₁-C₄)Alkoxy, (C₁-C₄)Alkylamino. Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₇-C₆)Alkenyl, Halo-(C₂-C₆)Alkenyl, (C₂-C₆)Alkinyl, Halo-(C₂-C₆)alkinyl, (C₁-C₄)Alkylamino-(C₁-C₄)alkyl. Di-[(C₁-C₄)alkyl-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 6 Ringgttedem, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder
Phenyl, Phenoxy, Phenylcarbonyl, Phenylcarbonyloxy, Phenylcarbonyl-(C₁-C₄)alkyl, Phenoxycarbonyl, (C₁-C₄)Alkyl-carbonyl, (C₁-C₄)Alkoxy-carbonyl, (C₁-C₄)Alkylamino-carbonyl, (C₁-C₄)Alkoxy carbonyl-(C₁-C₄)alkyl, (C₁-C₄)Alkylamino-carbonyl-(C₁-C₄)alkyl, Phenoxy-(C₁-C₄)alkyl, Phenyl-(C₁-C₄)alkyl, Heterocyclyl, Heterocyclylamino, Heterocyclyloxy, Heterocyclylthio, (C₁-C₅)Alkanoylamino, N-[(C₁-C₅)Alkanoyl]-N-[(C₁-C₄)alkyl]-amino, [(C₂-C₄)Alkenyl]carbonylamino, [(C₂-C₄)Alkinyl]carbonylamino, [(C₁-C₄)Alkoxy)carbonylamino, [(C₂-C₄)Alkenyloxy]carbonylamino, [(C₂₋C₄)Alkinyloxy]carbonylamino, Phenylcarbonylamino. Phenoxycarbonylamino, (C₁-C₄)Alkylsulfonyl, (C₂-C₄)Alkenylsulfonyl oder einen der letztgenannten 28 Reste, der im acyclischen Teil oder im cyclischen Teil durch einen oder mehrere Reste aus der Gruppe Halogen, Nitro, Cyano, (C₁-C₄)Alkoxy, (C₁-C₄)Alkylthio, (C₁-C₄)Haloalkoxy, Formyl, (C₁-C₄)Alkyl-carbonyl, (C₁-C₄)Alkoxy-carbonyl, (C₁-C₄)Alkoxy und im Falle cyclischer Teile auch (C₁-C₄)Alkyl und (C₁-C₄)Haloalkyl substituiert ist, wobei Heterocyclyl in den Resten jeweils 3 bis 9 Ringatome und 1 bis 3 Heteroringatome aus der Gruppe N, O und S enthält, oder zwei benachbarte Reste R⁵ gemeinsam einen ankondensierten Cyclus mit 4 bis 6 Ringatomen, der carbocyclisch ist oder Heteroringatome aus der Gruppe O, S und N enthält und der unsubstituiert oder durch einen oder mehrere Reste aus der Gruppe Halogen, (C₁-C₄)Alkyl und Oxo substituiert ist,
R⁸ und R⁹ jeweils unabhängig voneinander Wasserstoff, Formyl, Carboxy. Cyano, Thiocyanato, Aminocarbonyl, (C₁-C₄)Alkyl. Cyano-(C₁-C₄)alkyl, (C₁₋C₄)Alkoxy. (C₁-C₄)Alkylamino, Di-[(C₁-C₄)alkyl]-amino, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₁-C₄)Alkylthio, Halo-(C₁-C₄)alkylthio, (C₂-C₆)Alkonyl, Halo-(C₂-C₆)Alkenyl, (C₂-C₆)Alkinyl, Halo-(C₂-C₆)alkinyl, (C₁-C₄)Alkylamino-(C₁-C₄)alkyl, Di-[(C₁-C₄)alkyl)-amino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 6 Ringgliedem, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder
Phenol, Phenoxy, Phenylcarbonyl, Phenylcarbonyl-(C₁-C₄)alkyl, Phenoxycarbonyl, (C₁-C₄)Alkyl-carbonyl, (C₁-C₄)Alkoxy-carbonyl, (C₁-C₄)Alkylamino-carbonyl, (C₁-C₄)Alkoxy-carbonyl-(C₁-C₄)alkyl, (C₁-C₄)Alkylamino-carbonyl-(C₁-C₄)alkyl, Phenoxy-(C₁-C₄)alkyl, Phenyl-(C₁-C₄)alkyl, Heterocyclyl, Heterocyclylamino, Heterocyclyloxy, Heterocyclythio, (C₁-C₅)Alkanoylamino, N-[(C₁-C₅)Alkanoyl]-N-[(C₁-C₄)alkyl]-amino, [(C₂-C₄)Alkenyl]carbonylamino, [(C₂₋C₄)Alkinyl]carbonylamino, [(C₁-C₄)Alkoxy]carbonylamino, (C₂-C₄)Alkenyloxy]carbonylamino. [(C₂-C₄)Alkinyloxy]carbonylamino, Phenylcarbonylamino, Phenoxycarbonylamino, (C₁-C₄)Alkylsulfonyl, (C₂-C₄)Alkenylsulfonyl
oder einen der letztgenannten 27 Reste, der im acyclischen Teil oder im cyclischen Teil durch einen oder mehrere Reste aus der Gruppe Halogen, Nitro, Cyano, (C₁-C₄)Alkoxy, (C₁-C₄)Alkylthio, (C₁-C₄)Haloalkoxy, Formyl, (C₁-C₄)Alkyl-carbonyl, (C₁-C₄)Alkoxy carbonyl, (C₁-C₄)Alkoxy und im Falle cyclischer Teile auch (C₁-C₄)Alkyl und (C₁-C₄)Haloalkyl substituiert ist,
X¹, X², X³, X⁴ jeweils unabhängig voneinander ein Kohlenstoffatom, das mit einem Wasserstoffatom oder einer der definierten Substituenten R⁵ substituiert ist, oder ein Stickstoffatom oder zwei benachbarte Symbole X¹, X², X³ und X⁴ jeweils gemeinsam eine divalente Gruppe der Formel -O-. -S-, -NH- oder -NR-, worin R wie für R³ definiert ist, vorausgesetzt die Gruppen X¹, X², X³, X⁴ bilden zusammen mit der verknüpften C₂-Einheit des ankondensierten Rings einen carbocyclischen oder heterocyclischen aromatischen fünfgliedrigen oder sechsgliedrigen Ring,
(Y¹)ₘ m divalente Gruppen Y¹, wobei jede Gruppe Y¹ unabhängig von anderen Resten Y¹ eine Gruppe der Formel -O-, -CO-, -C(=NR*)-, -S(O)q-, -NR*- oder -N(O)-, wobei q = 0, 1 oder 2 ist und R- wie für R³ definiert ist, oder eine Gruppe der Formel CR⁸R⁹ bedeutet,
Y² eine Gruppe wie für Y¹ definiert oder eine direkte Bindung,
wobei zwei benachbarte Gruppen der Symbolpaare Y¹ und Y¹ oder der Symbolpaare Y¹ und Y² nicht heteroatomige Gruppen derselben Bedeutung darstellen und wobei die Gruppen (Y¹)ₘ und Y² zusammen mit der verknüpften C₂-Einheit des aromatischen Rings und dem mit R⁴ verknüpften C-Atom einen ankondensierten carbocyclischen oder heterocyclischen nicht aromatischen viergiledrigen bis achtgliedrigen Ring bilden,
bedeuten, wobei Heterocyclyl jeweils, wenn nicht näher definiert, auch in zusammengesetzten Resten jeweils 3 bis 9 Ringatome und 1 bis 3 Heteroringatome aus der Gruppe N, O und S aufweist

4. Verbindungen oder deren Salze gemäß einem der Ansprüche 1 bis 3, **dadurch gekennzeichnet, dass**
R¹, R² unabhängig voneinander Wasserstoff. Amino, Formyl, (C₁-C₄)Alkyl, (C₁-C₄)Alkylamino, Di-[(C₁-C₄)alkyl]-amino, Hydroxy (C₁-C₄)alkyl, (C₁-C₄)Alkoxy-(C₁-C₄)alkyl. Halo(C₁-C₄)alkoxy-(C₁-C₄)alkyl, (C₂-C₆)Alkenyl, (C₂-C₆)Alkinyl, Halo-(C₂-C₆)alkinyl, (C₁-C₄)Alkylamino-(C₁-C₄)alkyl oder Phenyl, Phenoxy, Phenylcarbonyl, Phenylcarbonyl-(C₁-C₄)alkyl, (C₁-C₄)Alkoxy-carbonyl-(C₁-C₄)alkyl, (C₁-C₄)Alkylamino-carbonyl-(C₁-C₄)alkyl, (C₁-C₄)Alky-carbonyl, (C₁-C₄)Alkoxy-carbonyl, Aminocarbonyl, (C₁-C₄)Alkylamino-carbonyl oder einen der letztgenannten 10 Reste, der im acyclischen Teil oder im cyclischen Teil durch einen oder mehrere Reste aus der Gruppe Halogen, Nitro, Cyano, (C₁-C₄)Alkyl, (C₁-C₄)Alkoxy, (C₁-C₄)Alkylthio. (C₁-C₄)Haloalkyl, Formyl, (C₁-C₄)Alkyl-carbonyl, (C₁-C₄)Alkoxy-carbonyl, (C₁-C₄)Alkoxy, substituiert ist,
R³ Wasserstoff, Amino, Formyl, (C₁-C₄)Alkyl, Cyano-(C₁-C₄)alkyl, (C₁-C₄)Alkoxy-(C₁-C₄)alkyl, (C₂-C₆)Alkenyl, Hato-(C₂-C₆)alkenyl, (C₂-C₆)Alkinyl oder Phenyl, (C₁-C₄)Alkylcarbonyl, (C₁-C₄)Alkoxycarbonyl, Aminocarbonyl, Phenoxy-(C₁-C₄)alkyl, Phenyl-(C₁-C₄)alkyl oder einen der letztgenannten 6 Reste, der im acyclischen Teil oder im acyclischen Teil durch einen oder mehrere Reste aus der Gruppe Halogen, (C₁-C₄)Alkyl und (C₁-C₄)Alkoxy substituiert ist;
R⁴ Wasserstoff. (C₁-C₄)Alkyl, Cyano-(C₁-C₄)alkyl, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₈)Alkenyl, (C₂-C₆)Alkinyl, (C₁-C₄)Alkylamino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis, 9 Ringgliedem, wobei die acyclischen Gruppen in den letztgenannten 3 Resten unsubstituiert oder durch einen oder mehrere Reste aus der Gruppe (C₁-C₄)Alkyl, Halogen und Cyano substituiert sind, oder Phenyl, Phenoxy-(C₁-C₄)alkyl, Phenyl-(C₁-C₄)alkyl, Heterocyclyl, oder einen der letztgenannten 4 Reste, der im acyclischen Teil oder im cyclischen Teil durch einen oder mehrere Reste aus der Gruppe Halogen, Nitro, Cyano, (C₁-C₄)Alkoxy, (C₁-C₄)Alkylthio, (C₁-C₄)Haloalkoxy, (C₁-C₄)Alkyl-carbonyl, (C₁-C₄)Alkoxy-carbonyl, (C₁-C₄)Alkoxy und im Falle cyclischer Teile auch (C₁-C₄)Alkyl und (C₁-C₄)Haloalkyl substituiert ist.
R⁵ wenn n = 1 ist, und die Reste R⁵, jeweils unabhängig voneinander, wenn n größer als 1 ist, Halogen. Hydroxy, Amino, Nitro, Formyl, Cyano, Aminocarbonyl, (C₁-C₄)Monoalkylaminocarbonyl, (C₁-C₄)Dialkylaminocarbonyl, (C₁-C₄)Alkyl, Cyano-(C₁-C₄)alkyl, (C₁-C₄)Alkoxy, (C₁-C₄)Alkylamino, Halo-(C₁-C₄)alkyl, Hydroxy-(C₁-C₄)alkyl, (C₁-C₄)Alkoxy-(C₁-C₄)alkyl, (C₂-C₄)Alkenyl, (C₂-C₄)Alkinyl, (C₁-C₄)Alkylemino-(C₁-C₄)alkyl, Di-[(C₁-C₄)alkyl]-amino-(C₁-C₄)alkyl, oder [(C₁-C₄)Alkyl]carbonyl, [(C₁-C₄)Haloalkyl]carbonyl, [(C₁-C₄)Alkoxy-(C₁-C₄)alkyl]carbonyl, Formylamino, [(C₁-C₄)Alkyl]carbonylamino, [(C₁-C₄)Haloalkyl]carbonylamino, Phenyl, Phenoxy, Phenylcarbonyl, Phenylcarbonylamino, Heterocyclyl oder einen der tetztgenannten 5 Reste, der durch einen oder mehrere Reste aus der Gruppe Halogen, (C₁-C₄)Alkyl, (C₁-C₄)Haloalkyl, (C₁-C₄)Alkoxy, (C₁-C₄)Haloalkoxy und (C₁-C₄)Alkylthio substituiert ist,
R⁸ und R⁹ jeweils unabhängig voneinander Wasserstoff, (C₁-C₄)Alkyl, Cyano-(C₁-C₄)alkyl, Halo-(C₁-C₄)alkyl, Hydroxy-(C₁-C₄)alkyl, (C₁-C₄)Alkoxy-(C₁-C₄)alkyl, Halo(C₁-C₄)alkoxy-(C₁-C₄)alkyl, (C₂-C₆)Alkenyl, Halo-(C₂-C₉)Alkenyl, (C₂-C₆)Alkinyl, (C₁-C₆)Alkylamino-(C₁-C₄)alkyl, (C₃-C₉)Cycloalkylamino-(C₁-C₄)alkyl, (C₃-C₉)Cycloalkyl, Heterocyclyl-(C₁-C₄)alkyl mit 3 bis 9 Ringgliedern, wobei die cyclischen Gruppen in den letztgenannten 3 Resten unsubstituiert oder durch einen oder mehrere Reste aus der Gruppe (C₁-C₄)Alkyl, Halogen und Cyano substituiert sind, oder Phenyl, Phenoxy-(C₁-C₄)alkyl, Phenyl-(C₁-C₄)alkyl, Heterocyclyl, oder einen der letztgenannten 4 Reste, der im acyclischen Teil oder im cyclischen Teil durch einen oder mehrere Reste aus der Gruppe Halogen, Nitro. Cyano, (C₁-C₄)Alkoxy, (C₁-C₄)Alkylthio, (C₁-C₄)Haloalkoxy, (C₁-C₄)Alkyl-carbonyl, (C₁-C₄)Alkoxy-carbonyl, (C₁-C₄)Alkoxy und im Falle cyclischer Teile auch (C₁-C₄)Alkyl und (C₁-C₄)Haloalkyl substituiert ist,
bedeuten.

5. Verbindungen oder deren Salze gemäß einem der Ansprüche 1 bis 4, **dadurch gekennzeichnet, dass**
X¹, X², X³, X⁴ jeweils unabhängig voneinander ein Kohlenstoffatom, das mit einem Wasserstoffatom oder einer der definierten Substituenten R⁵ substituiert ist, oder ein Stickstoffatom oder zwei benachbarte Symbole X¹, X². X³ und X⁴ jeweils gemeinsam eine divalente Gruppe der Formel -O-, -S-, -NH- oder-NR-, worin R Wasserstoff oder (C₁-C₄)Alkyl bedeutet, vorausgesetzt die Gruppen X¹, X², X³, X⁴ bilden zusammen mit der verknüpften C₂-Einheit des ankondensierten Rings einen carbocyclischen oder heterocyclischen aromatischen fünfgliedrigen oder sechsgliedrigen Ring,
(Y¹)ₘ m divalente Gruppen Y¹, wobei jede Gruppe Y¹ unabhängig von anderen Resten Y¹ eine Gruppe der Formel CH₂, CH(CH₃), CH(C₂H₅), CH(CH₃)₂ oder CH(C₆H₅) und m = 0, 1, 2 oder 3 und
Y² eine direkte Bindung oder eine Gruppe der Formel -O-, -S-, CH₂, CH(CH₃) oder (C₁-C₄)Alkylamino,
wobei zwei benachbarte Gruppen der Symbolpaare Y¹ und Y¹ oder der Symbolpaare Y¹ und Y² nicht heteroatomige Gruppen derselben Bedeutung darstellen und wobei die Gruppen (Y¹)ₘ und Y² zusammen mit der verknüpften C₂-Einheit des aromatischen Rings und dem mit R⁴ verknüpften C-Atom einen ankondensierten carbocyclischen oder heterocyclischen nicht aromatischen viergliedrigen bis achtgliedrigen Ring bilden,
bedeuten.

6. Verfahren zur Herstellung von Verbindungen der Formel (1) und deren Salzen, wie sie nach einem der Ansprüche 1 bis 5 definiert sind, **dadurch** gekennzeichet dass man
a) eine Verbindung der Formel **(II)**,
H-R¹³ (II)
worin R¹³ eine funktionelle Gruppe aus der Gruppe der Carbonsäureester, Carbonsäureorthoester, Carbonsäurechloride, Carbonsäureamide und Carbonsäureanhydride bedeutet,
mit einem Biguanid der Formel (III) oder einem Säureadditionssalz hiervon 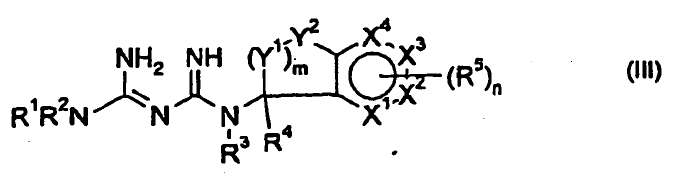 umsetzt oder
b) eine Verbindung der Formel (IV), 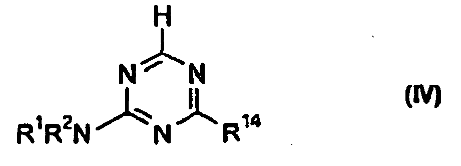 worin R¹⁴ einen austauschfähigen Rest oder eine Abgangsgruppe bedeutet, mit einem Amin der Formel (V) oder einem Säureadditionssalz hiervon 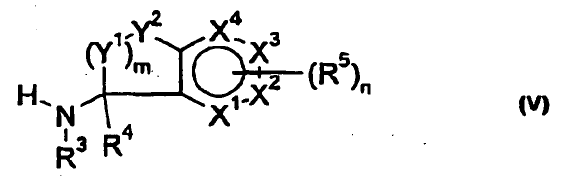 umsetzt oder
c) ein Diamino-1,3,5-triazin der Formel (VI) mit einem isocyanat der Formel (VII) 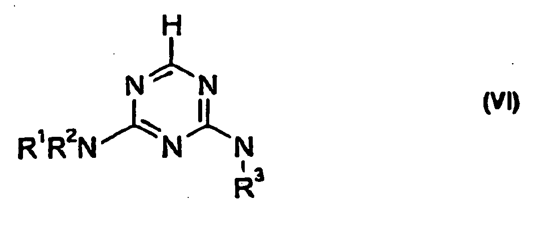 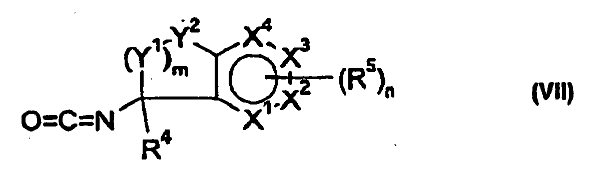 umsetzt oder
d) bei einem Triazin der Struktur (VIII) den Rest R¹⁵, der eine Abgangsgruppe oder ein abspaltbarer Rest ist, abspaltet, 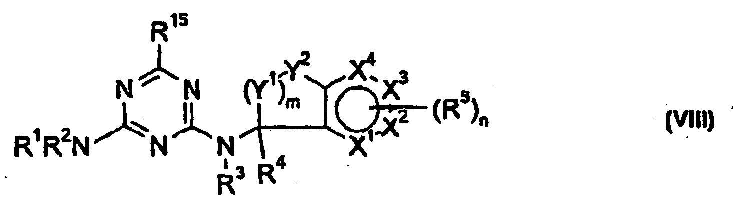
wobei in den Formeln (II), (III), (IV), (V), (VI), (VII) und (VIII) die Reste R¹, R², R³, R⁴. R⁵, X¹, X², X³, X⁴, Y¹ und Y² und die Symbole m und n wie in der Formel (I) definiert sind.

7. Herbizides oder pflanzenwachstumsregulierendes Mittel, **dadurch gekennzeichnet, dass** es eine oder mehrere Verbindungen der Formel (I) oder deren Salze nach einem der Ansprüche 1 bis 5 und im Pflanzenschutz verwendbare Formulierungshitfsmittel enthält

8. Verfahren zur Bekämpfung von Schadpflanzen oder zur Wächstumsregutierung von Pflanzen, **dadurch gekennzeichnet, dass** man eine wirksame Menge von einer oder mehreren Verbindungen der Formel (I) oder deren Salzen nach einem der Ansprüche 1 bis 5 auf die Pflanzen, Pftanzensamen oder die Anbaufläche appliziert.

9. Verwendung von Verbindungen der Formel (I) oder deren Salzen nach einem der Ansprüche 1 bis 5 als Herbizide oder Pflanzenwachstumsregulatoren.

10. Verwendung nach Anspruch 9, **dadurch gekennzeichnet, dass** die Verbindungen der Formel (I) oder deren Salze zur Bekämpfung von Schadpflanzen oder zur Wachstumsregulierung in Kulturen von Nutz- oder Zierpfianzen eingesetzt werden.

## Claims

1. A compound of the formula (I) or a salt thereof 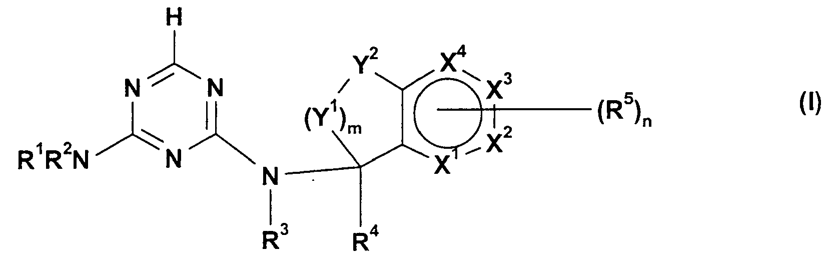 in which
R¹ and R² are each independently of one another hydrogen, amino, alkylamino or dialkylamino having in each case 1 to 4 carbon atoms in the alkyl radical or (C₃-C₆)cycloalkylamino, (C₅-C₆)cycloalkenylamino, an (acyclic or cyclic) hydrocarbon radical or hydrocarbonoxy radical having in each case 1 to 10 carbon atoms, preferably having 1 to 6 carbon atoms, or a heterocyclyl radical, heterocyclyloxy radical, heterocyclylthio radical or heterocyclylamino radical having in each case 3 to 9 ring atoms, preferably 3 to 6 ring atoms, and 1 to 3 hetero ring atoms from the group consisting of N, O and S, where each of the six last-mentioned radicals is unsubstituted or substituted by one or more radicals from the group consisting of halogen, hydroxyl, amino, nitro, formyl, aminocarbonyl, carboxyl, sulfonyl, cyano, thiocyanato, (C₁-C₆)alkoxy, (C₂-C₆)alkenyloxy, (C₂-C₆)alkynyloxy, (C₁-C₆)alkoxy-poly(alkyleneoxy), hydroxy-poly(alkyleneoxy), (C₁-C₆)alkylthio, mono- and di[(C₁-C₆)alkyl]amino, [(C₁-C₆)alkyl]carbonyl, [(C₂-C₆)alkenyl]carbonyl, [(C₂-C₆)alkynyl]carbonyl, [(C₁-C₆)alkoxy]carbonyl, [(C₂-C₆)alkenyloxy]carbonyl, [(C₂-C₆)alkynyloxy]carbonyl, mono- and di[(C₁-C₆)alkyl]aminocarbonyl, phenyl, phenoxy, (C₃-C₆)cycloalkyl, phenylcarbonyl, phenoxycarbonyl, heterocyclyl, (C₁-C₄)alkylsulfonyl and, in the case of cyclic radicals, also (C₁-C₄)alkyl,
where each of the 24 last-mentioned substituents is unsubstituted or substituted by one or more radicals from the group consisting of halogen, CN, SCN, NO₂, (C₁-C₄)alkoxy, (C₁-C₄)alkylthio, (C₁-C₄)haloalkoxy, formyl, (C₁-C₄)alkylcarbonyl, [(C₁-C₄)haloalkyl]carbonyl, [(C₁-C₄)alkoxy]carbonyl, [(C₁-C₄)haloalkoxy]carbonyl and, in the case of cyclic radicals, also (C₁-C₄)alkyl and (C₁-C₄)haloalkyl,
or an acyl radical of an organic acid from the group consisting of formyl, aminocarbonyl, mono- and di[(C₁-C₄)alkyl]aminocarbonyl, [(C₁-C₆)alkyl]carbonyl, [(C₂-C₆)alkenyl]carbonyl, [(C₂-C₆)alkynyl]carbonyl, [(C₁-C₆)alkoxy]carbonyl, [(C₂-C₆)alkenyloxy]carbonyl, phenylcarbonyl, (C₁-C₆)alkylsulfonyl, where each of the 9 last-mentioned radicals is unsubstituted in the aliphatic moiety or in the phenyl ring or substituted by one or more radicals from the group consisting of halogen, CN, SCN, NO₂, (C₁-C₄)alkoxy, (C₁-C₄)haloalkoxy, optionally substituted and preferably unsubstituted phenyl and, in the case of cyclic radicals, also (C₁-C₆)alkyl and (C₁-C₆)haloalkyl,
where heterocyclyl, unless defined in more detail, and including in composite radicals, has in each case 3 to 9 ring atoms, preferably 3 to 6 ring atoms, and 1 to 3 hetero ring atoms from the group consisting of N, O and S,
or
R¹ and R² together with the nitrogen atom of the group NR¹R² are a saturated or unsaturated, nonaromatic heterocyclic radical having 3 to 6 ring atoms and 1 to 4 hetero ring atoms, where the further hetero ring atoms optionally present in addition to the nitrogen atom are selected from the group consisting of N, O and S and the radical is unsubstituted or substituted by one or more radicals from the group consisting of halogen, CN, (C₁-C₄)alkyl, (C₁-C₄)haloalkyl, (C₁-C₄)alkoxy, (C₁-C₄)haloalkoxy, (C₁-C₄)alkoxy-carbonyl and oxo,
where each of the carbon-containing radicals R¹ and R² including substituents has 1 to 20 carbon atoms,
R³ is hydrogen, amino, (C₁-C₆)alkyl, (C₁-C₆)alkoxy, (C₂-C₆)alkenyl, (C₂-C₆)alkynyl, (C₃-C₆)cycloalkyl, (C₅-C₆)cycloalkenyl, phenyl or heterocyclyl,
where each of the eight last-mentioned radicals is unsubstituted or substituted by one or more radicals from the group consisting of halogen, hydroxyl, amino, nitro, formyl, carboxyl, cyano, thiocyanato, (C₁-C₄)alkoxy, (C₁-C₄)alkoxy-(C₁-C₄)alkoxy, hydroxy-(C₁-C₄)alkoxy, (C₁-C₄)alkoxy-poly(alkyleneoxy), hydroxy-poly(alkyleneoxy), (C₁-C₄)haloalkoxy, (C₁-C₄)alkylthio, (C₁-C₄)haloalkylthio, mono(C₁-C₄)alkylamino, di(C₁-C₄)alkylamino, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl, phenyl, phenoxy, (C₃-C₆)cycloalkyl, phenylcarbonyl, phenoxycarbonyl, heterocyclyl, where each of the 6 last-mentioned radicals is unsubstituted or substituted by one or more radicals from the group consisting of halogen, CN, SCN, NO₂, (C₁-C₄)alkyl, (C₁-C₄)haloalkyl, (C₁-C₄)alkoxy and (C₁-C₄)haloalkoxy, and (C₁-C₄)alkylsulfonyl and (C₁-C₄)haloalkylsulfonyl and, in the case of cyclic radicals, also (C₁-C₄)alkyl and (C₁-C₄)haloalkyl,
or an acyl radical of the formula -B*-A*, where
A* is hydrogen or (C₁-C₆)alkyl, (C₂-C₆)alkenyl, (C₂-C₆)alkynyl, (C₃-C₆)cycloalkyl, (C₅-C₆)cycloalkenyl or phenyl, where each of the six last-mentioned radicals is unsubstituted or substituted by one or more radicals from the group consisting of halogen, hydroxyl, amino, nitro, formyl, carboxyl, cyano, thiocyanato, (C₁-C₄)alkoxy, (C₁-C₄)haloalkoxy, (C₁-C₄)alkylthio, mono(C₁-C₄)alkylamino, di(C₁-C₄)alkylamino, phenyl and (C₃-C₆)cycloalkyl, where each of the 2 last-mentioned radicals is unsubstituted or substituted by one or more radicals from the group consisting of halogen, CN, SCN, NO₂, (C₁-C₄)alkyl, (C₁-C₄)haloalkyl, (C₁-C₄)alkoxy and (C₁-C₄)haloalkoxy, and, in the case of cyclic radicals, also (C₁-C₄)alkyl and (C₁-C₄)haloalkyl,
and
B* is a divalent group of the formula -CO-, -CO-O-, -CO-NR'-, -S(O)ₚ- or -S(O)ₚ-O-, where p = 0, 1 or 2 and R' is hydrogen, alkyl having 1 to 6 carbon atoms, phenyl, benzyl, cycloalkyl having 3 to 6 carbon atoms or alkanoyl having 1 to 4 carbon atoms,
where -B*-A* including substituents has 1 to 12 carbon atoms,
R⁴ is a radical of the formula -Z¹-R⁶, where Z¹ and R⁶ are as defined below,
R⁵ are each independently of one another halogen, cyano, isocyanato, nitro, a radical of the formula -Z²-R⁷, where Z² and R⁷ are as defined below,
or
two adjacent radicals R⁵ together are a fused-on cycle having 4 to 6 ring atoms which is carbocyclic or contains 1 to 3 hetero ring atoms from the group consisting of O, S and N and which is unsubstituted or substituted by one or more radicals from the group consisting of halogen, (C₁-C₄)alkyl and oxo,
R⁶ and R⁷, R⁸, R⁹ are each independently of one another hydrogen, except that R⁷ is not hydrogen if Z² is a direct bond, or an acyclic hydrocarbon radical having 1 to 10 carbon atoms or a cyclic hydrocarbon radical having 3 to 6 carbon atoms or a heterocyclic radical, where each of the last-mentioned carbon-containing radicals is unsubstituted or substituted by one or more radicals from the group consisting of halogen, hydroxyl, amino, nitro, formyl, aminocarbonyl, carboxyl, sulfonyl, cyano, thiocyanato, (C₁-C₆)alkoxy, (C₂-C₆)alkenyloxy, (C₂-C₆)alkynyloxy, (C₁-C₆)alkoxy-poly(alkyleneoxy), hydroxy-poly(alkyleneoxy), (C₁-C₆)alkylthio, mono- and di[(C₁-C₆)alkyl]amino, [(C₁-C₆)alkyl]carbonyl, [(C₂-C₆)alkenyl]carbonyl, [(C₂-C₆)alkynyl]carbonyl, [(C₁-C₆)alkoxy]carbonyl, [(C₂-C₆)alkenyloxy]carbonyl, [(C₂-C₆)alkynyloxy]carbonyl, mono- and di[(C₁-C₆)alkyl]aminocarbonyl, phenyl, phenoxy, (C₃-C₆)cycloalkyl, phenylcarbonyl, phenoxycarbonyl, heterocyclyl, (C₁-C₄)alkylsulfonyl and, in the case of cyclic radicals, also (C₁-C₄)alkyl,
where each of the 24 last-mentioned substituents is unsubstituted or substituted by one or more radicals from the group consisting of halogen, CN, SCN, NO₂, (C₁-C₄)alkoxy, (C₁-C₄)alkylthio, (C₁-C₄)haloalkoxy, formyl, (C₁-C₄)alkylcarbonyl, [(C₁-C₄)haloalkyl]carbonyl, [(C₁-C₄)alkoxy]carbonyl, [(C₁-C₄)haloalkoxy]carbonyl and, in the case of cyclic radicals, also (C₁-C₄)alkyl and (C₁-C₄)haloalkyl,
or R⁸ and R⁹ together with the carbon atom of a group CR⁸R⁹ (for Y¹ or Y²) or two radicals R⁸ or R⁹ from two groups Y¹ and/or Y² together with the attached atoms of the groups Y¹ and/or Y² are in each case a carbocyclic radical having 3 to 8 carbon atoms or a heterocyclic radical, where each of the two last-mentioned radicals is unsubstituted or substituted by one or more radicals from the group consisting of halogen, (C₁-C₄)alkyl, (C₁-C₄)haloalkyl, (C₁-C₄)alkoxy, (C₁-C₄)haloalkoxy, nitro and cycano,
X¹, X², X³, X⁴ are each independently of one another a carbon atom which is substituted by a hydrogen atom or one of the substituents R⁵ defined above, or a nitrogen atom, or two adjacent symbols X¹, X², X³ and X⁴ are in each case together a divalent group of the formula -O-, -S-, -NH- or -NR-, where R is as defined for R³, provided the groups X¹, X², X³, X⁴ together with the attached C₂ unit of the fused-on ring form a carbocyclic or heterocyclic aromatic five- or six-membered ring,
(Y¹)ₘ are m divalent groups Y¹, where each group Y¹ independently of the other radicals Y¹ is a group of the formula -O-, -CO-, -C(=NR*)-, -S(O)_{q}-, -NR*- or -N(O)-, where q = 0, 1 or 2 and R* is as defined for R³ or a group of the formula CR⁸R⁹, where R⁸ and R⁹ are as defined above,and
Y² is a group as defined for Y¹ or a direct bond,
where two adjacent groups of the symbol pairs Y¹ and Y¹ or of the symbol pairs Y¹ and Y² are groups with no heteroatoms having the same meaning, and where the groups (Y¹)ₘ and Y² together with the attached C₂ unit of the aromatic ring and the carbon atom attached to R⁴ form a fused-on carbocyclic or heterocyclic nonaromatic four- to eight-membered ring,
Z¹ and the groups Z² are each independently of one another a direct bond or a divalent group of the formula -O-, -S(O)ₚ-, -S(O)ₚ-O-, -O-S(O)ₚ-, -CO-, -O-CO-, -CO-O-, -NR'-, -O-NR'-, -NR'-O-, -NR'-CO-, -CO-NR'-, where p = 0, 1 or 2 and R' is hydrogen, alkyl having 1 to 6 carbon atoms, phenyl, benzyl, (C₃-C₆)cycloalkyl or alkanoyl having 1 to 6 carbon atoms,
m is 0, 1, 2, 3 or 4 and
n is 0, 1, 2, 3 or 4.

2. A compound or a salt thereof as claimed in claim 1, wherein
R¹ and R² are each independently of one another hydrogen, amino, alkylamino or dialkylamino having in each case 1 to 4 carbon atoms in the alkyl radical or (C₃-C₆)cycloalkylamino, (C₅-C₆)cycloalkenylamino, a hydrocarbon radical or hydrocarbonoxy radical having in each case 1 to 10 carbon atoms or a heterocyclyl radical, heterocyclyloxy radical, heterocyclylthio radical or heterocyclylamino radical having in each case 3 to 9 ring atoms and 1 to 3 hetero ring atoms from the group consisting of N, O and S, where each of the six last-mentioned radicals is unsubstituted or substituted by one or more radicals from the group consisting of halogen, hydroxyl, amino, nitro, formyl, aminocarbonyl, carboxyl, sulfonyl, cyano, thiocyanato, (C₁-C₆)alkoxy, (C₂-C₆)alkenyloxy, (C₂-C₆)alkynyloxy, (C₁-C₆)alkoxy-poly(alkyleneoxy), hydroxy-poly(alkyleneoxy), (C₁-C₆)alkylthio, mono- and di[(C₁-C₆)alkyl]amino, ((C₁-C₆)alkyl]carbonyl, [(C₂-C₆)alkenyl]carbonyl, [(C₂-C₆)alkynyl]carbonyl, [(C₁-C₆)alkoxy]carbonyl, [(C₂-C₆)alkenyloxy]carbonyl, [(C₂-C₆)alkynyloxy]carbonyl, mono- and di[(C₁-C₆)alkyl]aminocarbonyl, phenyl, phenoxy, (C₃-C₆)cycloalkyl, phenylcarbonyl, phenoxycarbonyl, heterocyclyl, (C₁-C₄)alkylsulfonyl and, in the case of cyclic radicals, also (C₁-C₄)alkyl,
where each of the 24 last-mentioned substituents is unsubstituted or substituted by one or more radicals from the group consisting of halogen, CN, SCN, NO₂, (C₁-C₄)alkoxy, (C₁-C₄)alkylthio, (C₁-C₄)haloalkoxy, formyl, (C₁-C₄)alkylcarbonyl, [(C₁-C₄)haloalkyl]carbonyl, [(C₁-C₄)alkoxy]carbonyl, [(C₁-C₄)haloalkoxy]carbonyl and, in the case of cyclic radicals, also (C₁-C₄)alkyl and (C₁-C₄)haloalkyl,
or an acyl radical of an organic acid from the group consisting of formyl, aminocarbonyl, mono- and dif(C₁-C₄)alkyl]aminocarbonyl, [(C₁-C₆)alkyl]carbonyl, [(C₂-C₆)alkenyl]carbonyl, [(C₂-C₆)alkynyl]carbonyl, [(C₁-C₆)alkoxy]carbonyl, [(C₂-C₆)alkenyloxy]carbonyl, phenylcarbonyl, (C₁-C₆)alkylsulfonyl, where each of the 9 last-mentioned radicals is unsubstituted in the aliphatic moiety or in the phenyl ring or substituted by one or more radicals from the group consisting of halogen, CN, SCN, NO₂, (C₁-C₄)alkoxy, (C₁-C₄)haloalkoxy, optionally substituted and unsubstituted phenyl and, in the case of cyclic radicals, also (C₁-C₆)alkyl and (C₁-C₆)haloalkyl, or
R¹ and R² together with the nitrogen atom of the group NR¹R² are a saturated or unsaturated, nonaromatic heterocyclic radical having 3 to 6 ring atoms and 1 to 4 hetero ring atoms, where the further hetero ring atoms optionally present in addition to the nitrogen atom are selected from the group consisting of N, O and S and the radical is unsubstituted or substituted by one or more radicals from the group consisting of halogen, CN, (C₁-C₄)alky), (C₁-C₄)haloalkyl, (C₁-C₄)alkoxy, (C₁-C₄)haloalkoxy, (C₁-C₄)alkoxycarbonyl and oxo,
R³ is hydrogen, amino, (C₁-C₆)alkyl, (C₁-C₆)alkoxy, (C₂-C₆)alkenyl, (C₂-C₆)alkynyl, (C₃-C₆)cycloalkyl, (C₅-C₆)cycloalkenyl, phenyl or heterocyclyl, where each of the eight last-mentioned radicals is unsubstituted or substituted by one or more radicals from the group consisting of halogen, hydroxyl, amino, nitro, formyl, carboxyl, cyano, thiocyanato, (C₁-C₄)alkoxy, (C₁-C₄)alkoxy-(C₁-C₄)alkoxy, hydroxy-(C₁-C₄)alkoxy, (C₁-C₄)alkoxy-poly(alkyleneoxy), hydroxy-poly(alkyleneoxy), (C₁-C₄)haloalkoxy, (C₁-C₄)alkylthio, (C₁-C₄)haloalkylthio, mono(C₁-C₄)alkylamino, di(C₁-C₄)alkylamino, [(C₁-C₄)alkyl]carbonyl, [(C₁-C₄)alkoxy]carbonyl, aminocarbonyl, mono(C₁-C₄)alkylaminocarbonyl, di(C₁-C₄)alkylaminocarbonyl, phenyl, phenoxy, (C₃-C₆)cycloalkyl, phenylcarbonyl, phenoxycarbonyl, heterocyclyl, where each of the 6 last-mentioned radicals is unsubstituted or substituted by one or more radicals from the group consisting of halogen, CN, SCN, NO₂, (C₁-C₄)alkyl, (C₁-C₄)haloalkyl, (C₁-C₄)alkoxy and (C₁-C₄)haloalkoxy, and (C₁-C₄)alkylsulfonyl and (C₁-C₄)haloalkylsulfonyl and, in the case of cyclic radicals, also (C₁-C₄)alkyl and (C₁-C₄)haloalkyl,
or an acyl radical of the formula -B*-A*, where
A* is hydrogen or (C₁-C₆)alkyl, (C₂-C₆)alkenyl, (C₂-C₆)alkynyl, (C₃-C₆)cycloalkyl, (C₅-C₆)cycloalkenyl or phenyl, where each of the six last-mentioned radicals is unsubstituted or substituted by one or more radicals from the group consisting of halogen, hydroxyl, amino, nitro, formyl, carboxyl, cyano, thiocyanato, (C₁-C₄)alkoxy, (C₁₋C₄)haloalkoxy, (C₁-C₄)alkylthio, mono(C₁-C₄)alkylamino, di(C₁-C₄)alkylamino, phenyl and (C₃-C₆)cycloalkyl, where each of the 2 last-mentioned radicals is unsubstituted or substituted by one or more radicals from the group consisting of halogen, CN, SCN, NO₂, (C₁-C₄)alkyl, (C₁-C₄)haloalkyl, (C₁-C₄)alkoxy and (C₁-C₄)haloalkoxy, and, in the case of cyclic radicals, also (C₁-C₄)alkyl and (C₁-C₄)haloalkyl,
and
B* is a divalent group of the formula -CO-, -CO-O-, -CO-NR'-, -S(O)ₚ- or -S(O)ₚ-O-, where p = 0, 1 or 2 and R' is hydrogen, alkyl having 1 to 6 carbon atoms, phenyl, benzyl, cycloalkyl having 3 to 6 carbon atoms or alkanoyl having 1 to 4 carbon atoms,
where -B*-A* including substituents has 1 to 12 carbon atoms,
R⁴ is a radical of the formula -Z¹-R⁶, where Z¹ and R⁶ are as defined below,
R⁵ are each independently of one another halogen, CN, SCN, NO₂, a radical of the formula -Z²-R⁷, where Z² and R⁷ are as defined below, or two adjacent radicals R⁵ together are a fused-on cycle having 4 to 6 ring atoms which is carbocyclic or contains 1 to 3 hetero ring atoms from the group consisting of O, S and N and which is unsubstituted or substituted by one or more radicals from the group consisting of halogen, (C₁-C₄)alkyl and oxo, and
R⁶ and R⁷, R⁸, R⁹ are each independently of one another hydrogen, except that R⁷ is not hydrogen if Z² is a direct bond, or an acyclic hydrocarbon radical having 1 to 10 carbon atoms or a cyclic hydrocarbon radical having 3 to 6 carbon atoms or a heterocyclic radical, where each of the 3 last-mentioned carbon-containing radicals is unsubstituted or substituted by one or more radicals from the group consisting of halogen, hydroxyl, amino, nitro, formyl, aminocarbonyl, carboxyl, sulfonyl, cyano, thiocyanato, (C₁-C₆)alkoxy, (C₂-C₆)alkenyloxy, (C₂-C₆)alkynyloxy, (C₁-C₆)alkoxy-poly(alkyleneoxy), hydroxy-poly(alkyleneoxy), (C₁-C₆)alkylthio, mono- and di[(C₁-C₆)alkyl]amino, [(C₁-C₆)alkyl]carbonyl, [(C₂-C₆)alkenyl]carbonyl, [(C₂-C₆)alkynyl]carbonyl, [(C₁-C₆)alkoxy]carbonyl, [(C₂-C₆)alkenyloxy]carbonyl, [(C₂-C₆)alkynyloxy]carbonyl, mono- and di[(C₁-C₆)alkyl]aminocarbonyl, phenyl, phenoxy, (C₃-C₆)cycloalkyl, phenylcarbonyl, phenoxycarbonyl, heterocyclyl, (C₁-C₄)alkylsulfonyl and, in the case of cyclic radicals, also (C₁-C₄)alkyl,
where each of the 24 last-mentioned substituents is unsubstituted or substituted by one or more radicals from the group consisting of halogen, CN, SCN, NO₂, (C₁-C₄)alkoxy, (C₁-C₄)alkylthio, (C₁-C₄)haloalkoxy, formyl, (C₁-C₄)alkylcarbonyl, [(C₁-C₄)haloalkyl]carbonyl, [(C₁-C₄)alkoxy]carbonyl, [(C₁-C₄)haloalkoxy]carbonyl and, in the case of cyclic radicals, also (C₁-C₄)alkyl and (C₁-C₄)haloalkyl,
X¹, X², X³, X⁴ are each independently of one another a carbon atom which is substituted by a hydrogen atom or one of the substituents R⁵ defined above, or a nitrogen atom, or two adjacent symbols X¹, X², X³ and X⁴ are in each case together a divalent group of the formula -O-, -S-, -NH- or -NR-, where R is as defined for R³, provided the groups X¹, X², X³, X⁴ together with the attached C₂ unit of the fused-on ring form a carbocyclic or heterocyclic aromatic five- or six-membered ring,
(Y¹)ₘ are m divalent groups Y¹, where each group Y¹ independently of the other radicals Y¹ is a group of the formula -O-, -CO-, -C(=NR*)-, -S(O)_{q}-, -NR*- or -N(O)-, where q = 0, 1 or 2 and R* is as defined for R³ or a group of the formula CR⁸R⁹,
Y² is a group as defined for Y¹ or a direct bond,
where two adjacent groups of the symbol pairs Y¹ and Y¹ or of the symbol pairs Y¹ and Y² are groups with no heteroatoms having the same meaning, and where the groups (Y¹)ₘ and Y² together with the attached C₂ unit of the aromatic ring and the carbon atom attached to R⁴ form a fused-on carbocyclic or heterocyclic nonaromatic four- to eight-membered ring, and
Z¹ and the groups Z² are each independently of one another a direct bond or a divalent group of the formula -O-, -S(O)ₚ-, -S(O)ₚ-O-, -O-S(O)ₚ-, -CO-, -O-CO-, -CO-O-, -NR'-, -O-NR'-, -NR'-O-, -NR'-CO-, -CO-NR'-, where p = 0, 1 or 2 and R' is hydrogen, alkyl having 1 to 6 carbon atoms, phenyl, benzyl, cycloalkyl having 3 to 6 carbon atoms or alkanoyl having 1 to 6 carbon atoms,
where heterocyclyl, unless defined otherwise, and including in fused radicals, has in each case 3 to 9 ring atoms and 1 to 3 hetero ring atoms from the group consisting of N, O and S.

3. A compound or a salt thereof as claimed in claim 1 or 2, wherein
R¹ and R² are each independently of one another hydrogen, amino, mono- or di[(C₁-C₄)alkyl]amino or (C₃-C₆)cycloalkylamino, (C₅-C₆)cycloalkenylamino, a hydrocarbon radical or hydrocarbonoxy radical having in each case 1 to 10 carbon atoms or a heterocyclyl radical, heterocyclyloxy radical, heterocyclylthio radical or heterocyclylamino radical having in each case 3 to 9 ring atoms and 1 to 3 hetero ring atoms from the group consisting of N, O and S, where each of the six last-mentioned radicals is unsubstituted or substituted by one or more radicals from the group consisting of halogen, hydroxyl, amino, nitro, formyl, aminocarbonyl, carboxyl, sulfonyl, cyano, thiocyanato, (C₁-C₄)alkoxy, (C₂-C₄)alkenyloxy, (C₂-C₄)alkynyloxy, (C₁-C₄)alkoxy-poly(alkyleneoxy), hydroxy-poly(alkyleneoxy), (C₁-C₄)alkylthio, mono- and di[(C₁-C₄)alkyl]amino, [(C₁-C₄)alkyl]carbonyl, [(C₂-C₄)alkenyl]carbonyl, [(C₂-C₄)alkynyl]carbonyl, [(C₁-C₄)alkoxy]carbonyl, [(C₂-C₄)alkenyloxy]carbonyl, [(C₂-C₄)alkynyloxy]carbonyl, mono- and di[(C₁-C₄)alkyl]aminocarbonyl, phenyl, phenoxy, (C₃-C₆)cycloalkyl, phenylcarbonyl, phenoxycarbonyl, heterocyclyl, (C₁-C₄)alkylsulfonyl and, in the case of cyclic radicals, also (C₁-C₄)alkyl,
where each of the 24 last-mentioned substituents is unsubstituted or substituted by one or more radicals from the group consisting of halogen, CN, SCN, NO₂, (C₁-C₄)alkoxy, (C₁-C₄)haloalkoxy, formyl, (C₁-C₄)alkylcarbonyl, [(C₁-C₄)haloalkyl]carbonyl, [(C₁-C₄)alkoxy]carbonyl, [(C₁-C₄)haloalkoxy]carbonyl and, in the case of cyclic radicals, also (C₁-C₄)alkyl and (C₁-C₄)haloalkyl,
or an acyl radical of an organic acid from the group consisting of formyl, aminocarbonyl, mono- or di[(C₁-C₄)alkyl]aminocarbonyl, [(C₁-C₄)alkyl]carbonyl, [(C₂-C₄)alkenyl]carbonyl, [(C₂-C₄)alkynyl]carbonyl, [(C₁-C₄)alkoxy]carbonyl, [(C₂-C₄)alkenyloxy]carbonyl, phenylcarbonyl, (C₁-C₄)alkylsulfonyl, where each of the 9 last-mentioned radicals is unsubstituted in the aliphatic moiety or in the phenyl ring or substituted by one or more radicals from the group consisting of halogen, CN, SCN, NO₂, (C₁-C₄)alkoxy, (C₁-C₄)haloalkoxy, optionally substituted and unsubstituted phenyl and, in the case of cyclic radicals, also (C₁-C₄)alkyl and (C₁-C₄)haloalkyl,
or
R¹ and R² together with the nitrogen atom of the group NR¹R² are a saturated or unsaturated, nonaromatic heterocyclic radical having 3 to 6 ring atoms and 1 to 4 hetero ring atoms, where the further hetero ring atoms optionally present in addition to the nitrogen atom are selected from the group consisting of N, O and S and the radical is unsubstituted or substituted by one or more radicals from the group consisting of halogen, CN, (C₁-C₄)alkyl, (C₁-C₄)haloalkyl, (C₁-C₄)alkoxy, (C₁-C₄)haloalkoxy, (C₁-C₄)alkoxycarbonyl and oxo,
R³ is hydrogen, amino, formyl, (C₁-C₄)alkyl, cyano-(C₁-C₄)alkyl, (C₁-C₄)haloalkyl, mono-, di- or polyhydroxy-(C₁-C₄)alkyl, hydroxy-poly[(C₂-C₄)alkyleneoxy]-(C₁-C₄)alkyl, mono-, di- or poly(C₁-C₄)alkoxy-(C₁-C₄)alkyl, (C₁-C₄)alkoxy-poly[(C₂-C₄)alkyleneoxy]-(C₁-C₄)alkyl, (C₁-C₄)haloalkoxy-(C₁-C₄)alkyl, (C₂-C₆)alkenyl, (C₂-C₆)haloalkenyl, (C₂-C₆)alkynyl, (C₂-C₆)haloalkynyl or aminocarbonyl
or
(C₁-C₄)alkylamino-(C₁-C₄)alkyl, di[(C₁-C₄)alkyl]amino-(C₁-C₄)alkyl, (C₁-C₄)alkylaminocarbonyl, di(C₁-C₄)alkylaminocarbonyl, (C₁-C₅)alkanoyl, [(C₂-C₄)alkenyl]carbonyl, [(C₁-C₄)alkoxy]carbonyl, (C₂-C₄)alkenyloxycarbonyl, aminocarbonyl-(C₁-C₄)alkyl, (C₁-C₄)alkylaminocarbonyl-(C₁-C₄)alkyl, di[(C₁-C₄)alkyl]aminocarbonyl-(C₁-C₄)alkyl, (C₁-C₄)alkoxycarbonyl-(C₁-C₄)alkyl, (C₁-C₅)alkanoyl-(C₁-C₄)alkyl, [(C₂-C₄)alkenyl]carbonyl-(C₁-C₄)alkyl, [(C₁-C₄)alkoxy]carbonyl-(C₁-C₄)alkyl or (C₂-C₄)alkenyloxycarbonyl-(C₁-C₄)alkyl, where each of the 16 last-mentioned radicals is unsubstituted or substituted by one or more radicals from the group consisting of halogen, CN, (C₁-C₄)alkoxy and (C₁-C₄)haloalkoxy, or (C₃-C₆)cycloalkyl, (C₃-C₆)cycloalkylamino-(C₁-C₄)alkyl, phenyl, phenylcarbonyl, phenoxycarbonyl, phenylcarbonyl-(C₁-C₄)alkyl, phenoxy-(C₁-C₄)alkyl, phenyl-(C₁-C₄)alkyl, heterocyclyl, heterocyclyl-(C₁-C₄)alkyl or one of the 10 last-mentioned radicals which is substituted in the acylic moiety or in the cyclic moiety by one or more radicals from the group consisting of halogen, nitro, cyano, (C₁-C₄)alkyl, (C₁-C₄)alkoxy, (C₁-C₄)alkylthio, (C₁-C₄)haloalkyl, (C₁-C₄)haloalkoxy, formyl, (C₁-C₄)alkylcarbonyl, (C₁-C₄)alkoxycarbonyl and (C₁-C₄)alkoxy,
R⁴ is hydrogen, formyl, carboxyl, cyano, thiocyanato, aminocarbonyl, (C₁-C₄)alkyl, cyano-(C₁-C₄)alkyl, (C₁-C₄)alkoxy, (C₁-C₄)alkylamino, di[(C₁-C₄)alkyl]amino, halo-(C₁-C₄)alkyl, hydroxy-(C₁-C₄)alkyl, (C₁-C₄)alkoxy-(C₁-C₄)alkyl, halo-(C₁-C₄)alkoxy-(C₁-C₄)alkyl, (C₁-C₄)alkylthio, halo-(C₁-C₄)alkylthio, (C₂-C₆)alkenyl, halo-(C₂-C₆)alkenyl, (C₂-C₆)alkynyl, halo-(C₂-C₆)alkynyl, (C₁-C₄)alkylamino-(C₁-C₄)alkyl, di[(C₁-C₄)alkyl]amino-(C₁-C₄)alkyl, (C₃-C₉)cycloalkylamino-(C₁-C₄)alkyl, (C₃-C₉)cycloalkyl, heterocyclyl-(C₁-C₄)alkyl having 3 to 6 ring members where the cyclic groups in the 3 last-mentioned radicals are unsubstituted or substituted by one or more radicals from the group consisting of (C₁-C₄)alkyl, halogen and cyano, or
phenyl, phenoxy, phenylcarbonyl, phenylcarbonyl-(C₁-C₄)alkyl, phenoxycarbonyl, (C₁-C₄)alkylcarbonyl, (C₁-C₄)alkoxycarbonyl, (C₁-C₄)alkylaminocarbonyl, (C₁-C₄)alkoxycarbonyl-(C₁-C₄)alkyl, (C₁-C₄)alkylaminocarbonyl-(C₁-C₄)alkyl, phenoxy-(C₁-C₄)alkyl, phenyl-(C₁-C₄)alkyl, heterocyclyl, heterocyclylamino, heterocyclyloxy, heterocyclylthio, (C₁-C₅)alkanoylamino, N-[(C₁-C₅)alkanoyl]-N-[(C₁-C₄)alkyl]amino, [(C₂-C₄)alkenyl]carbonylamino, [(C₂-C₄)alkynyl]carbonylamino, [(C₁-C₄)alkoxy]carbonylamino, [(C₂-C₄)alkenyloxy]carbonylamino, [(C₂-C₄)alkynyloxy]carbonylamino, phenylcarbonylamino, phenoxycarbonylamino, (C₁-C₄)alkylsulfonyl, (C₂-C₄)alkenylsulfonyl or one of the 27 last-mentioned radicals which is substituted in the acyclic moiety or in the cyclic moiety by one or more radicals from the group consisting of halogen, nitro, cyano, (C₁-C₄)alkoxy, (C₁-C₄)alkylthio, (C₁-C₄)haloalkoxy, formyl, (C₁-C₄)alkylcarbonyl, (C₁-C₄)alkoxycarbonyl, (C₁-C₄)alkoxy and, in the case of cyclic moieties, also (C₁-C₄)alkyl and (C₁-C₄)haloalkyl,
R⁵, if n = 1, and the radicals R⁵ in each case independently of one another, if n is greater than 1, is/are halogen, hydroxyl, amino, nitro, formyl, carboxyl, cyano, thiocyanato, aminocarbonyl, (C₁-C₄)alkyl, cyano-(C₁-C₄)alkyl, (C₁-C₄)alkoxy, (C₁-C₄)alkylamino, di[(C₁-C₄)alkyl]amino, halo-(C₁-C₄)alkyl, hydroxy-(C₁-C₄)alkyl, (C₁-C₄)alkoxy-(C₁-C₄)alkyl, halo-(C₁-C₄)alkoxy-(C₁-C₄)alkyl, (C₁-C₄)alkylthio, halo-(C₁-C₄)alkylthio, (C₂-C₆)alkenyl, halo-(C₂-C₆)alkenyl, (C₂-C₆)alkynyl, halo-(C₂-C₆)alkynyl, (C₁-C₄)alkylamino-(C₁-C₄)alkyl, di[(C₁-C₄)alkyl]amino-(C₁-C₄)alkyl, (C₃-C₉)cycloalkylamino-(C₁-C₄)alkyl, (C₃-C₉)cycloalkyl, heterocyclyl-(C₁-C₄)alkyl having 3 to 6 ring members, where the cyclic groups in the 3 last-mentioned radicals are unsubstituted or substituted by one or more radicals from the group consisting of (C₁-C₄)alkyl, halogen and cyano,or
phenyl, phenoxy, phenylcarbonyl, phenylcarbonyloxy, phenylcarbonyl-(C₁-C₄)alkyl, phenoxycarbonyl, (C₁-C₄)alkylcarbonyl, (C₁-C₄)alkoxycarbonyl, (C₁-C₄)alkylaminocarbonyl, (C₁-C₄)alkoxycarbonyl-(C₁-C₄)alkyl, (C₁-C₄)alkylaminocarbonyl-(C₁-C₄)alkyl, phenoxy-(C₁-C₄)alkyl, phenyl-(C₁-C₄)alkyl, heterocyclyl, heterocyclylamino, heterocyclyloxy, heterocyclylthio, (C₁-C₅)alkanoylamino, N-[(C₁-C₅)alkanoyl]-N-[(C₁-C₄)alkyl]amino, [(C₂-C₄)alkenyl]carbonylamino, [(C₂-C₄)alkynyl]carbonylamino, [(C₁-C₄)alkoxy]carbonylamino, [(C₂-C₄)alkenyloxy]carbonylamino, [(C₂-C₄)alkynyloxy]carbonylamino, phenylcarbonylamino, phenoxycarbonylamino, (C₁-C₄)alkylsulfonyl, (C₂-C₄)alkenylsulfonyl or one of the 28 last-mentioned radicals which is substituted in the acyclic moiety or in the cyclic moiety by one or more radicals from the group consisting of halogen, nitro, cyano, (C₁-C₄)alkoxy, (C₁-C₄)alkylthio, (C₁-C₄)haloalkoxy, formyl, (C₁-C₄)alkylcarbonyl, (C₁-C₄)alkoxycarbonyl, (C₁-C₄)alkoxy and, in the case of cyclic moieties, also (C₁-C₄)alkyl and (C₁-C₄)haloalkyl,
where heterocyclyl in the radicals contains in each case 3 to 9 ring atoms and 1 to 3 hetero ring atoms from the group consisting of N, O and S,
or two adjacent radicals R⁵ together are a fused-on cycle having 4 to 6 ring atoms which is carbocyclic or contains hetero ring atoms from the group consisting of O, S and N and which is unsubstituted or substituted by one or more radicals from the group consisting of halogen, (C₁-C₄)alkyl and oxo,
R⁸ and R⁹ are each independently of one another hydrogen, formyl, carboxyl, cyano, thiocyanato, aminocarbonyl, (C₁-C₄)alkyl, cyano-(C₁-C₄)alkyl, (C₁-C₄)alkoxy, (C₁-C₄)alkylamino, di[(C₁-C₄)alkyl]amino, halo-(C₁-C₄)alkyl, hydroxy-(C₁-C₄)alkyl, (C₁-C₄)alkoxy-(C₁-C₄)alkyl, halo-(C₁-C₄)alkoxy-(C₁-C₄)alkyl, (C₁-C₄)alkylthio, halo-(C₁-C₄)alkylthio, (C₂-C₆)alkenyl, halo-(C₂-C₆)alkenyl, (C₂-C₆)alkynyl, halo-(C₂-C₆)alkynyl, (C₁-C₄)alkylamino-(C₁-C₄)alkyl, di[(C₁-C₄)alkyl]amino-(C₁-C₄)alkyl, (C₃-C₉)cyclooalkylamino-(C₁-C₄)alkyl, (C₃-C₉)cycloalkyl, heterocyclyl-(C₁-C₄)alkyl having 3 to 6 ring members, where the cyclic groups in the 3 last-mentioned radicals are unsubstituted or substituted by one or more radicals from the group consisting of (C₁-C₄)alkyl, halogen and cyano, or phenyl, phenoxy, phenylcarbonyl, phenylcarbonyl-(C₁-C₄)alkyl, phenoxycarbonyl, (C₁-C₄)alkylcarbonyl, (C₁-C₄)alkoxycarbonyl, (C₁-C₄)alkylaminocarbonyl, (C₁-C₄)alkoxycarbonyl-(C₁-C₄)alkyl, (C₁-C₄)alkylaminocarbonyl-(C₁-C₄)alkyl, phenoxy-(C₁-C₄)alkyl, phenyl-(C₁-C₄)alkyl, heterocyclyl, heterocyclylamino, heterocyclyloxy, heterocyclylthio, (C₁-C₅)alkanoylamino, N-[(C₁-C₅)alkanoyl]-N-[(C₁-C₄)alkyl]amino, [(C₂-C₄)alkenyl]carbonylamino, [(C₂-C₄)alkynyl]carbonylamino, [(C₁-C₄)alkoxy]carbonylamino, [(C₂-C₄)alkenyloxy]carbonylamino, [(C₂-C₄)alkynyloxy]carbonylamino, phenylcarbonylamino, phenoxycarbonylamino, (C₁-C₄)alkylsulfonyl, (C₂-C₄)alkenylsulfonyl or one of the 27 last-mentioned radicals which is substituted in the acyclic moiety or in the cyclic moiety by one or more radicals from the group consisting of halogen, nitro, cyano, (C₁-C₄)alkoxy, (C₁-C₄)alkylthio, (C₁-C₄)haloalkoxy, formyl, (C₁-C₄)alkylcarbonyl, (C₁-C₄)alkoxycarbonyl, (C₁-C₄)alkoxy and, in the case of cyclic moieties, also (C₁-C₄)alkyl and (C₁-C₄)haloalkyl,
X¹, X², X³, X⁴ are each independently of one another a carbon atom which is substituted by a hydrogen atom or one of the substituents R⁵ defined above, or a nitrogen atom, or two adjacent symbols X¹, X², X³ and X⁴ are in each case together a divalent group of the formula -O-, -S-, -NH- or -NR-, where R is as defined for R³, with the proviso that the groups X¹, X², X³, X⁴ together with the attached C₂ unit of the fused-on ring form a carbocyclic or heterocyclic aromatic, five- or six-membered ring,
(Y¹)ₘ are m divalent groups Y¹, where each group Y¹ independently of the other radicals Y¹ is a group of the formula -O-, -CO-, -C(=NR*)-, -S(O)_{q}-, -NR*- or -N(O)-, where q = 0, 1 or 2 and R* is as defined for R³ or a group of the formula CR⁸R⁹,
Y² is a group as defined for Y¹ or a direct bond,
where two adjacent groups of the symbol pairs Y¹ and Y¹ or of the symbol pairs Y¹ and Y² are groups with no heteroatoms having the same meaning, and where the groups (Y¹)ₘ and Y² together with the attached C₂ unit of the aromatic ring and the carbon atom attached to R⁴ form a fused-on carbocyclic or heterocyclic nonaromatic four- to eight-membered ring,
where heterocyclyl, unless defined otherwise, and including in fused radicals, has in each case 3 to 9 ring atoms and 1 to 3 hetero ring atoms from the group consisting of N, O and S.

4. A compound or a salt thereof as claimed in any of claims 1 to 3, wherein
R¹, R² independently of one another are hydrogen, amino, formyl, (C₁-C₄)alkyl, (C₁-C₄)alkylamino, di[(C₁-C₄)alkyl]amino, hydroxy-(C₁-C₄)alkyl, (C₁-C₄)alkoxy-(C₁-C₄)alkyl, halo-(C₁-C₄)alkoxy-(C₁-C₄)alkyl, (C₂-C₆)alkenyl, (C₂-C₆)alkynyl, halo-(C₂-C₆)alkynyl, (C₁-C₄)alkylamino-(C₁-C₄)alkyl or phenyl, phenoxy, phenylcarbonyl, phenylcarbonyl-(C₁-C₄)alkyl, (C₁-C₄)alkoxycarbonyl-(C₁-C₄)alkyl, (C₁-C₄)alkylaminocarbonyl-(C₁-C₄)alkyl, (C₁-C₄)alkylcarbonyl, (C₁-C₄)alkoxycarbonyl, aminocarbonyl, (C₁-C₄)alkylaminocarbonyl or one of the 10 last-mentioned radicals which is substituted in the acyclic moiety or in the cyclic moiety by one or more radicals from the group consisting of halogen, nitro, cyano, (C₁-C₄)alkyl, (C₁-C₄)alkoxy, (C₁-C₄)alkylthio, (C₁-C₄)haloalkyl, formyl, (C₁-C₄)alkylcarbonyl, (C₁-C₄)alkoxycarbonyl, (C₁-C₄)alkoxy,
R³ is hydrogen, amino, formyl, (C₁-C₄)alkyl, cyano-(C₁-C₄)alkyl, (C₁-C₄)alkoxy-(C₁-C₄)alkyl, (C₂-C₆)alkenyl, halo-(C₂-C₆)alkenyl, (C₂-C₆)alkynyl or phenyl, (C₁-C₄)alkylcarbonyl, (C₁-C₄)alkoxycarbonyl, aminocarbonyl, phenoxy-(C₁-C₄)alkyl, phenyl-(C₁-C₄)alkyl or one of the 6 last-mentioned radicals which is substituted in the acyclic moiety or in the cyclic moiety by one or more radicals from the group consisting of halogen, (C₁-C₄)alkyl and (C₁-C₄)alkoxy,
R⁴ is hydrogen, (C₁-C₄)alkyl, cyano-(C₁-C₄)alkyl, halo-(C₁-C₄)alkyl, hydroxy-(C₁-C₄)alkyl, (C₁-C₄)alkoxy-(C₁-C₄)alkyl, halo-(C₁-C₄)alkoxy-(C₁-C₄)alkyl, (C₂-C₆)alkenyl, halo-(C₂-C₆)alkenyl, (C₂-C₆)alkynyl, (C₁-C₄)alkylamino-(C₁-C₄)alkyl, (C₃-C₉)cycloalkylamino-(C₁-C₄)alkyl, (C₃-C₉)cycloalkyl, heterocyclyl-(C₁-C₄)alkyl having 3 to 9 ring members, where the cyclic groups in the 3 last-mentioned radicals are unsubstituted or substituted by one or more radicals from the group consisting of (C₁-C₄)alkyl, halogen and cyano, or phenyl, phenoxy-(C₁-C₄)alkyl, phenyl-(C₁-C₄)alkyl, heterocyclyl, or one of the 4 last-mentioned radicals which is substituted in the acyclic moiety or in the cyclic moiety by one or more radicals from the group consisting of halogen, nitro, cyano, (C₁-C₄)alkoxy, (C₁-C₄)alkylthio, (C₁-C₄)haloalkoxy, (C₁-C₄)alkylcarbonyl, (C₁-C₄)alkoxycarbonyl, (C₁-C₄)alkoxy and, in the case of cyclic moieties, also (C₁-C₄)alkyl and (C₁-C₄)haloalkyl,
R⁵, if n = 1, and the radicals R⁵ in each case independently of one another, if n is greater than 1, is/are halogen, hydroxyl, amino, nitro, formyl, cyano, aminocarbonyl, (C₁-C₄)monoalkylaminocarbonyl, (C₁-C₄)dialkylaminocarbonyl, (C₁-C₄)alkyl, cyano-(C₁-C₄)alkyl, (C₁-C₄)alkoxy, (C₁-C₄)alkylamino, halo-(C₁-C₄)alkyl, hydroxy-(C₁-C₄)alkyl, (C₁-C₄)alkoxy-(C₁-C₄)alkyl, (C₂-C₄)alkenyl, (C₂-C₄)alkynyl, (C₁-C₄)alkylamino-(C₁-C₄)alkyl, di[(C₁-C₄)alkyl]amino-(C₁-C₄)alkyl, or [(C₁-C₄)alkyl]carbonyl, [(C₁-C₄)haloalkyl]carbonyl, [(C₁-C₄)alkoxy-(C₁-C₄)alkyl)carbonyl, formylamino, [(C₁-C₄)alkyl]carbonylamino, [(C₁-C₄)haloalkyl]carbonylamino, phenyl, phenoxy, phenylcarbonyl, phenylcarbonylamino, heterocyclyl or one of the 5 last-mentioned radicals which is substituted by one or more radicals from the group consisting of halogen, (C₁-C₄)alkyl, (C₁-C₄)haloalkyl, (C₁-C₄)alkoxy, (C₁-C₄)haloalkoxy and (C₁-C₄)alkylthio,
R⁸ and R⁹ are each independently of one another hydrogen, (C₁-C₄)alkyl, cyano-(C₁-C₄)alkyl, halo-(C₁-C₄)alkyl, hydroxy-(C₁-C₄)alkyl, (C₁-C₄)alkoxy-(C₁-C₄)alkyl, halo-(C₁-C₄)alkoxy-(C₁-C₄)alkyl, (C₂-C₆)alkenyl, halo-(C₂-C₆)alkenyl, (C₂-C₆)alkynyl, (C₁-C₄)alkylamino-(C₁-C₄)alkyl, (C₃-C₉)cycloalkylamino-(C₁-C₄)alkyl, (C₃-C₉)cycloalkyl, heterocyclyl-(C₁-C₄)alkyl having 3 to 9 ring members, where the cyclic groups in the 3 last-mentioned radicals are unsubstituted or substituted by one or more radicals from the group consisting of (C₁-C₄)alkyl, halogen and cyano, or phenyl, phenoxy-(C₁-C₄)alkyl, phenyl-(C₁-C₄)alkyl, heterocyclyl, or one of the 4 last-mentioned radicals which is substituted in the acyclic moiety or in the cyclic moiety by one or more radicals from the group consisting of halogen, nitro, cyano, (C₁-C₄)alkoxy, (C₁-C₄)alkylthio, (C₁-C₄)haloalkoxy, (C₁-C₄)alkylcarbonyl, (C₁-C₄)alkoxycarbonyl, (C₁-C₄)alkoxy and, in the case of cyclic moieties, also (C₁-C₄)alkyl and (C₁-C₄)haloalkyl.

5. A compound or a salt thereof as claimed in any of claims 1 to 4, wherein
X¹, X², X³, X⁴ are each independently of one another a carbon atom which is substituted by a hydrogen atom or one of the substituents R⁵ defined above,
or a nitrogen atom or two adjacent symbols X¹, X², X³ and X⁴ are in each case together a divalent group of the formula -O-, -S-, -NH- or -NR-, where R is hydrogen or (C₁-C₄)alkyl, with the proviso that the groups X¹, X², X³, X⁴ together with the attached C₂ unit of the fused-on ring form a carbocyclic or heterocyclic aromatic, five- or six-membered ring,
(Y¹)ₘ are m divalent groups Y¹, where each group Y¹ independently of the other radicals Y¹ is a group of the formula CH₂, CH(CH₃), CH(C₂H₅), CH(CH₃)₂ or CH(C₆H₅) and m = 0, 1, 2 or 3 and
Y² is a direct bond or a group of the formula -O-, -S-, CH₂, CH(CH₃) or (C₁-C₄)alkylamino,
where two adjacent groups of the symbol pairs Y¹ and Y¹ or of the symbol pairs Y¹ and Y² are groups without heteroatoms having the same meaning, and where the groups (Y¹)ₘ and Y² together with the attached C₂ unit of the aromatic ring and with the carbon atom attached to R⁴ form a fused-on carbocyclic or heterocyclic nonaromatic, four- to eight-membered ring.

6. A process for preparing compounds of the formula (I) and salts thereof as defined in any of claims 1 to 5, which comprises
a) reacting a compound of the formula (II) ,
H-R¹³ (II)
in which R¹³ is a functional group from the group of the carboxylic esters, carboxylic orthoesters, carbonyl chlorides, carboxamides and carboxylic anhydrides
with a biguanide of the formula (III) or an acid addition salt thereof 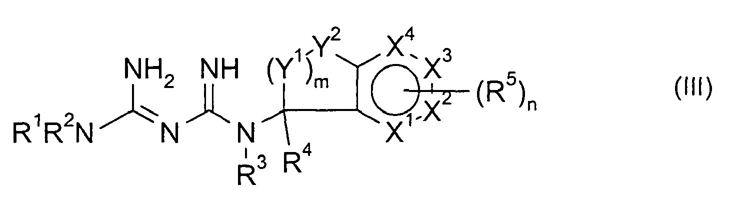 or
b) reacting a compound of the formula (IV), 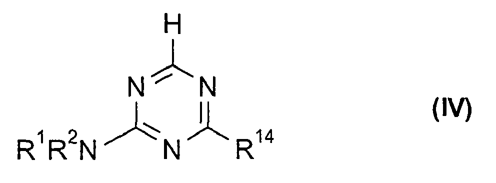 where R¹⁴ is an exchangeable radical or a leaving group, with an amine of the formula (V) or an acid addition salt thereof 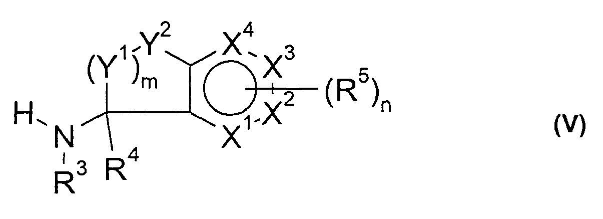 or
c) reacting a diamino-1,3,5-triazine of the formula (VI) with an isocyanate of the formula (VII) 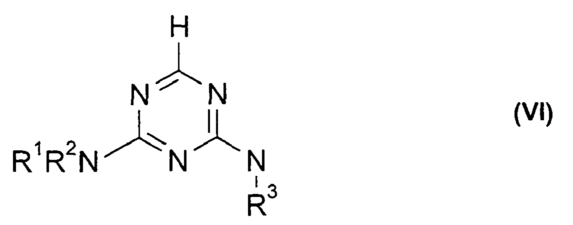 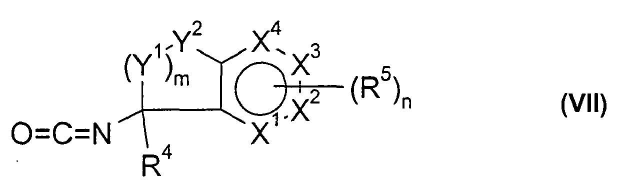 or
d) removing, in a triazine of the structure (VIII), the radical R¹⁵ which is a leaving group or a radical that can be removed 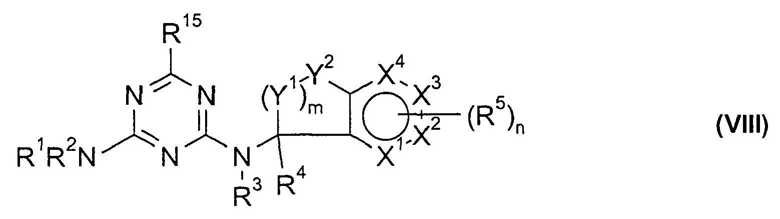
where in the formulae (II), (III), (IV), (V), (VI), (VII) and (VIII) the radicals R¹, R², R³, R⁴, R⁵, X¹, X², X³, X⁴, Y¹ and Y² and the symbols m and n are as defined in formula (I).

7. A herbicidal or plant-growth-regulating composition, which comprises one or more compounds of the formula (I) or salts thereof as claimed in any of claims 1 to 5 and formulation auxiliaries which can be used in crop protection.

8. A method for controlling harmful plants or for regulating the growth of plants, which comprises applying an effective amount of one or more compounds of the formula (I) or salts thereof as claimed in any of claims 1 to 5 onto the plants, plant seeds or the area under cultivation.

9. The use of compounds of the formula (I) or salts thereof as claimed in any of claims 1 to 5 as herbicides or plant growth regulators.

10. The use as claimed in claim 9, wherein the compounds of the formula (I) or salts thereof are used for controlling harmful plants or for regulating growth in crops of useful or ornamental plants.

## Revendications

1. Composés de formule (I) ou leurs sels 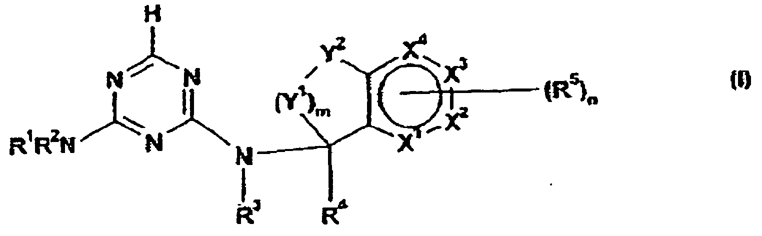 dans laquelle
R¹ et R² indépendamment l'un de l'autre, représentent un atome d'hydrogène, un groupe amino, alkylamino ou dialkylamino comportant respectivement de 1 à 4 atomes de C dans le radical alkyle ou un groupe cycloalkylamino en C₃ à C₈, cycloalcényl-amino en C₅ à C₈, un radical hydrocarbure (acyclique ou cyclique) ou un oxyradical hydrocarbure comportant de 1 à 10 atomes de C, de préférence de 1 à 6 atomes de C, ou bien un radical hétérocyclyle, un oxyradical hétérocyclyle, un radical thio-hétérocyclyle ou un radical amino hétérocyclyle avec, chacun, de 3 à 9 atomes cycliques, de préférence de 3 à 6 atomes cycliques, et de 1 à 3 atomes hétérocycliques du groupe N, O et S, chacun des six derniers radicaux cités étant non substitué, ou substitué par un ou plusieurs radicaux du groupe halogène, hydroxy, amino, nitro, formyle, aminocarbonyle, carboxy, sulfonyle, cyano, thiocyanato, alcoxy en C₁ à C₆, alcényloxy en C₂ à C₆, alcynyloxy en C₂ à C₆, alcoxy-poly(alkylénoxy) en C₁ à C₆, hydroxy-poly(alkylénoxy), alkylthio en C₁ à C₆, mono et dialkyl(en C₁ à C₆)amino, alkyl(en C₁ à C₆)carbonyle, alcényl(en C₂ à C₆)carbonyle, alcynyl(en C₂ à C₆)-carbonyle, alcoxy(en C₁ à C₆)carbonyle, alcényloxy(en C₂ à C₆)carbonyle, alcynyloxy(en C₂ à C₆)carbonyle, mono et dialkyl(en C₁ à C₆)amino-carbonyle, phényle, phénoxy, cycloalkyle en C₃ à C₆, phénylcarbonyle, phénoxycarbonyle, hétérocyclyle, alkylsulfonyle en C₁ à C₄ et, dans le cas de radicaux cycliques, également alkyle en C₁ à C₄,
chacun des 24 derniers substituants cités étant non substitué ou substitué par un ou plusieurs radicaux du groupe halogène, CN, SCN, NO₂, alcoxy en C₁ à C₄, alkylthio en C₁ à C₄, haloalcoxy en C₁ à C₄, formyle, alkylcarbonyle en C₁ à C₄, haloalkyl(en C₁ à C₄)carbonyle, alcoxy(en C₁ à C₄)carbonyle, haloalcoxy(en C₁ à C₄)carbonyle et, dans le cas de radicaux cycliques, également alkyle en C₁ à C₄ et haloalkyle en C₁ à C₄,
ou par un radical acyle d'un acide organique du groupe formyle, aminocarbonyle, mono ou dialkyl(en C₁ à C₄)aminocarbonyle, alkyl(en C₁ à C₆)carbonyle, alcényl(en C₂ à C₆)carbonyle, alcynyl(en C₂ à C₆)carbonyle, alcoxy(en C₁à C₆)carbonyle, alcényloxy(en C₂ à C₆)carbonyle, phénylcarbonyle, alkylsulfonyle en C₁ à C₆ chacun des 9 derniers radicaux cités étant non substitué dans la partie aliphatique ou dans le cycle phényle ou bien substitué par un ou plusieurs radicaux du groupe halogène, CN, SCN, NO₂, alcoxy en C₁ à C₄, haloalcoxy en C₁ à C₄, phényle le cas échéant substitué et, de préférence, non substitué, et, dans le cas de radicaux cycliques, également alkyle en C₁ à C₆ et haloalkyle en C₁ à C₆,
le radical hétérocyclyle, s'il n'est pas définit plus précisément, pouvant présenter aussi, dans les radicaux composés, respectivement 3 à 9 atomes cycliques, de préférence 3 à 6 atomes cycliques et 1 à 3 atomes hétérocycliques du groupe N, O et S,
ou
R¹ et R² comportent, conjointement à l'atome d'azote du groupe NR¹R², un radical hétérocyclique saturé ou insaturé, non aromatique comportant 3 à 6 atomes cycliques et 1 à 4 atomes hétérocycliques, les éventuels autres atomes hétérocycliques étant choisis, outre l'atome de N, dans le groupe N, O et S et le radical étant non substitué ou bien substitué par un ou plusieurs radicaux du groupe halogène, CN, alkyle en C₁ à C₄, haloalkyle en C₁ à C₄, alcoxy en C₁ à C₄, haloalcoxy en C₁ à C₄, alcoxy(en C₁ à C₄)carbonyle et oxo,
chacun des radicaux R¹ et R², contenant du carbone, y compris les substituants, présentent 1 à 20 atomes de C,
R³ représentant un atome d'hydrogène, un radical amino, alkyle en C₁ à C₆, alcoxy en C₁ à C₆, alcényle en C₂ à C₈, alcynyle en C₂ à C₆, cycloalkyle en C₃ à C₆, cycloalcényle en C₅ à C₆, phényle ou hétérocyclyle, chacun des huit derniers radicaux cités étant non substitué ou bien substitué par un ou plusieurs radicaux du groupe halogène, hydroxy, amino, nitro, formyle, carboxy, cyano, thiocyanato, alcoxy en C₁ à C₄, alcoxy(en C₁ à C₄)-alcoxy(en C₁ à C₄), hydroxyalcoxy en C₁ à C₄, alcoxy(en C₁ à C₄)-poly(alkylénoxy), hydroxy-poly(alkylénoxy), haloalcoxy en C₁ à C₄, alkylthio en C₁ à C₄, haloalkylthio en C₁ à C₄, monoalkyl(en C₁ à C₄)amino, dialkyl(en C₁ à C₄)amino, alkyl (en C₁ à C₄) carbonyle, alcoxy(en C₁ à C₁)carbonyle, aminocarbonyle, monoalkyl(en C₁ à C₄)aminocarbonyle, dialkyl(en C₁ à C₄)aminocarbonyle, phényle, phénoxy, cycloalkyle en C₃ à C₆, phénylcarbonyle, phénoxycarbonyle, hétérocyclyle, chacun des 6 derniers radicaux étant non substitué ou bien substitué par un ou plusieurs radicaux du groupe halogène, CN, SCN, NO₂, alkyle en C₁ à C₄, haloalkyle en C₁ à C₄, alcoxy en C₁ à C₄ et haloalcoxy (en C₁ à C₄), et alkyl(en C₁ à C₄)sulfonyle et haloalkylsulfonyle en C₁ à C₄ et, dans le cas de radicaux cycliques, également alkyle en C₁ à C₄ et haloalkyle en C₁ à C₄,
ou bien un radical acyle de formule -B*-A*, dans laquelle
A* représente un atome d'hydrogène ou un radical alkyle en C₁ à C₆, alcényle en C₂ à C₆, alcynyle en C₂ à C₆, cycloalkyle en C₃ à C₆, cycloalcényle en C₅ à C₆ ou phényle, chacun des six derniers radicaux cités étant non substitué ou bien substitué par un ou plusieurs radicaux du groupe halogène, hydroxy, amino, nitro, formyle, carboxy, cyano, thiocyanato, alcoxy en C₁ à C₄, haloalcoxy en C₁ à C₄, alkylthio en C₁ à C₄, monoalkyl(en C₁ à C₄)amino, dialkyl(en C₁ à C₄)amino, phényle et cycloalkyle en C₃ à C₆, chacun des 2 derniers radicaux cités étant non substitué ou bien substitué par un ou plusieurs radicaux du groupe halogène, CN, SCN, NO₂, alkyle en C₁ à C₄, haloalkyle en C₁ à C₄, alcoxy en C₁ à C₄ et haloalcoxy en C₁ à C₄, et, dans le cas de radicaux cycliques également alkyle en C₁ à C₄ et haloalkyle en C₁ à C₄,
et
B* un groupe divalent de formule -CO-, -CO-O-, -CO-NR'-, -S(O)ₚ- ou -S(O)ₚ-O-, p étant égal à 0, 1 ou 2 et R' représentant un atome d'hydrogène, un radical alkyle comportant de 1 à 6 atomes de C, phényle, benzyle, cycloalkyle comportant de 3 à 6 atomes de C ou alcanoyle comportant de 1 à 4 atomes de C,
-B*-A*, y compris les substituants, présentant 1 à 12 de carbone,
R⁴ est un radical de formule -Z¹-R⁶, Z¹ et R⁶ étant définis comme indiqué ci-dessous,
R⁵ représente, indépendamment l'un de l'autre, un radical halogène, cyano, isocyanato, nitro, un radical de formule -Z²-R⁷, Z² et R⁷ étant définis comme indiqués ci-dessous
ou
deux radicaux R⁵ voisins représentant un cycle condensé comportant de 4 à 6 atomes cycliques qui est carbocyclique ou contient de 1 à 3 atomes hétérocycliques du groupe O, S et N et qui est non substitué ou substitué par un ou plusieurs radicaux du groupe halogène, alkyle en C₁ à C₄ et oxo,
R⁶ et R⁷, R⁸, R⁹ représentant, respectivement indépendamment l'un de l'autre, un atome d'hydrogène, à l'exception de R⁷ qui est un atome d'hydrogène lorsque Z² est une liaison directe, ou bien un radical hydrocarbure comportant de 1 à 10 atomes de C ou un radical hydrocarbure comportant de 3 à 6 atomes de C ou un radical hétérocyclique, chacun des derniers radicaux contenant du carbone cités étant non substitué ou bien substitué par un ou plusieurs radicaux du groupe halogène, hydroxy, amino, nitro, formyle, aminocarbonyle, carboxy, sulfonyle, cyano, thiocyanato, alcoxy en C₁ à C₆, alcényloxy en C₂ à C₆, alcynyloxy en C₂ à C₆, alcoxy(C₁ à C₆)-poly(alkylénoxy), hydroxy-poly(alkylénoxy), alkylthio en C₁ à C₆, mono et dialkyl(en C₁ à C₆)amino, alkyl(en C₁ à C₆)carbonyle, alcényl(en C₂ à C₆)carbonyle, alcynyl(en C₂ à C₆)carbonyle, alcoxy(en C₁ à C₆)carbonyle, alcényloxy(en C₂ à C₆)carbonyle, alcynyloxy(en C₂ à C₆)carbonyle, mono et dialkyl(en C₁ à C₈)aminocarbonyle, phényle, phénoxy, cycloalkyle en C₃ à C₆, phénylcarbonyle, phénoxycarbonyle, hétérocyclyle, alkylsulfonyle en C₁ à C₄ et, en cas de radicaux cycliques, également alkyle en C₁ à C₄,
chacun des 24 derniers substituants cités étant non substitué ou substitué par un ou plusieurs radicaux du groupe du groupe halogène, CN, SCN, NO₂, alcoxy en C₁ à C₄, alkylthio en C₁ à C₄, haloalcoxy en C₁ à C₄, formyle, alkylcarbonyle en C₁ à C₄, haloalkyl(en C₁ à C₄)carbonyle, alcoxy(en C₁ à C₄)carbonyle, haloalcoxy(en C₁ à C₄)carbonyle et, dans le cas de radicaux cycliques, également alkyle en C₁ à C₄ et haloalkyle en C₁ à C₄,
ou R⁸ et R⁹ conjointement à l'atome C d'un groupe CR⁸R⁹ (pour Y¹ ou Y²) ou deux radicaux R⁸ et R⁹ provenant de deux groupes Y¹ et/ou Y² conjointement aux atomes associés des groupes Y¹ et/ou Y² représentent respectivement un radical carbocyclique comportant de 3 à 8 atomes de C ou bien un radical hétérocyclique, chacun des deux derniers radicaux cités étant non substitué ou bien substitué par un ou plusieurs radicaux du groupe halogène, alkyle en C₁ à C₄, haloalkyle en C₁ à C₄, alcoxy en C₁ à C₄, haloalcoxy en C₁ à C₄, nitro et cyano,
X¹, X², X³, X⁴, respectivement indépendamment l'un de l'autre, représentent un atome de carbone substitué par un atome d'hydrogène ou l'un des substituants R⁵ définis, ou un atome d'azote ou deux symboles voisins X¹, X², X³ et X⁴, respectivement conjointement un groupe divalent de formule -O-, -S-, -NH- ou -NR-, dans laquelle R est défini pour R³, à condition que les groupes X¹, X², X³ et X⁴ forment avec l'unité C₂ associée du cycle condensé un cycle aromatique à cinq ou six éléments carbocyclique ou hétérocyclique,
(Y¹)ₘ m représente des groupes Y¹ divalents, chaque groupe Y¹ représentant, indépendamment d'autres radicaux Y¹, un groupe de formule -O-, -CO-, -C(=NR*)-, -S(O)q, -NR*- ou -N(O)-, q étant égal à 0, 1 ou 2 et R* étant défini comme pour R³, ou bien un groupe de formule CR⁸R⁹, R¹ et R⁹ étant définis comme ci-dessus
et
Y² représente un groupe défini comme pour Y¹ ou une liaison directe, deux groupes voisins de la paire de symboles Y¹ et Y¹ ou de la paire de symboles Y¹ et Y² représentant des groupes non hétéroatomiques ayant la même signification et les groupes (Y¹)ₘ et Y² formant avec l'unité C₂ associée du cycle aromatique et avec l'atome de C associé à R⁴ un cycle non aromatique à quatre à huit éléments carbocyclique ou hétérocyclique condensé,
Z¹ et les groupes Z² représentent, respectivement indépendamment l'un de l'autre, une liaison directe ou un groupe divalent de formule -O-, -S(O)p, -S(O)p-O-, - O-S(O)p, -CO-, -O-CO-, -CO-O-, -NR'-, -O-NR'-, -NR'-O-, -NR'-CO-, -CO-NR'- et p étant égal à 0, 1 ou 2 et R' représentant un atome d'hydrogène, un radical alkyle comportant de 1 à 6 atomes de C, phényle, benzyle, cycloalkyle en C₃ à C₆ ou alcanoyle comportant de 1 à 6 atomes de C,
m est égal à 0, 1, 2, 3 ou 4 et
n est égale à 0, 1, 2, 3 ou 4.

2. Composés ou leurs sels selon la revendication 1, **caractérisé en ce que**
R¹ et R² représentent, respectivement indépendamment l'un de l'autre, un atome d'hydrogène, un radical amino, alkylamino ou dialkylamino comportant respectivement de 1 à 4 atomes de C dans le radical alkyle ou cycloalkylamino en C₃ à C₈, cycloalcénylamino en C₅ à C₆, un radical hydrocarbure ou un oxyradical hydrocarbure comportant respectivement de 1 à 10 atomes de C ou un radical hétérocyclyle, un oxyradical hétérocyclyle, un radical thio-hétérocyclyle ou un radical amino hétérocyclyle comportant respectivement de 3 à 9 atomes cycliques et de 1 à 3 atomes hétérocycliques du groupe N, O et S, chacun des six derniers radicaux cités étant non substitué ou bien substitué par un ou plusieurs radicaux du groupe halogène, hydroxy, amino, nitro, formyle, aminocarbonyle, carboxy, sulfonyle, cyano, thiocyanato, alcoxy en C₁ à C₆, alcényloxy en C₂ à C₆, alcynyloxy en C₁ à C₆, alcoxy(en C₁ à C₈)-poly(alkylénoxy), hydroxy-poly-(alkylénoxy), alkylthio en C₁ à C₈, mono et dialkyl(en C₁ à C₆)amino, alkyl(en C₁ à C₆)carbonyle, alcényl(en C₁ à C₆)carbonyle, alcynyl(en C₂ à C₆)carbonyle, alcoxy(en C₁ à C₆)carbonyle, alcényloxy(en C₁ à C₆)carbonyle, alcynyloxy(en C₂ à C₆)carbonyle, mono et dialkyl(en C₁ à C₆)aminocarbonyle, phényle, phénoxy, cycloalkyle en C₃ à C₆, phénylcarbonyle, phénoxycarbonyle, hétérocyclyle, alkylsulfonyle en C₁ à C₄, et dans le cas de radicaux cycliques, également alkyle en C₁ à C₄,
chacun des 24 derniers substituants cités étant non substitué ou substitué par un ou plusieurs radicaux du groupe halogène, CN, SCN, NO₂, alcoxy en C₁ à C₄, alkylthio en C₁ à C₄, haloalcoxy en C₁ à C₄, formyle, alkylcarbonyle en C₁ à C₄, haloalkyl(en C₁ à C₄)carbonyle, alcoxy(en C₁ à C₄)carbonyle, haloalcoxy(en C₁ à C₄)carbonyle et, dans le cas de radicaux cycliques, également alkyle en C₁ à C₄ et haloalkyle en C₁ à C₄,
ou un radical acyle d'un acide organique du groupe formyle, aminocarbonyle, mono ou dialkyl(en C₁ à C₄)aminocarbonyle, alkyl(en C₁ à C₆)carbonyle, alcényl(en C₂ à C₆)carbonyle, alcynyl(en C₂ à C₆)carbonyle, alcoxy(en C₁ à C₆)carbonyle, alcényloxy(en C₂ à C₈)carbonyle, phénylcarbonyle, alkyl(en C₁ à C₆)sulfonyle, chacun des 9 derniers radicaux cités étant non substitué dans la partie aliphatique ou dans le cycle phényle ou bien substitué par un ou plusieurs radicaux du groupe halogène, CN, SCN, NO₂, alcoxy en C₁ à C₄, haloalcoxy en C₁ à C₄, phényle le cas échéant substitué et non substitué, et, dans le cas de radicaux cycliques, également alkyle en C₁ à C₆ et haloalkyle en C₁ à C₆, ou R¹ et R² comportant, conjointement à l'atome d'azote du groupe NR¹R² un radical hétérocyclique saturé ou insaturé, non aromatique comportant 3 à 6 atomes cycliques et 1 à 4 atomes hétérocycliques, les éventuels autres atomes hétérocycliques étant choisis, outre l'atome de N, dans le groupe N, O et S et le radical étant non substitué ou bien substitué par un ou plusieurs radicaux du groupe halogène, CN, alkyle en C₁ à C₄, haloalkyle en C₁ à C₄, alcoxy en C₁ à C₄, haloalcoxy en C₁ à C₄, alcoxy(en C₁ à C₄)carbonyle et oxo,
R³ représente un atome d'hydrogène, un radical amino, alkyle en C₁ à C₆, alcoxy en C₁ à C₈, alcényle en C₂ à C₆, alcynyle en C₂ à C₆, cycloalkyle en C₃ à C₆, cycloalcényle en C₅ à C₆, phényle ou hétérocyclyle, chacun des huit derniers radicaux cités étant non substitué ou bien substitué par un ou plusieurs radicaux du groupe halogène, hydroxy, amino, nitro, formyle, carboxy, cyano, thiocyanato, alcoxy en C₁ à C₄, alcoxy(en C₁ à C₄)-alcoxy(en C₁ à C₄), hydroxyalcoxy en C₁ à C₄, alcoxy(en C₁ à C₄)-poly(alkylénoxy), hydroxy-poly(alkylénoxy), haloalcoxy en C₁ à C₄, alkylthio en C₁ à C₄, haloalkylthio en C₁ à C₄, monoalkyl(en C₁ à C₄)amino, dialkyl(en C₁ à C₄)amino, alkyl(en C₁ à C₄)carbonyle, alcoxy(en C₁ à C₄)carbonyle, aminocarbonyle, monoalkyl(en C₁ à C₄)aminocarbonyle, dialkyl(en C₁ à C₄)aminocarbonyle, phényle, phénoxy, cycloalkyle en C₃ à C₆, phénylcarbonyle, phénoxycarbonyle, hétérocyclyle, chacun des 6 derniers radicaux étant non substitué ou bien substitué par un ou plusieurs radicaux du groupe halogène, CN, SCN, NO₂ alkyle en C₁ à C₄, haloalkyle en C₁ à C₄, alcoxy en C₁ à C₄ et haloalcoxy (en C₁ à C₄), et alkyl(en C₁ à C₄)sulfonyle et haloalkylsulfonyle (en C₁ à C₄) et, dans le cas de radicaux cycliques, également alkyle (en C₁ à C₁) et haloalkyle (en C₁ à C₄),
ou bien un radical acyle de forme -B*-A*, dans laquelle
A* représente un atome d'hydrogène ou un radical alkyle en C₁ à C₆, alcényle en C₂ à C₆, alcynyle en C₂ à C₆, cycloalkyle en C₃ à C₆, cycloalcényle en C₅ à C₆ ou phényl, chacun des six derniers radicaux étant non substitué ou bien substitué par un
ou plusieurs radicaux du groupe halogène, hydroxy, amino, nitro, formyle, carboxy, cyano, thiocyanato, alcoxy en C₁ à C₄, haloalcoxy en C₁ à C₄, alkylthio en C₁ à C₄, monoalkyl(en C₁ à C₄)amino, dialkyl(en C₁ à C₄)amino, phényle et cycloalkyle en C₃ à C₆, chacun des 2 derniers radicaux cités étant non substitué ou bien substitué par un ou plusieurs radicaux du groupe halogène, CN, SCN, NO₂, alkyle en C₁ à C₄, haloalkyle en C₁ à C₄, alcoxy en C₁ à C₄ et haloalcoxy en C₁ à C₄, et, dans le cas de radicaux cycliques également alkyle en C₁ à C₄ et haloalkyle en C₁ à C₄,
et
B* un groupe divalent de formule -CO-, -CO-O-, -CO-NR'-, -S(O)p- ou -S(O)pO-, p étant égal à 0, 1 ou 2 et R' représentant un atome d'hydrogène, un radical alkyle comportant de 1 à 6 atomes de C, phényle, benzyle, cycloalkyle comportant de 3 à 6 atomes de C ou alcanoyle comportant de 1 à 4 atomes de C,
-B*-A*, y compris les substituants, présentant 1 à 12 de carbone,
R⁴ est un radical de formule -Z¹-R⁶, Z¹ et R⁶ étant définis comme indiqué ci-dessous,
R⁵ représente, respectivement indépendamment l'un de l'autre, un radical halogène, CN, SCN, NO₂ un radical de formule -Z²-R⁷, Z² et R⁷ étant définis comme indiqués ci-dessous ou deux radicaux R⁵ voisins représentant conjointement un cycle condensé comportant de 4 à 6 atomes cycliques qui est carbocyclique ou contient de 1 à 3 atomes hétérocycliques du groupe O, S et N et qui est non substitué ou substitué par un ou plusieurs radicaux du groupe halogène, alkyle en C₁ en C₄ et oxo.
R⁶ et R⁷, R⁸, R⁹ représentent, respectivement indépendamment l'un de l'autre, un atome d'hydrogène, à l'exception de R⁷ qui est un atome d'hydrogène lorsque Z² est une liaison directe, ou bien un radical hydrocarbure acyclique comportant de 1 à 10 atomes de C ou un radical hydrocarbure cyclique comportant de 3 à 6 atomes de C ou un radical hétérocyclique, chacun des 3 derniers radicaux contenant du carbone cités étant non substitué ou bien substitué par un ou plusieurs radicaux du groupe halogène, hydroxy, amino, nitro, formyle, aminocarbonyle, carboxy, sulfonyle, cyano, thiocyanato, alcoxy en C₁ à C₆, alcényloxy en C₂ à C₆, alcynyloxy en C₂ à C₆, alcoxy(C₁ à C₆)-poly(alkylénoxy), hydroxy-poly(alkylénoxy), alkylthio en C₁ à C₆, mono et dialkyl(C₁ à C₆)amino, alkyl(C₁ à C₆)carbonyle, alcényl(C₂ à C₆)carbonyle, alcynyl(C₂ à C₆)carbonyle, alcoxy(C₁ à C₆)carbonyle, alcényloxy(C₂ à C₆)carbonyle, alcynyloxy(C₂ à C₆)carbonyle, mono et dialkyl(C₁ à C₆)aminocarbonyle, phényle, phénoxy, cycloalkyle en C₃ à C₆, phénylcarbonyle, phénoxycarbonyle, hétérocyclyle, alkylsulfonyle en C₁ à C₄ et, en cas de radicaux cycliques, également alkyle en C₁ à C₄,
chacun des 24 derniers substituants cités étant non substitué ou substitué par un ou plusieurs radicaux du groupe halogène, CN, SCN, NO₂, alcoxy en C₁ à C₄, alkylthio en C₁ à C₄, haloalcoxy en C₁ à C₄, formyle, alkylcarbonyle en C₁ à C₄, haloalkyl(en C₁ à C₄)carbonyle, alcoxy(en C₁ à C₄)carbonyle, haloalcoxy(en C₁ à C₄)carbonyle et, dans le cas de radicaux cycliques, également alkyle en C₁ à C₄ et haloalkyle en C₁ à C₄,
X¹, X², X³, X⁴, respectivement indépendamment l'un de l'autre, représentent un atome de carbone substitué par un atome d'hydrogène ou l'un des substituant R⁵ définis, ou un atome d'azote ou deux symboles voisins X¹, X², X³ et X⁴, respectivement conjointement un groupe divalent de formule -O-, -S-, -NH- ou -NR-, dans laquelle R est défini comme pour R³, à condition que les groupes X¹, X², X³ et X⁴ forment avec l'unité C₂ associée du cycle condensé un cycle aromatique à cinq ou six éléments carbocyclique ou hétérocyclique,
(Y¹)ₘ m représente des groupes Y¹ divalents, chaque groupe Y¹ représentant, indépendamment d'autres radicaux Y¹, un groupe de formule -O-, -CO-, -C(=NR*)-, -S(O)q, -NR*- ou -N(O)-, q étant égal à 0, 1 ou 2 et R* étant défini comme pour R³ ou bien un groupe de formule CR⁸R⁹,
Y² représente un groupe comme défini comme pour Y¹ ou une liaison directe,
deux groupes voisins des paires de symboles Y¹ et Y¹ ou des paires de symboles Y¹ et Y² représentent des groupes non hétéroatomiques ayant la même signification et les groupes (Y¹)ₘ et Y² formant avec l'unité C₂ associée du cycle aromatique et avec l'atome de C associé à R⁴ un cycle non aromatique à quatre à huit éléments carbocyclique ou hétérocyclique condensé,
Z¹ et les groupes Z² représentant, respectivement indépendamment l'un de l'autre, une liaison directe ou un groupe divalent de formule -O-, -S(O)p, -S(O)p-O-, - O-S(O)ₚ, -CO-, -O-CO-, -CO-O-, -NR'-, -O-NR'-, -NR'-O-, -NR'-CO-, -CO-NR'- et p étant égal à 0, 1 ou 2 et R' représentant un atome d'hydrogène, un radical alkyle comportant de 1 à 6 atomes de C, phényle, benzyle, cycloalkyle comportant 3 à 6 atomes de carbone ou alcanoyle comportant de 1 à 6 atomes de C,
le radical hétérocyclyle présentant respectivement, s'il n'est pas défini plus précisément, même dans des radicaux composés, respectivement 3 à 9 atomes cycliques et 1 à 3 atomes hétérocycliques du groupe N, O et S.

3. Composés ou leurs sels selon la revendication 1 ou la revendication 2,
**caractérisés en ce que**
R¹ et R² représentent respectivement, indépendamment l'un de l'autre, un atome d'hydrogène, un radical amino, mono ou dialkyl(en C₁ à C₄)amino ou cycloalkyl(en C₃ à C₆)amino, cycloalcényl(en C₅ à C₈)-amino, un radical hydrocarbure ou un oxyradical hydrocarbure comportant respectivement de 1 à 10 atomes de C, ou un radical hétérocyclyle, un oxyradical hétérocyclyle, un radical thio-hétérocyclyle ou un radical hétérocyclylamino comportant de 3 à 9 atomes cycliques et de 1 à 3 atomes hétérocycliques du groupe N, O et S, chacun des six derniers radicaux cités étant non substitué, ou substitué par un ou plusieurs radicaux du groupe halogène, hydroxy, amino, nitro, formyle, aminocarbonyle, carboxy, sulfonyle, cyano, thiocyanato, alcoxy en C₁ à C₄, alcényloxy en C₂ à C₄, alcynyloxy en C₂ à C₄, alcoxy-poly(alkylénoxy) en C₁ à C₄, hydroxy-poly(alkylénoxy), alkylthio en C₁ à C₄, mono et dialkyl(en C₁ à C₄)amino, alkyl(en C₁ à C₄)carbonyle, alcényl(en C₂ à C₄)carbonyle, alcynyl(en C₂ à C₄)-carbonyle, alcoxy(en C₁ à C₄)carbonyle, alcényloxy(en C₂ à C₄)carbonyle, alcynyloxy(en C₂ à C₄)carbonyle, mono et dialkyl(en C₁ à C₄)aminocarbonyle, phényle, phénoxy, cycloalkyle (en C₃ à C₆), phénylcarbonyle, phénoxycarbonyle, hétérocyclyle, alkylsulfonyle (en C₁ à C₄) et, dans le cas de radicaux cycliques, également alkyle (en C₁ à C₄),
chacun des 24 derniers substituants cités étant non substitué ou substitué par un
ou plusieurs radicaux du groupe halogène, CN, SCN, NO₂, alcoxy en C₁ à C₄, haloalcoxy en C₁ à C₄, formyle, alkylcarbonyle en C₁ à C₄, haloalkyl(en C₁ à C₄)carbonyle, alcoxy(en C₁ à C₄)carbonyle, haloalcoxy(en C₁ à C₄)carbonyle et, dans le cas de radicaux cycliques, également alkyle en C₁ à C₄ et haloalkyle en C₁ à C₄,
ou un radical acyle d'un acide organique du groupe formyle, aminocarbonyle, mono ou dialkyl(en C₁ à C₄)aminocarbonyle, alkyl(en C₁ à C₄)carbonyle, alcényl(en C₂ à C₄)carbonyle, alcynyl(en C₂ à C₄)carbonyle, alcoxy(en C₁ à C₄)carbonyle, alcényloxy(en C₂ à C₄)carbonyle, phénylcarbonyle, alkyl(en C₁ à C₄)sulfonyle chacun des 9 derniers radicaux cités étant non substitué dans la partie aliphatique ou dans le cycle phényle ou bien substitué par un ou plusieurs radicaux du groupe halogène, CN, SCN, NO₂ alcoxy en C₁ à C₄, haloalcoxy en C₁ à C₄, phényle le cas échéant substitué et non substitué, et, dans le cas de radicaux cycliques, également alkyle en C₁ à C₄ et haloalkyle en C₁ à C₄,
ou
R¹ et R² comportant, conjointement à l'atome d'azote du groupe NR¹R², un radical hétérocyclique saturé ou insaturé, non aromatique comportant 3 à 6 atomes cycliques et 1 à 4 atomes hétérocycliques, les éventuels autres atomes hétérocycliques, étant choisis, outre l'atome de N, dans le groupe N, O et S et le radical étant non substitué ou bien substitué par un ou plusieurs radicaux du groupe halogène, CN, alkyle en C₁ à C₄, haloalkyle en C₁ à C₄, alcoxy en C₁ à C₄, haloalcoxy en C₁ à C₄, alcoxy(en C₁ à C₄)carbonyle et oxo,
R³ représente un atome d'hydrogène, un radical amino, formyle, alkyle en C₁ à C₄, cyanoalkyle en C₁ à C₄, haloalkyle en C₁ à C₄, mono, di, ou polyhydroxyalkyle en C₁ à C₄, hydroxy-poly-alkylénoxy(en C₂ à C₄)-alkyle en C₁ à C₄, mono, di ou poly-alcoxy(en C₁ à C₄)-alkyle en C₁ à C₄, alcoxy(en C₂ à C₄)-poly-alkylénoxy(en C₂ à C₄)-alkyle en C₁ à C₄, haloalcoxy(en C₁ à C₄)-alkyle en C₁ à C₄, alcényle en C₂ à C₆, haloalcényle en C₂ à C₆, alcynyle en C₂ à C₆, haloalcynyle en C₂ à C₆ ou aminocarbonyle ou
alkylamino(en C₁ à C₄)-alkyle en C₁ à C₄, dialkyl(en C₁ à C₄)-amino-alkyle en C₁ à C₄, alkylamino(en C₁ à C₄)-carbonyle, dialkylamino(en C₁ à C₄)-carbonyle, alcanoyle en C₁ à C₅, alcényl(en C₂ à C₄)carbonyle, alcoxy(en C₁ à C₄)carbonyle, alcényloxy(en C₂ à C₄)-carbonyle, aminocarbonyl-alkyle en C₁ à C₄, alkylamino(en C₁ à C₄)carbonyle-alkyle en C₁ à C₄, dialkyl(en C₁ à C₄)amino-carbonyl-alkyle en C₁ à C₄, alcoxy(en C₁ à C₄)-carbonyl-alkyle en C₁ à C₄, alcanoyl(en C₁ à C₅)-alkyle en C₁ à C₄, alcényl(en C₂ à C₄)carbonyl-alkyle en C₁ à C₁, alcoxy(en C₁ à C₄)carbonyle-alkyle en C₁ à C₄ ou alcényloxy(en C₂ à C₄)carbonyl-alkyle en C₁ à C₄, chacun des 16 derniers radicaux cités étant non substitué ou substitué par un ou plusieurs radicaux du groupe halogène CN, alcoxy en C₁ à C₄ et haloalcoxy en C₁ à C₄ ou
cycloalkyle en C₃ à C₈, cycloalkylamino(en C₃ à C₆)-alkyle en C₁ à C₄, phényle, phénylcarbonyle, phénoxycarbonyle, phénylcarbonyl-alkyle en C₁ à C₄, phénoxy-alkyle en C₁ à C₄, phényl-alkyle en C₁ à C₄, hétérocyclyle, hétérocyclyl-alkyle en C₁ à C₄ ou l'un des 10 derniers radicaux cités substitué dans la partie acyclique ou dans la partie cyclique par un ou plusieurs radicaux du groupe halogène, nitro, cyano, alkyle en C₁ à C₄, alcoxy en C₁ à C₄, alkylthio en C₁ à C₄, haloalkyle en C₁ à C₄, haloalcoxy en C₁ à C₄, formyle, alkyl(en C₁ à C₄)-carbonyle, alcoxy(en C₁ à C₄)-carbonyle et alcoxy en C₁ à C₄,
R⁴ représente un atome d'hydrogène, un radical formyle, carboxy, cyano, thiocyanato, aminocarbonyle, alkyle en C₁ à C₄, cyano-alkyle en C₁ à C₄, alcoxy en C₁ à C₄, alkylamino en C₁ à C₄, dialkyl(en C₁ à C₄)-amino, haloalkyle en C₁ à C₄, hydroxyalkyle en C₁ à C₄, alcoxy(en C₁ à C₄)-alkyle en C₁ à C₄, haloalcoxy(en C₁ à C₄)-alkyle en C₁ à C₄, alkylthio en C₁ à C₄, haloalkylthio en C₁ à C₄, alcényle en C₁ à C₆, haloalcényle C₂ à C₆, alcynyle C₂ à C₆, haloalcynyle C₂ à C₆, alkylamino(en C₁ à C₄)-alkyle en C₁ à C₄, dialkyl(en C₁ à C₄)-amino-alkyle en C₁ à C₄, cycloalkylamino(en C₃ à C₉)-alkyle en C₁ à C₄, cycloalkyle en C₃ à C₉, hétérocyclyl-alkyle en C₁ à C₄ avec 3 à 6 éléments cycliques, les groupes cycliques dans les 3 derniers radicaux cités étant non substitués ou substitués par un ou plusieurs radicaux du groupe alkyle en C₁ à C₄, halogène et cyano, ou bien phényle, phénoxy, phénylcarbonyle, phénylcarbonyl-alkyle en C₁ à C₄, phénoxycarbonyle, alkyl(en C₁ à C₄)-carbonyle, alcoxy(en C₁ à C₄)-carbonyle, alkylamino(en C₁ à C₄)-carbonyle, alcoxy(en C₁ à C₄)-carbonyl-alkyle en C₁ à C₄, alkylamino(en C₁ à C₄)-carbonyl-alkyle en C₁ à C₄, phénoxy-alkyle en C₁ à C₄, phényl-alkyle en C₁ à C₄, hétérocyclyle, hétérocyclylamino, hétérocyclyloxy, hétérocyclylthio, alcanoylamino en C₁ à C₅, N-alcanoyl(en C₁ à C₅)-N-alkyl(en C₁ à C₄)-amino, alcényl(en C₂ à C₄)carbonylamino, alcynyl(en C₂ à C₄)carbonylamino, alcoxy(en C₁ à C₄)carbonylamino, alcényloxy(en C₂ à C₄)carbonylamino, alcynyloxy(en C₂ à C₄)carbonylamino, phénylcarbonylamino, phénoxycarbonylamino, alkylsulfonyle en C₁ à C₄, alcénylsulfonyle en C₂ à C₄ ou l'un des 27 derniers radicaux cités substitué, dans la partie acyclique ou dans la partie cyclique, par un ou plusieurs radicaux du groupe halogène, nitro, cyano, alcoxy en C₁ à C₄, alkylthio en C₁ à C₄, haloalcoxy en C₁ à C₄, formyle, alkyl(en C₁ à C₄)-carbonyle, alcoxy(en C₁ à C₄)-carbonyle, alcoxy en C₁ à C₄ et, dans le cas de parties cycliques, également par un groupe alkyle en C₁ à C₄ et haloalkyle en C₁ à C₄,
R⁵ Si n est égal à 1, les radicaux R⁵, respectivement indépendants l'un de l'autre, représentent, si n est supérieur à 1, un radical halogène, hydroxy, amino, nitro, formyle, carboxy, cyano, thiocyanato, aminocarbonyle, alkyle en C₁ à C₄, cyano-alkyle en C₁ à C₄, alcoxy en C₁ à C₄, alkylamino en C₁ à C₄, dialkyl(en C₁ à C₄)-amino, haloalkyle en C₁ à C₄, hydroxyalkyle en C₁ à C₄, alcoxy(en C1 à C₄)-alkyle en C₁ à C₄, halo(en C₁ à C₄)alcoxy-alkyle en C₁ à C₄, alkylthio en C₁ à C₄, halo(en C₁ à C₄)alkylthio, alcényle en C₂ à C₆, haloalcényle en C₂ à C₆, alcynyle en C₂ à C₆, haloalcynyle en C₂ à C₆, alkylamino(en C₁ à C₄)-alkyle en C₁ à C₄, dialkyl(en C₁ à C₄)-amino-alkyle en C₁ à C₄, cycloalkylamino(en C₃ à C₉)-alkyle en C₁ à C₄, cycloalkyle en C₃ à C₉, hétérocyclyle-alkyle en C₁ à C₄ avec 3 à 6 éléments cycliques, les groupes cycliques dans les 3 derniers radicaux cités étant non substitués ou substitués par un ou plusieurs radicaux du groupe alkyle en C₁ à C₄, halogène et cyano, ou
phényle, phénoxy, phénylcarbonyle, phénylcarbonyloxy, phénylcarbonyl-alkyle en C₁ à C₄, phénoxycarbonyle, alkyl(en C₁ à C₄)-carbonyle, alcoxy(en C₁ à C₄)-carbonyle, alkylamino(en C₁ à C₄)-carbonyle, alcoxy(en C₁ à C₄)carbonyl-alkyle en C₁ à C₄, alkylamino(en C₁ à C₄)-carbonyl-alkyle en C₁ à C₄, phénoxyalkyle en C₁ à C₄, phénylalkyle en C₁ à C₄, hétérocyclyle, hétérocyclylamino, hétérocyclyloxy, hétérocyclylthio, alcanoylamino en C₁ à C₅, N-alcanoyl(en C₁ à C₅)-N-alkyl(en C₁ à C₄)-amino, alcényl(en C₂ à C₄)carbonylamino, alcynyl(en C₂ à C₄)carbonylamino, alcoxy(en C₁ à C₄)carbonylamino, alcényloxy(en C₂ à C₄)carbonylamino, alcynyloxy(en C₂ à C₄)carbonylamino, phénylcarbonylamino, phénoxycarbonylamino, alkylsulfonyle en C₁ à C₄, alcénylsulfonyl en C₁ à C₄ ou l'un des 28 derniers radicaux cités substitué, dans la partie acyclique ou dans la partie cyclique, par un ou plusieurs radicaux du groupe halogène, nitro, cyano, alcoxy en C₁ à C₄, alkylthio en C₁ à C₄, haloalcoxy en C₁ à C₄, formyle, alkyl(en C₁ à C₄)-carbonyle, alcoxy(en C₁ à C₄)-carbonyle, alcoxy en C₁ à C₄ et, dans le cas de parties cycliques, également par un groupe alkyle en C₁ à C₄ et haloalkyle en C₁ à C₄, le radical hétérocyclyle dans les radicaux contenant respectivement 3 à 9 atomes cycliques et 1 à 3 atomes hétérocycliques du groupe N, O et S,
ou deux radicaux R⁵ voisins représentant un cycle condensé comportant de 4 à 6 atomes cycliques qui est carbocyclique ou contient des atomes hétérocycliques du groupe O, S et N et qui est non substitué ou substitué par un ou plusieurs radicaux du groupe halogène, alkyle en C₁ à C₄ et oxo,
R⁸ et R⁹ représentent, respectivement indépendamment l'un de l'autre, un atome d'hydrogène, un radical formyle, carboxy, cyano, thiocyanato, aminocarbonyle, alkyle en C₁ à C₄, cyano-alkyle en C₁ à C₄, alcoxy en C₁ à C₄, alkylamino en C₁ à C₄, dialkyl(en C₁ à C₄)-amino, haloalkyle en C₁ à C₄, hydroxyalkyle en C₁ à C₄, alcoxy(en C₁ à C₄)-alkyle en C₁ à C₄, haloalcoxy(en C₁ à C₄)-alkyle en C₁ à C₄, alkylthio en C₁ à C₄, haloalkylthio en C₁ à C₄, alcényle en C₂ à C₆, haloalcényle en C₂ à C₆, alcynyle en C₂ à C₆, haloalcynyle en C₂ à C₆, alkylamino(en C₁ à C₄)-alkyle en C₁ à C₄, dialkyl(en C₁ à C₄)amino-alkyle en C₁ à C₄, cycloalkylamino(en C₃ à C₉)-alkyle en C₁ à C₄, cycloalkyle en C₃ à C₉, hétérocyclyl-alkyle en C₁ à C₄ avec 3 à 6 éléments cycliques, les groupes cycliques dans les 3 derniers radicaux cités étant non substitués ou substitués par un ou plusieurs radicaux du groupe alkyle en C₁ à C₄, halogène et cyano, ou
phényle, phénoxy, phénoxycarbonyle, phénylcarbonyl-alkyle en C₁ à C₄, phénoxycarbonyle, alkyl(en C₁ à C₄)-carbonyle alcoxy(en C₁ à C₄)-carbonyle, alkylamino(en C₁ à C₄)-carbonyle, alcoxy(en C₁ à C₄)carbonyl-alkyle en C₁ à C₄, alkylamino(en C₁ à C₄)-carbonyl-alkyle en C₁ à C₄, phénoxyalkyle en C₁ à C₄, phénylalkyle en C₁ à C₄, hétérocyclyle, hétérocyclylamino, hétérocyclyloxy, hétérocyclylthio, alcanoylamino en C₁ à C₅, N-alcanoyl(en C₁ à C₅)-N-alkyl(en C₁ à C₄)-amino, alcényl(en C₂ à C₄)carbonylamino, alcynyl(en C₂ à C₄)carbonylamino, alcoxy(en C₁ à C₄)carbonylamino, alcényloxy(en C₂ à C₄)carbonylamino, alcynyloxy(en C₂ à C₄)carbonylamino, phénylcarbonylamino, phénoxycarbonylamino, alkylsulfonyle en C₁ à C₄, alcénylsulfonyl en C₂ à C₄
ou l'un des 27 derniers radicaux cités substitué, dans la partie acyclique ou dans la partie cyclique, par un ou plusieurs radicaux du groupe halogène, nitro, cyano, alcoxy en C₁ à C₄, alkylthio en C₁ à C₄, haloalcoxy en C₁ à C₄, formyle, alkyl(en C₁ à C₄)-carbonyle, alcoxy(en C₁ à C₄)-carbonyle, alcoxy en C₁ à C₄ et, dans le cas de parties cycliques, également par un groupe alkyle en C₁ à C₄ et haloalkyle en C₁ à C₄,
X¹, X², X³, X⁴, respectivement indépendamment l'un de l'autre, représentent un atome de carbone substitué par un atome d'hydrogène ou l'un des substituants R⁵ définis, ou un atome d'azote ou deux symboles voisins X¹, X², X³ et X⁴, respectivement conjointement à un groupe divalent de formule -O-, -S-, -NH- ou - NR-, dans laquelle R est défini comme pour R³, à condition que les groupes X¹, X², X³ et X⁴ forment avec l'unité C₂ associée du cycle condensé un cycle aromatique à cinq ou six éléments carbocyclique ou hétérocyclique,
(Y¹)ₘ m représente des groupes Y¹ divalents, chaque groupe Y¹ représentant, indépendamment d'autres radicaux Y¹, un groupe de formule -O-, -CO-, -C(=NR*)-, -S(O)_{q}, -NR*- ou -N(O)-, q étant égal à 0, 1 ou 2 et R* étant défini comme pour R³, ou bien un groupe de formule CR⁸R⁹,
et
Y² représente un groupe comme défini pour Y¹ ou une liaison directe,
deux groupes voisins de la paire de symboles Y¹ et Y¹ ou de la paire de symboles Y¹ et Y² représentent des groupes non hétéroatomiques ayant la même signification et les groupes (Y¹)ₘ et Y^{z} formant avec l'unité C₂ associée du cycle aromatique et avec l'atome de C associé à R⁴ un cycle non aromatique à quatre à huit éléments carbocyclique ou hétérocyclique condensé,
le radical hétérocyclyle présentant respectivement, s'il n'est pas défini plus précisément, également dans des radicaux composés, respectivement 3 à 9 atomes cycliques et 1 à 3 atomes hétérocycliques du groupe N, O et S.

4. Composés ou leurs sels selon l'une quelconque des revendications 1 à 3,
**caractérisés en ce que**
R¹, R² indépendamment l'un de l'autre, représentent un atome d'hydrogène, un radical amino, formyle, alkyle en C₁ à C₄, alkylamino en C₁ à C₄, dialkyl(en C₁ à C₄)-amino, hydroxyalkyle en C₁ à C₄, alcoxy(en C₁ à C₄)-alkyle en C₁ à C₄, haloalcoxy(en C₁ à C₄)-alkyle en C₁ à C₄, alcényle en C₂ à C₆, alcynyle en C₂ à C₆, haloalcynyle en C₂ à C₆, alkylamino(en C₁ à C₄)-alkyle en C₁ à C₄ ou phényle, phénoxy, phénylcarbonyle, phénylcarbonylalkyle en C₁ à C₄, alcoxy(en C₁ à C₄)carbonyl-alkyle en C₁ à C₄, alkylamino(en C₁ à C₄)carbonyl-alkyle en C₁ à C₄, alkyl(en C₁ à C₄)carbonyle, alcoxy(en C₁ à C₄)carbonyle, aminocarbonyle, alkylamino(en C₁ à C₄)-carbonyle ou l'un des 10 derniers radicaux cités substitué dans la partie acyclique ou dans la partie cyclique par un ou plusieurs radicaux du groupe halogène nitro, cyano, alkyle en C₁ à C₄, alcoxy en C₁ à C₄, alkylthio en C₁ à C₄, haloalkyle en C₁ à C₄, formyle, alkyl(en C₁ à C₄)-carbonyle, alcoxy(en C₁ à C₄)-carbonyle, alcoxy en C₁ à C₄,
R³ représente un atome d'hydrogène, un radical amino, formyle, alkyle en C₁ à C₄, cyanoalkyle en C₁ à C₄, alcoxy(en C₁ à C₄)-alkyle en C₁ à C₄, alcényle en C₂ à C₆, haloalcényle en C₂ à C₆, alcynyle en C₂ à C₆ ou phényle, alkylcarbonyle en C₁ à C₄, alcoxycarbonyle en C₁ à C₄, aminocarbonyle, phénoxyalkyle en C₁ à C₄, phénylalkyle en C₁ à C₄ ou l'un des 6 derniers radicaux cités substitué dans la partie acyclique ou dans la partie cyclique par un ou plusieurs radicaux du groupe halogène, alkyle en C₁ à C₄ et alcoxy en C₁ à C₄ ;
R⁴ représente un atome d'hydrogène, un radical alkyle en C₁ à C₄, cyanoalkyle en C₁ à C₄, haloalkyle en C₁ à C₄, hydroxyalkyle en C₁ à C₄, alcoxy(en C₁ à C₄)-alkyle en C₁ à C₄, haloalcoxy(en C₁ à C₄)-alkyle en C₁ à C₄, alcényle en C₂ à C₆, haloalcényle en C₂ à C₆, alcynyle en C₂ à C₆, alkylamino(en C₁ à C₄)-alkyle en C₁ à C₄, cycloalkylamino(en C₃ à C₉)-alkyle en C₁ à C₄, cycloalkyle en C₃ à C₉, hétérocyclyl-alkyle en C₁ à C₄ avec 3 à 9 éléments cycliques, les groupes cycliques dans les 3 derniers radicaux cités étant non substitués ou bien substitués par un ou plusieurs radicaux du groupe alkyle en C₁ à C₄, halogène et cyano, ou phényle, phénoxyalkyle en C₁ à C₄, phénylalkyle en C₁ à C₄, hétérocyclyle, ou l'un des 4 derniers radicaux cités substitué dans la partie acyclique ou dans la partie cyclique par un ou plusieurs radicaux du groupe halogène, nitro, cyano, alcoxy en C₁ à C₄, alkylthio en C₁ à C₄, haloalcoxy en C₁ à C₄, alkyl(en C₁ à C₄)-carbonyle, alcoxy(en C₁ à C₄)-carbonyle, alcoxy en C₁ à C₄ et, dans le cas de parties cycliques, également un radical alkyle en C₁ à C₄ et haloalkyle en C₁ à C₄,
R⁵ si n est égal à 1, et les radicaux R⁵, respectivement indépendants l'un de l'autre, représentent, si n est supérieur à 1, un radical halogène, hydroxy amino, nitro, formyle, cyano, aminocarbonyle, monoalkylaminocarbonyle en C₁ à C₄, dialkylaminocarbonyle en C₁ à C₄, alkyle en C₁ à C₄, cyano-alkyle en C₁ à C₄, alcoxy en C₁ à C₄, alkylamino en C₁ à C₄, haloalkyle en C₁ à C₄, hydroxyalkyle en C₁ à C₄, alcoxy(en C₁ à C₄)-alkyle en C₁ à C₄, alcényle en C₂ à C₄, alcynyle en C₂ à C₄, alkylamino(en C₁ à C₄)-alkyle en C₁ à C₄, dialkyl(en C₁ à C₄)-amino-alkyle en C₁ à C₄, ou
alkylcarbonyle en C₁ à C₄, haloalkyl(en C₁ à C₄)-carbonyle, alcoxy(en C₁ à C₄)-alkyl(en C₁ à C₄)carbonyle, formylamino, alkyl(en C₁ à C₄)-carbonylamino, haloalkyl(en C₁ à C₄)carbonylamino,
phényle, phénoxy, phénylcarbonyle, phénylcarbonylamino, hétérocyclyle ou l'un des 5 derniers radicaux cités substitué par un ou plusieurs radicaux du groupe halogène, alkyle en C₁ à C₄, haloalkyle en C₁ à C₄, alcoxy en C₁ à C₄, haloalcoxy en C₁ à C₄ et alkylthio en C₁ à C₄,
R⁸ et R⁹ représentent, respectivement indépendamment l'un de l'autre, un atome d'hydrogène, un radical alkyle en C₁ à C₄, cyano-alkyle en C₁ à C₄, haloalkyle en C₁à C₄, hydroxyalkyle en C₁ à C₄, alcoxy(en C₁ à C₄)-alkyle en C₁ à C₄, haloalcoxy(en C₁ à C₄)-alkyle en C₁ à C₄, alcényle en C₂ à C₆, halo-alcényle en C₂ à C₈, alcynyle en C₂ à C₆, alkylamino(en C₁ à C₄)-alkyle en C₁ à C₄, cycloalkylamino(en C₃ à C₉)-alkyle en C₁ à C₄, cycloalkyle en C₃ à C₉, hétérocyclyl-alkyle en C₁ à C₄ avec 3 à 9 éléments cycliques, les groupes cycliques dans les 3 derniers radicaux cités étant non substitués ou bien substitués par un ou plusieurs radicaux du groupe alkyle en C₁ à C₄, halogène et cyano, ou
phényle, phénoxy-alkyle en C₁ à C₄, phénylalkyle en C₁ à C₄, hétérocyclyle, ou l'un des 4 derniers radicaux cités substitué, dans la partie acyclique ou dans la partie cyclique, par un ou plusieurs radicaux du groupe halogène, nitro, cyano, alcoxy en C₁ à C₄, alkylthio en C₁ à C₄, haloalcoxy en C₁ à C₄, alkyl(en C₁ à C₄)-carbonyle, alcoxy(en C₁ à C₄)-carbonyle, alcoxy en C₁ à C₄ et, dans le cas de partie cycliques, également, par un groupe alkyle en C₁ à C₄ et haloalkyle en C₁ à C₄.

5. Composés ou leurs sels selon l'une quelconque des revendications 1 à 4,
**caractérisés en ce que**,
X¹, X², X³, X⁴, respectivement indépendamment l'un de l'autre, représentent un atome de carbone substitué par un atome d'hydrogène ou l'un des substituant R⁵ définis, ou un atome d'azote ou deux symboles voisins X¹, X², X³ et X⁴, respectivement conjointement un groupe divalent de formule -O-, -S-, -NH- ou -NR-, dans laquelle R représente un atome d'hydrogène ou un radical en C₁ à C₄, à condition que les groupes X¹, X², X³ et X⁴ forment avec l'unité C₂ associée du cycle condensé un cycle aromatique à cinq ou six éléments carbocyclique ou hétérocyclique,
(Y¹)ₘ m représente des groupes Y¹ divalents, chaque groupe Y¹ représentant, indépendamment d'autres radicaux Y¹, un groupe de formule CH₂, CH(CH₃), CH(C₂H₅), CH(CH₃)₂ ou CH(C₆H₅) et m = 0, 1, 2 ou 3 et
Y² représente une liaison directe ou un groupe de formule -O-, -S-, CH₂, CH(CH₃) ou alkylamino en C₁ à C₄,
deux groupes voisins de la paire de symboles Y¹ ou Y¹ ou de la paire de symboles Y¹ et Y² étant des groupes non hétéroatomiques ayant la même signification et les groupes (Y¹)ₘ et Y² formant avec l'unité C₂ associée du cycle aromatique et l'atome de C associé à R⁴ un cycle non aromatique à quatre à huit éléments carbocyclique ou hétérocyclique condensé.

6. Procédé de préparation de composés de formule (I) et leurs sels tels que définis selon l'une quelconque des revendications 1 à 5,
**caractérisé en ce qu'**on convertit
a) un composé de formule (II)
**H- R¹³** (II)
dans laquelle R¹³ est un groupe fonctionnel du groupe des esters de l'acide carboxylique, des orthoesters de l'acide carboxylique, des chlorures de l'acide carboxylique, des amides de l'acide carboxylique et des anhydrides de l'acide carboxylique,
avec un biguanide de formule (III) ou un sel d'addition d'acide de celui-ci 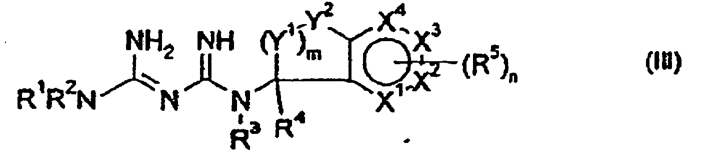 ou on convertit
b) un composé de formule (IV) 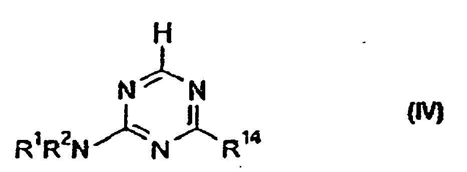 dans laquelle R¹⁴ représente un radical remplaçable ou un groupe partant, avec une amine de formule (V) ou un sel d'addition d'acide de celle-ci 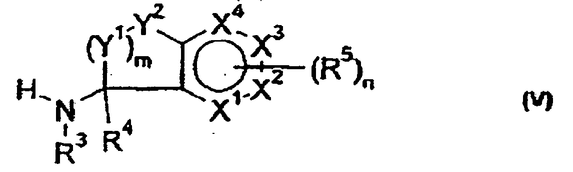 ou on convertit
c) une diamino-1,3,5-triazine de formule (VI) avec un isocyanate de formule (VII) 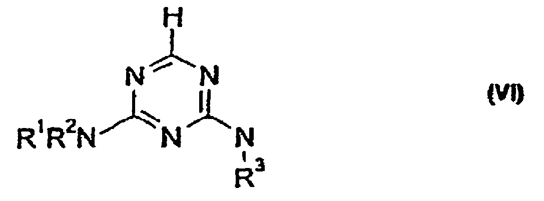 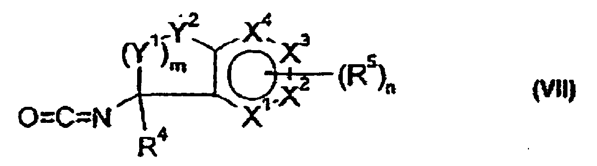 ou on clive,
d) pour une triazine de structure (VIII), le radical R¹⁵ qui est un groupe partant
ou un radical pouvant être clivé. 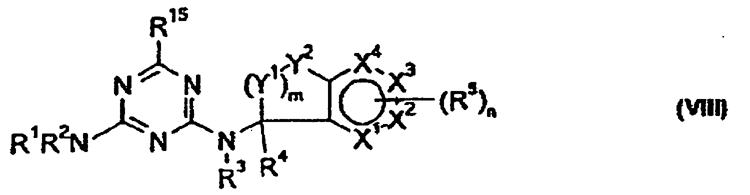 dans les formules (II), (III), (IV), (V), (VI), (VII) et (VIII), les radicaux R¹, R², R³, R⁴, R⁵, X¹, X², X³, X⁴, Y¹ et Y² et les symboles m et n étant définis comme dans la formule (I).

7. Herbicide ou produit régulant la croissance des végétaux,
**caractérisé en ce qu'**
il contient un ou plusieurs composés de formule (I) ou ses sels selon l'une quelconque des revendications 1 à 5 et des auxiliaires de formulation utilisables dans la protection phytosanitaire.

8. Procédé permettant de lutter contre les mauvaises herbes ou de réguler la croissance de végétaux,
**caractérisé en ce qu'**
on applique une quantité efficace d'un ou plusieurs composés de formule (I) ou leurs sels selon l'une quelconque des revendications 1 à 5 sur les plantes, les graines de plantes ou la surface agricole.

9. Utilisation de composés de formule (I) et leurs sels selon l'une quelconque des revendications 1 à 5 en tant qu'herbicides ou régulateurs de la croissance des végétaux.

10. Utilisation selon la revendication 9,
**caractérisée en ce qu'**
on utilise les composés de formule (I) ou leurs sels pour lutter contre les mauvaises herbes ou pour réguler la croissance dans les cultures de plantes de rapport ou de plantes décoratives.
